# EUROPEAN PATENT APPLICATION

(11) **EP 4 471 005 A1**
(43) Date of publication of application: **04.12.2024**
(21) Application number: 23742997.2
(22) Date of filing: 20.01.2023
(51) Int. Cl.: C07C 237/08, A61K 47/68, A61K 47/66, A61K 47/60, A61P 35/00

(54) **CONJUGATE AND USE THEREOF**

(30) Priority: 24.01.2022 CN 202210078846; 28.07.2022 CN 202210897004; 01.08.2022 CN 202210917922
(71) Applicant: Beijing Chempion Biotechnology Co., Ltd., Beijing 100094 (CN)
(72) Inventor: CHEN, Jian, Nanjing, Jiangsu 210061 (CN); ZHAO, Haibo, Nanjing, Jiangsu 210061 (CN)
(74) Representative: Cabinet Becker et Associés
(86) International application number: PCT/CN2023/073332
(87) International publication number: WO 2023/138682

(57) **Abstract**

The present invention relates to a compound of formula (I), or a tautomer, stereoisomer, pharmaceutically acceptable salt or isotopic variant thereof. The compound represented by formula (I) or the tautomer, stereoisomer, pharmaceutically acceptable salt or isotopic variant thereof is used as a novel linker compound, and can be used for preparing an ADC drug having a high DAR value. The present invention also relates to an ADC drug prepared by the linker.

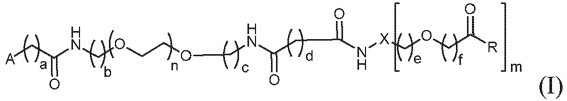

## Description

### TECHNICAL FIELD

The present disclosure relates to the biomedical field, specifically, relates to conjugates and uses thereof, more specifically, relates to compounds, conjugates, pharmaceutical compositions and uses thereof in the manufacture of medicaments.

### BACKGROUND

Antibody-Drug Conjugates (ADCs) are a class of biological drugs that link cytotoxic small molecules (cytotoxins) and antibodies by using permanent or unstable chemical linkers. The antibody binds to the specific antigen on the tumor cell membrane and induces endocytosis, allowing the antibody and the cytotoxic small molecule attached to the antibody to enter the cell. Subsequently, after lysosomal degradation, the small molecule drug is released into the cell and induces apoptosis.

The key technical points of ADC drugs include the selection of payload (toxin), linker, antibody, target, and conjugation technology.

The requirements of ADC for linked toxins include: 1. sufficient water solubility and stability in serum, because ADC may circulate in the body for several days; 2. toxins must have a functional group that can be used to conjugate with the linker; 3. toxins must be insensitive to enzymatic degradation reactions by lysosomes; 4. toxins reduce the aggregation effect (lipophilic substances are prone to occur) and change the interaction between ADC and pGp (permeability glycoprotein, drug efflux pump, easy to bind to lipophilic substances), which is the main cause of multidrug resistance (MDR) in tumor cells. In addition, for ADCs with cleavable linkers, bystander effects require toxins to kill the target cell and then come out and enter the cell membrane to kill the surrounding cells, requiring the toxins to have a certain lipid-water partition coefficient (LogP) and positive/neutral charge. Currently, the most widely used cytotoxic drugs in clinical practice can be divided into two categories according to their mechanism of action:

DNA damaging agents: calicheamicin (CLM, belonging to enediyne antibacterial drugs) acting on DNA, which causes DNA cleavage and cell death by binding to the minor groove of the DNA double helix.

Tubulin inhibitors: they bind to microtubules to prevent the aggregation of microtubules, arrest the cell cycle, and then induce tumor cell apoptosis. Tubulin inhibitors mainly include: dolastatin and its derivatives of auristatins (e.g. MMAE, MMAF, and MMAD); maytansine and its derivatives (maytansinoids, such as DM1, DM2, DM3, and DM4); halichondrin B and its derivatives (e.g. eribulin). At present, the vast majority of the ADC projects in clinical research use tubulin inhibitors, and there are products approved for marketing (Adcetris uses MMAE of auristatins, and Kadcyla uses maytansine derivative DM1). Auristatin dominates, accounting for more than 50% of ADC drugs under development. However, these tubulin inhibitors have shown significant ocular toxicity and peripheral neuropathy, which in many cases led to treatment interruption or dose reduction. These results clearly indicate the need for continued investigation of other cytotoxic agents and MTAs as ADC loadings that may provide toxicity and improve the therapeutic index.

Linkers are bridges connecting antibodies and cytotoxic drugs. An ideal conjugate must be stable in vitro or in blood circulation to prevent the systemic toxicity due to early release of cytotoxic drugs, and at the same time be able to quickly release effective cytotoxic drugs to kill cancer cells after entering the cancer cells. An ideal linker plays a key role in the success or failure of a drug, and the properties of a linker determine the pharmacokinetic properties and therapeutic effect of a drug. An ideal linker is one that does not release cytotoxins until the ADC reaches the target, and releases the cytotoxins only after the ADC reaches the cell. The drug release mode of a linker is divided into cleavable and non-cleavable modes. The non-cleavable mode is a form in which the linker is still connected to a cytotoxin after ADC has been digested by lysosome. There are three types of cleavable modes: the first is an acid-sensitive linker, which triggers the hydrolysis of acid-dependent groups, such as hydrazone groups, in the linker at low pH; the second is a glutathione-sensitive linker, wherein the linker containing a disulfide bond is reduced and cleaved by glutathione after reaching a cell because the concentration of glutathione in the cell is higher than that in plasma; and the last one is a lysosomal protease-sensitive linker, wherein some proteases in lysosomes can recognize and cleave specific peptides in the linker to release drugs.

Traditional cysteine-based site-specific antibody-drug conjugates (ADCs) are limited to one drug per cysteine. However, some applications require a higher drug-to-antibody ratio (DAR), for example, when using a payload with a low potency. Higher drug loading can be achieved using classical cysteine conjugation methods, but these methods may lead to heterogeneity, as well as suboptimal efficacy and pharmacokinetics.

Due to technical limitations, traditional ADC drugs with a high DAR value often lead to problems of pharmacokinetic instability, increased drug metabolism rate, reduced half-life, and increased systemic toxicity. Therefore, ADC drug technology still needs to be further developed and improved.

### SUMMARY

The present disclosure aims to solve, at least to a certain extent, one of the technical problems in the related art, such as providing ADC drugs with a high DAR value that solve the problems of pharmacokinetic instability, increased drug metabolism rate, reduced half-life, and increased systemic toxicity.

In the first aspect of the present disclosure, the present disclosure relates to a compound of formula (I), or a tautomer, a stereoisomer, a pharmaceutically acceptable salt or an isotopic variant thereof, 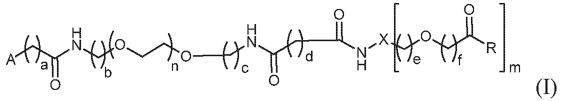 wherein,
a, b, c, d, e and f are each independently 0, 1, 2, 3, 4, 5, 6, 7, 8, 9 or 10; alternatively 0, 1, 2, 3, 4 or 5; alternatively 0, 1, 2 or 3; alternatively 1 or 2; alternatively a is 0 or 2, b, c and d are 2, and e and f are 1;
n is 1, 2, 3, 4, 5, 6, 7, 8, 9, 10, 11, 12, 13, 14, 15, 16, 17, 18, 19 or 20; alternatively 1, 2, 3, 4, 5, 6, 7, 8, 9 or 10; alternatively 1, 2, 3, 4 or 5; alternatively 1, 2 or 3; alternatively 1;
m is 2 or 3;
X is C, Nor Si;
A is -NH₂, -NH-PG1, 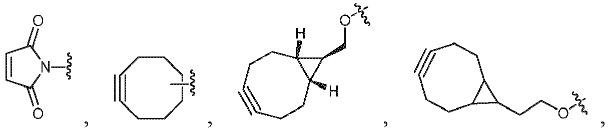 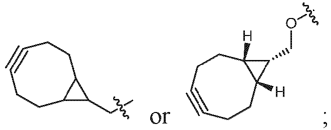
R is -OH, -O-PG2, 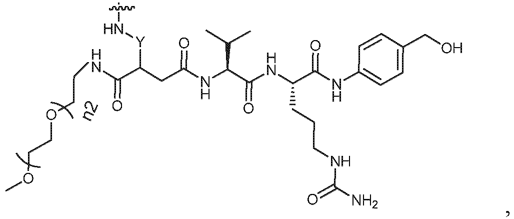 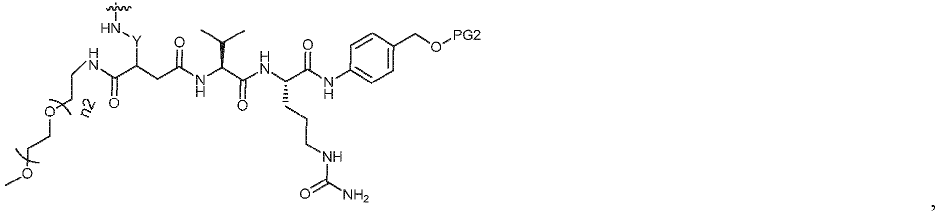 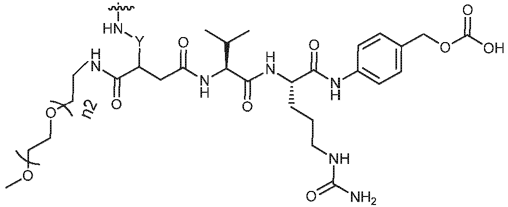 or 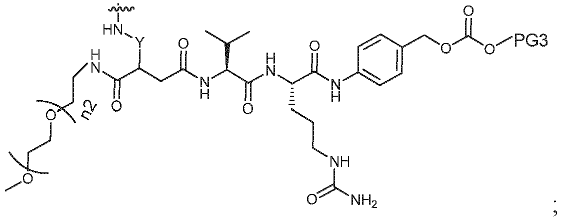
wherein PG1, PG2 and PG3 are protecting groups;
n2 is 5, 6, 7, 8, 9, 10, 11, 12, 13, 14, 15, 16, 17, 18, 19 or 20; alternatively 5, 6, 7, 8, 9, 10, 11, 12, 13, 14 or 15; alternatively 7, 8, 9, 10 or 11;
Y is a bond or 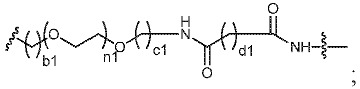
wherein n1 is 1, 2, 3, 4, 5, 6, 7, 8, 9, 10, 11, 12, 13, 14, 15, 16, 17, 18, 19 or 20; alternatively 1, 2, 3, 4, 5, 6, 7, 8, 9 or 10; alternatively 1, 2, 3, 4 or 5; alternatively 1, 2 or 3; alternatively n1 is 3, 4, 5, 6, 7 or 8, alternatively 3;
b1, c1 and d1 are each independently 0, 1, 2, 3, 4, 5, 6, 7, 8, 9 or 10; alternatively 0, 1, 2, 3, 4 or 5; alternatively 0, 1, 2 or 3; alternatively 1 or 2; alternatively b1, c1 and d1 are 2;
when X is C or Si, m is 3;
when X is N, m is 2;
with the proviso that, when A is 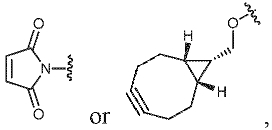 then n is not 1.

According to the embodiments of the present disclosure, the compound represented by formula (I) or a tautomer, a stereoisomer or a pharmaceutically acceptable salt thereof as a novel linker compound can be used for the manufacture of an ADC drug with high DAR value in a site-specific manner with each cysteine as a conjugation site, thereby reducing the exposure of small molecule drugs in vivo, improving the safety of small molecule drugs, and benefiting drug quality control. Moreover, the prepared ADC drug with high DAR value is more cytotoxic to target cells. According to the embodiments of the present disclosure, the compound represented by formula (I) as a linker introduces various types of water-soluble groups, which effectively avoid the phenomenon that ADC is extremely prone to aggregation and precipitation in plasma due to poor water solubility, so that the toxin comes out after killing the target cells and enters the cell membrane to kill the surrounding cells, thus showing significant advantages in terms of drug efficacy and side effects. The ADC drug prepared with the compound represented by formula (I) according to the embodiments of the present disclosure as a linker solves the problems of pharmacokinetic instability, increased drug metabolism rate, reduced half-life and increased systemic toxicity caused by high DAR value.

The ADC molecules of the present disclosure have a variety of structural specificities. For example, in an alternative embodiment, the molecule of the present disclosure with f being 1 can avoid the bond cleavage caused by anti-Markovnikov elimination; for example, in another alternative embodiment, the (CH₂CH₂O)ₙ₂ chain in the R group is connected by a stable amide bond; for example, in another alternative embodiment, the presence of a Y group containing a PEG chain in the R group is helpful for the preparation and stability of the molecule.

According to the embodiments of the present disclosure, the above compounds may further comprise at least one of the following additional technical features:
According to the embodiments of the present disclosure, provided herein is a compound with a structure represented by formula (II), or a tautomer, a stereoisomer, a pharmaceutically acceptable salt or an isotopic variant thereof, 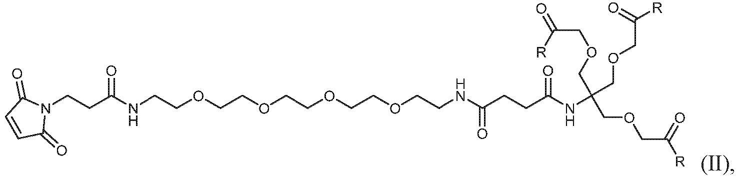 wherein R is as defined in the context.

According to the embodiments of the present disclosure, provided herein is a compound with a structure represented by formula (III), or a tautomer, a stereoisomer, a pharmaceutically acceptable salt or an isotopic variant thereof, 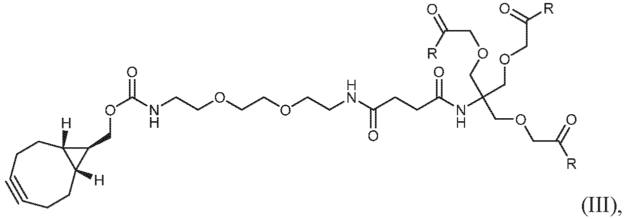 wherein R is as defined in the context.

According to the embodiments of the present disclosure, provided herein is a compound with a structure represented by formula (IV), or a tautomer, a stereoisomer, a pharmaceutically acceptable salt or an isotopic variant thereof, 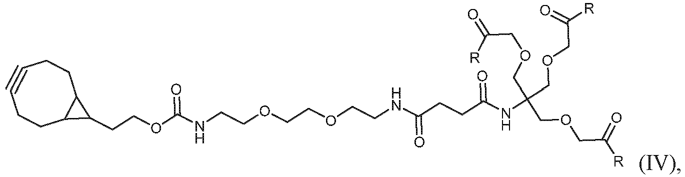 wherein R is as defined in the context.

According to the embodiments of the present disclosure, provided herein is a compound with a structure represented by formula (V), or a tautomer, a stereoisomer, a pharmaceutically acceptable salt or an isotopic variant thereof, 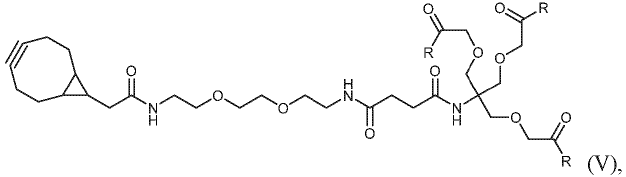 wherein R is as defined in the context.

According to the embodiments of the present disclosure, R is -OH, -O-PG2, 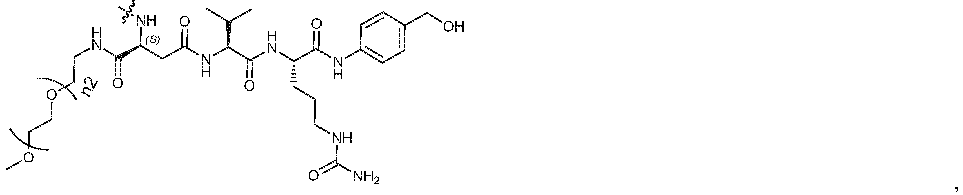 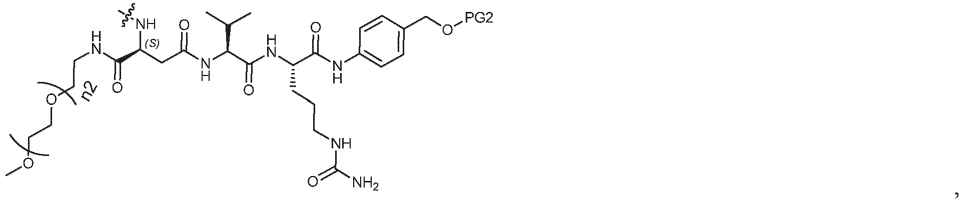 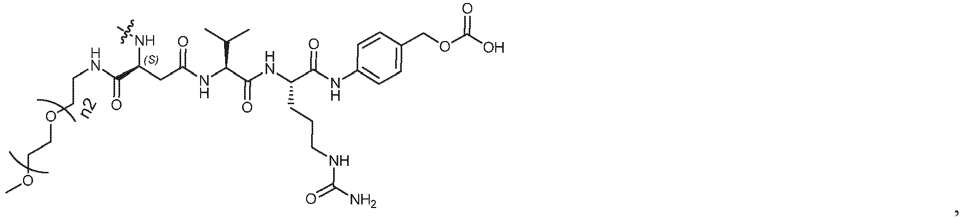 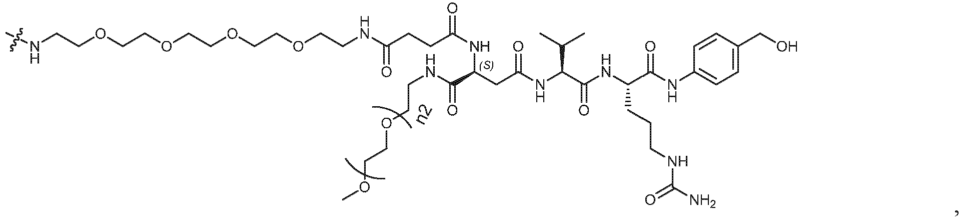 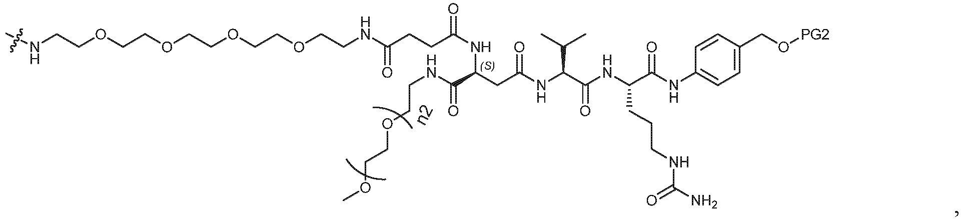 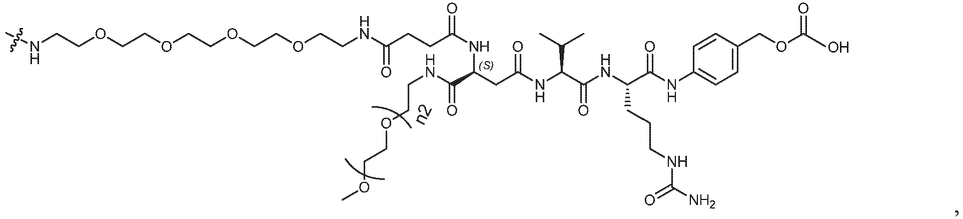 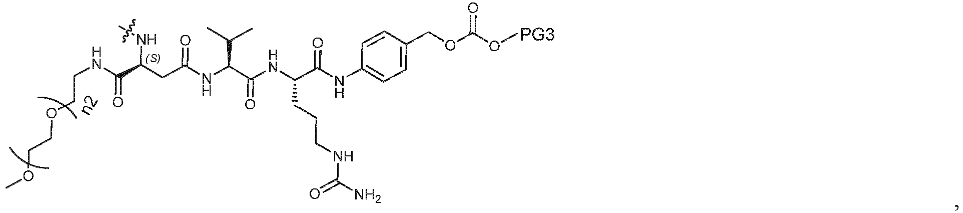 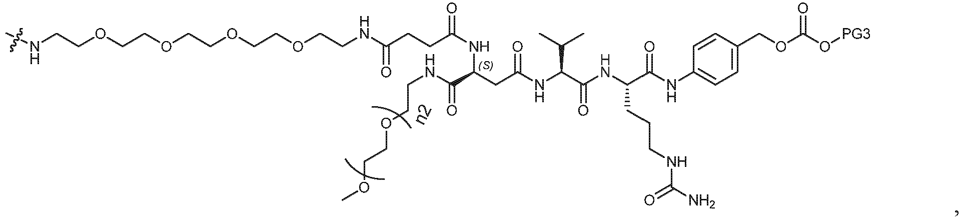 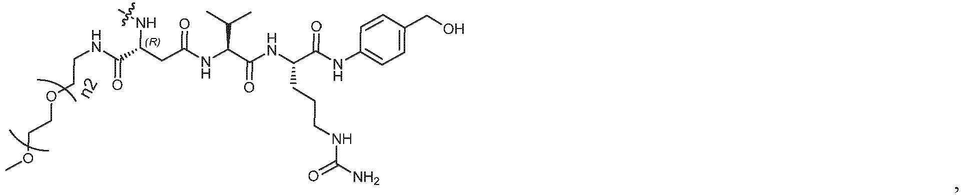 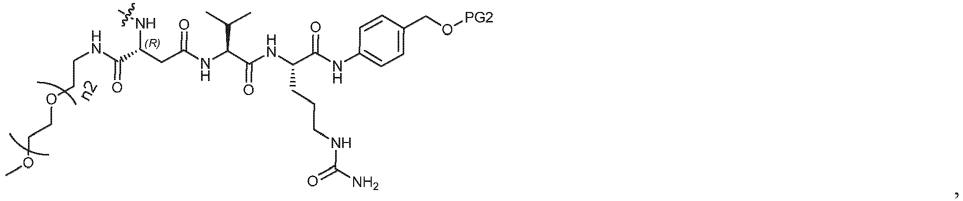 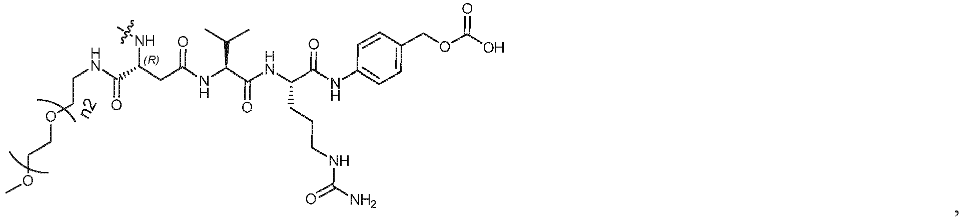 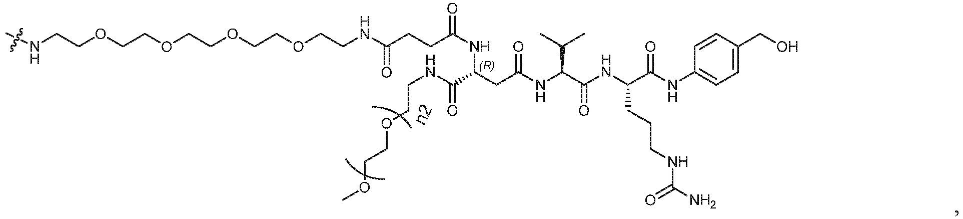 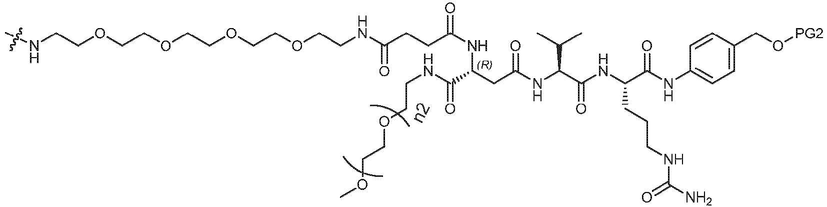 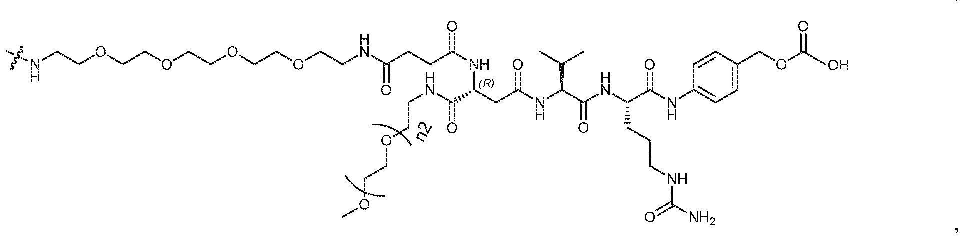 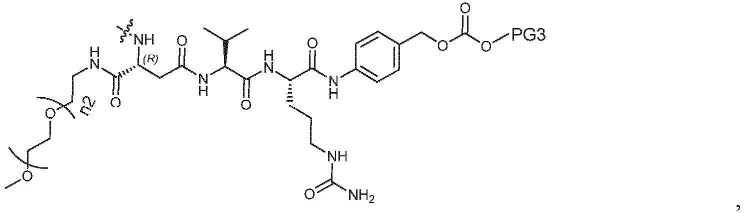 or

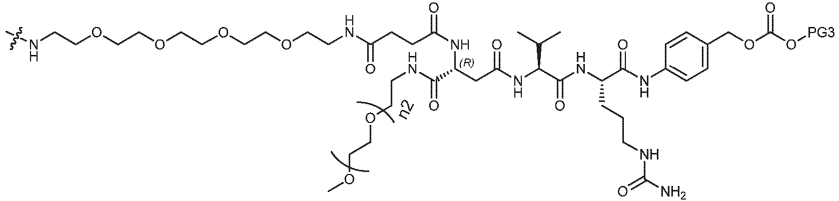

According to the embodiments of the present disclosure, n2 is 7 or 11.

According to the embodiments of the present disclosure, the PG1 is an amino-protecting group; optionally, the amino-protecting group is selected from acetyl, trifluoroacetyl, tert-butoxycarbonyl (BOC, Boc), benzyloxycarbonyl (CBZ, Cbz) and 9-fluorenylmethyleneoxycarbonyl (Fmoc); the PG2 is a hydroxyl-protecting group; optionally, the hydroxyl-protecting group is selected from acetyl and silyl; the PG3 is a carboxyl-protecting group; optionally, the carboxyl-protecting group is selected from -CH₂CH₂SO₂Ph, cyanoethyl, 2-(trimethylsilyl)ethyl, 2-(trimethylsilyl)ethoxymethyl, 2-(p-toluenesulfonyl)ethyl, 2-(p-nitrobenzenesulfonyl)ethyl, 2-(diphenylphosphino)ethyl and nitroethyl.

In the second aspect of the present disclosure, provided herein is a conjugate. According to the embodiments of the present disclosure, the conjugate comprises the above compound and a drug moiety to which the compound is covalently bonded via an R group. According to a specific embodiment of the present disclosure, the drug moiety is a small molecule drug or is called payload.

According to the embodiments of the present disclosure, the conjugate of the second aspect further comprises a targeting moiety to which one or more of the conjugates are covalently bonded via an A group. In turn, the conjugate of the second aspect can move to the target site in a directed manner under the mediation of the targeting movement of the targeting moiety.

In the third aspect of the present disclosure, provided herein is a conjugate. According to the embodiments of the present disclosure, the conjugate comprises a targeting moiety and one or more of the above compounds, wherein the compound is covalently bonded to the targeting moiety via an A group. In turn, the conjugate according to the third aspect can move to the target site in a directed manner under the mediation of the targeting movement of the targeting moiety.

According to the embodiments of the present disclosure, the drug is selected from at least one of eribulin, monomethyl auristatin E, and SN-38, which have the following structures: 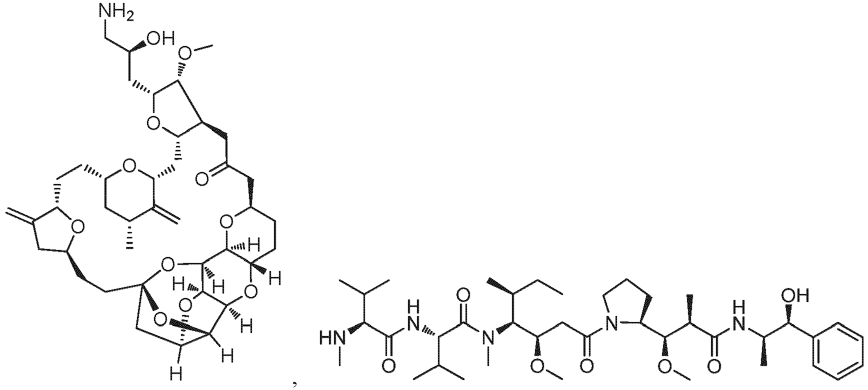 and 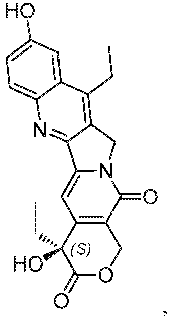 or an isotopic variant thereof.

According to the embodiments of the present disclosure, the conjugate of the second aspect is selected from the following specific compounds, or tautomers, stereoisomers, pharmaceutically acceptable salts or isotopic variants thereof, wherein the compounds are represented by the structure shown in formula (VI): 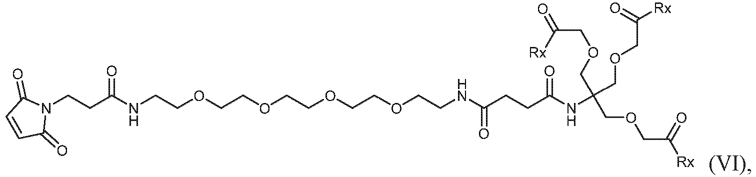 wherein Rx is 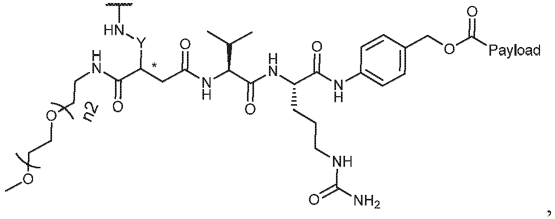 and n2, *, Y and payload are as defined in Table 1 below:

**Table 1: Detailed definitions of n2, *, Y and payload.**

| n2 | Chirality of the carbon atom at * | Y | Payload |
|---|---|---|---|
| 5 | S | Bond | 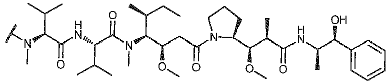 (MMAE) |
| 5 | R | Bond | MMAE |
| 5 | S | 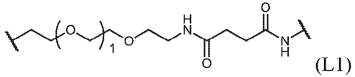 | MMAE |
| 5 | R | L1 | MMAE |
| 5 | S | 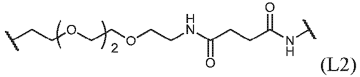 | MMAE |
| 5 | R | L2 | MMAE |
| 5 | S | 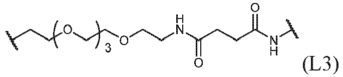 | MMAE |
| 5 | R | L3 | MMAE |
| 5 | S | 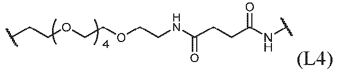 | MMAE |
| 5 | R | L4 | MMAE |
| 5 | S | 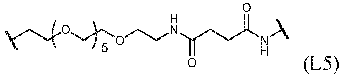 | MMAE |
| 5 | R | L5 | MMAE |
| 5 | S | 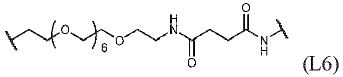 | MMAE |
| 5 | R | L6 | MMAE |
| 5 | S | 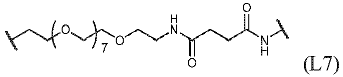 | MMAE |
| 5 | R | L7 | MMAE |
| 5 | S | 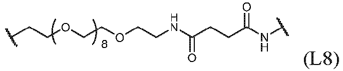 | MMAE |
| 5 | R | L8 | MMAE |
| 5 | S | 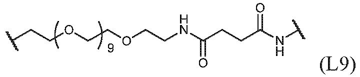 | MMAE |
| 5 | R | L9 | MMAE |
| 5 | S | 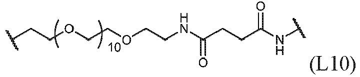 | MMAE |
| 5 | R | L10 | MMAE |
| 5 | S | 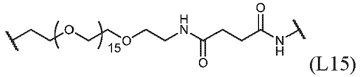 | MMAE |
| 5 | R | L15 | MMAE |
| 5 | S | 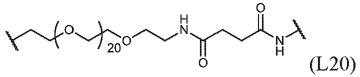 | MMAE |
| 5 | R | L20 | MMAE |
| 5 | S | Bond | 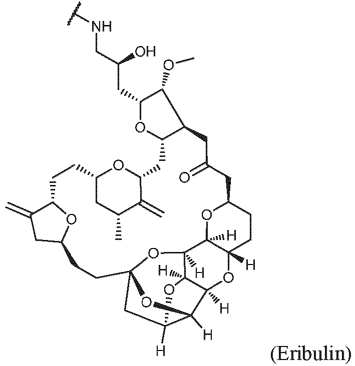 |
| 5 | R | Bond | Eribulin |
| 5 | S | L1 | Eribulin |
| 5 | R | L1 | Eribulin |
| 5 | S | L2 | Eribulin |
| 5 | R | L2 | Eribulin |
| 5 | S | L3 | Eribulin |
| 5 | R | L3 | Eribulin |
| 5 | S | L4 | Eribulin |
| 5 | R | L4 | Eribulin |
| 5 | S | L5 | Eribulin |
| 5 | R | L5 | Eribulin |
| 5 | S | L6 | Eribulin |
| 5 | R | L6 | Eribulin |
| 5 | S | L7 | Eribulin |
| 5 | R | L7 | Eribulin |
| 5 | S | L8 | Eribulin |
| 5 | R | L8 | Eribulin |
| 5 | S | L9 | Eribulin |
| 5 | R | L9 | Eribulin |
| 5 | S | L10 | Eribulin |
| 5 | R | L10 | Eribulin |
| 5 | S | L15 | Eribulin |
| 5 | R | L15 | Eribulin |
| 5 | S | L20 | Eribulin |
| 5 | R | L20 | Eribulin |
| 5 | S | Bond | 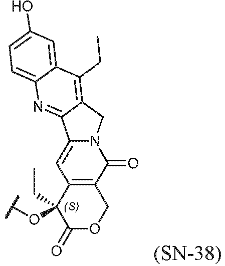 |
| 5 | R | Bond | SN-38 |
| 5 | S | L1 | SN-38 |
| 5 | R | L1 | SN-38 |
| 5 | S | L2 | SN-38 |
| 5 | R | L2 | SN-38 |
| 5 | S | L3 | SN-38 |
| 5 | R | L3 | SN-38 |
| 5 | S | L4 | SN-38 |
| 5 | R | L4 | SN-38 |
| 5 | S | L5 | SN-38 |
| 5 | R | L5 | SN-38 |
| 5 | S | L6 | SN-38 |
| 5 | R | L6 | SN-38 |
| 5 | S | L7 | SN-38 |
| 5 | R | L7 | SN-38 |
| 5 | S | L8 | SN-38 |
| 5 | R | L8 | SN-38 |
| 5 | S | L9 | SN-38 |
| 5 | R | L9 | SN-38 |
| 5 | S | L10 | SN-38 |
| 5 | R | L10 | SN-38 |
| 5 | S | L15 | SN-38 |
| 5 | R | L15 | SN-38 |
| 5 | S | L20 | SN-38 |
| 5 | R | L20 | SN-38 |
| 6 | S | Bond | MMAE |
| 6 | R | Bond | MMAE |
| 6 | S | L1 | MMAE |
| 6 | R | L1 | MMAE |
| 6 | S | L2 | MMAE |
| 6 | R | L2 | MMAE |
| 6 | S | L3 | MMAE |
| 6 | R | L3 | MMAE |
| 6 | S | L4 | MMAE |
| 6 | R | L4 | MMAE |
| 6 | S | L5 | MMAE |
| 6 | R | L5 | MMAE |
| 6 | S | L6 | MMAE |
| 6 | R | L6 | MMAE |
| 6 | S | L7 | MMAE |
| 6 | R | L7 | MMAE |
| 6 | S | L8 | MMAE |
| 6 | R | L8 | MMAE |
| 6 | S | L9 | MMAE |
| 6 | R | L9 | MMAE |
| 6 | S | L10 | MMAE |
| 6 | R | L10 | MMAE |
| 6 | S | L15 | MMAE |
| 6 | R | L15 | MMAE |
| 6 | S | L20 | MMAE |
| 6 | R | L20 | MMAE |
| 6 | S | Bond | Eribulin |
| 6 | R | Bond | Eribulin |
| 6 | S | L1 | Eribulin |
| 6 | R | L1 | Eribulin |
| 6 | S | L2 | Eribulin |
| 6 | R | L2 | Eribulin |
| 6 | S | L3 | Eribulin |
| 6 | R | L3 | Eribulin |
| 6 | S | L4 | Eribulin |
| 6 | R | L4 | Eribulin |
| 6 | S | L5 | Eribulin |
| 6 | R | L5 | Eribulin |
| 6 | S | L6 | Eribulin |
| 6 | R | L6 | Eribulin |
| 6 | S | L7 | Eribulin |
| 6 | R | L7 | Eribulin |
| 6 | S | L8 | Eribulin |
| 6 | R | L8 | Eribulin |
| 6 | S | L9 | Eribulin |
| 6 | R | L9 | Eribulin |
| 6 | S | L10 | Eribulin |
| 6 | R | L10 | Eribulin |
| 6 | S | L15 | Eribulin |
| 6 | R | L15 | Eribulin |
| 6 | S | L20 | Eribulin |
| 6 | R | L20 | Eribulin |
| 6 | S | Bond | SN-38 |
| 6 | R | Bond | SN-38 |
| 6 | S | L1 | SN-38 |
| 6 | R | L1 | SN-38 |
| 6 | S | L2 | SN-38 |
| 6 | R | L2 | SN-38 |
| 6 | S | L3 | SN-38 |
| 6 | R | L3 | SN-38 |
| 6 | S | L4 | SN-38 |
| 6 | R | L4 | SN-38 |
| 6 | S | L5 | SN-38 |
| 6 | R | L5 | SN-38 |
| 6 | S | L6 | SN-38 |
| 6 | R | L6 | SN-38 |
| 6 | S | L7 | SN-38 |
| 6 | R | L7 | SN-38 |
| 6 | S | L8 | SN-38 |
| 6 | R | L8 | SN-38 |
| 6 | S | L9 | SN-38 |
| 6 | R | L9 | SN-38 |
| 6 | S | L10 | SN-38 |
| 6 | R | L10 | SN-38 |
| 6 | S | L15 | SN-38 |
| 6 | R | L15 | SN-38 |
| 6 | S | L20 | SN-38 |
| 6 | R | L20 | SN-38 |
| 7 | S | Bond | MMAE |
| 7 | R | Bond | MMAE |
| 7 | S | L1 | MMAE |
| 7 | R | L1 | MMAE |
| 7 | S | L2 | MMAE |
| 7 | R | L2 | MMAE |
| 7 | S | L3 | MMAE |
| 7 | R | L3 | MMAE |
| 7 | S | L4 | MMAE |
| 7 | R | L4 | MMAE |
| 7 | S | L5 | MMAE |
| 7 | R | L5 | MMAE |
| 7 | S | L6 | MMAE |
| 7 | R | L6 | MMAE |
| 7 | S | L7 | MMAE |
| 7 | R | L7 | MMAE |
| 7 | S | L8 | MMAE |
| 7 | R | L8 | MMAE |
| 7 | S | L9 | MMAE |
| 7 | R | L9 | MMAE |
| 7 | S | L10 | MMAE |
| 7 | R | L10 | MMAE |
| 7 | S | L15 | MMAE |
| 7 | R | L15 | MMAE |
| 7 | S | L20 | MMAE |
| 7 | R | L20 | MMAE |
| 7 | S | Bond | Eribulin |
| 7 | R | Bond | Eribulin |
| 7 | S | L1 | Eribulin |
| 7 | R | L1 | Eribulin |
| 7 | S | L2 | Eribulin |
| 7 | R | L2 | Eribulin |
| 7 | S | L3 | Eribulin |
| 7 | R | L3 | Eribulin |
| 7 | S | L4 | Eribulin |
| 7 | R | L4 | Eribulin |
| 7 | S | L5 | Eribulin |
| 7 | R | L5 | Eribulin |
| 7 | S | L6 | Eribulin |
| 7 | R | L6 | Eribulin |
| 7 | S | L7 | Eribulin |
| 7 | R | L7 | Eribulin |
| 7 | S | L8 | Eribulin |
| 7 | R | L8 | Eribulin |
| 7 | S | L9 | Eribulin |
| 7 | R | L9 | Eribulin |
| 7 | S | L10 | Eribulin |
| 7 | R | L10 | Eribulin |
| 7 | S | L15 | Eribulin |
| 7 | R | L15 | Eribulin |
| 7 | S | L20 | Eribulin |
| 7 | R | L20 | Eribulin |
| 7 | S | Bond | SN-38 |
| 7 | R | Bond | SN-38 |
| 7 | S | L1 | SN-38 |
| 7 | R | L1 | SN-38 |
| 7 | S | L2 | SN-38 |
| 7 | R | L2 | SN-38 |
| 7 | S | L3 | SN-38 |
| 7 | R | L3 | SN-38 |
| 7 | S | L4 | SN-38 |
| 7 | R | L4 | SN-38 |
| 7 | S | L5 | SN-38 |
| 7 | R | L5 | SN-38 |
| 7 | S | L6 | SN-38 |
| 7 | R | L6 | SN-38 |
| 7 | S | L7 | SN-38 |
| 7 | R | L7 | SN-38 |
| 7 | S | L8 | SN-38 |
| 7 | R | L8 | SN-38 |
| 7 | S | L9 | SN-38 |
| 7 | R | L9 | SN-38 |
| 7 | S | L10 | SN-38 |
| 7 | R | L10 | SN-38 |
| 7 | S | L15 | SN-38 |
| 7 | R | L15 | SN-38 |
| 7 | S | L20 | SN-38 |
| 7 | R | L20 | SN-38 |
| 8 | S | Bond | MMAE |
| 8 | R | Bond | MMAE |
| 8 | S | L1 | MMAE |
| 8 | R | L1 | MMAE |
| 8 | S | L2 | MMAE |
| 8 | R | L2 | MMAE |
| 8 | S | L3 | MMAE |
| 8 | R | L3 | MMAE |
| 8 | S | L4 | MMAE |
| 8 | R | L4 | MMAE |
| 8 | S | L5 | MMAE |
| 8 | R | L5 | MMAE |
| 8 | S | L6 | MMAE |
| 8 | R | L6 | MMAE |
| 8 | S | L7 | MMAE |
| 8 | R | L7 | MMAE |
| 8 | S | L8 | MMAE |
| 8 | R | L8 | MMAE |
| 8 | S | L9 | MMAE |
| 8 | R | L9 | MMAE |
| 8 | S | L10 | MMAE |
| 8 | R | L10 | MMAE |
| 8 | S | L15 | MMAE |
| 8 | R | L15 | MMAE |
| 8 | S | L20 | MMAE |
| 8 | R | L20 | MMAE |
| 8 | S | Bond | Eribulin |
| 8 | R | Bond | Eribulin |
| 8 | S | L1 | Eribulin |
| 8 | R | L1 | Eribulin |
| 8 | S | L2 | Eribulin |
| 8 | R | L2 | Eribulin |
| 8 | S | L3 | Eribulin |
| 8 | R | L3 | Eribulin |
| 8 | S | L4 | Eribulin |
| 8 | R | L4 | Eribulin |
| 8 | S | L5 | Eribulin |
| 8 | R | L5 | Eribulin |
| 8 | S | L6 | Eribulin |
| 8 | R | L6 | Eribulin |
| 8 | S | L7 | Eribulin |
| 8 | R | L7 | Eribulin |
| 8 | S | L8 | Eribulin |
| 8 | R | L8 | Eribulin |
| 8 | S | L9 | Eribulin |
| 8 | R | L9 | Eribulin |
| 8 | S | L10 | Eribulin |
| 8 | R | L10 | Eribulin |
| 8 | S | L15 | Eribulin |
| 8 | R | L15 | Eribulin |
| 8 | S | L20 | Eribulin |
| 8 | R | L20 | Eribulin |
| 8 | S | Bond | SN-38 |
| 8 | R | Bond | SN-38 |
| 8 | S | L1 | SN-38 |
| 8 | R | L1 | SN-38 |
| 8 | S | L2 | SN-38 |
| 8 | R | L2 | SN-38 |
| 8 | S | L3 | SN-38 |
| 8 | R | L3 | SN-38 |
| 8 | S | L4 | SN-38 |
| 8 | R | L4 | SN-38 |
| 8 | S | L5 | SN-38 |
| 8 | R | L5 | SN-38 |
| 8 | S | L6 | SN-38 |
| 8 | R | L6 | SN-38 |
| 8 | S | L7 | SN-38 |
| 8 | R | L7 | SN-38 |
| 8 | S | L8 | SN-38 |
| 8 | R | L8 | SN-38 |
| 8 | S | L9 | SN-38 |
| 8 | R | L9 | SN-38 |
| 8 | S | L10 | SN-38 |
| 8 | R | L10 | SN-38 |
| 8 | S | L15 | SN-38 |
| 8 | R | L15 | SN-38 |
| 8 | S | L20 | SN-38 |
| 8 | R | L20 | SN-38 |
| 9 | S | Bond | MMAE |
| 9 | R | Bond | MMAE |
| 9 | S | L1 | MMAE |
| 9 | R | L1 | MMAE |
| 9 | S | L2 | MMAE |
| 9 | R | L2 | MMAE |
| 9 | S | L3 | MMAE |
| 9 | R | L3 | MMAE |
| 9 | S | L4 | MMAE |
| 9 | R | L4 | MMAE |
| 9 | S | L5 | MMAE |
| 9 | R | L5 | MMAE |
| 9 | S | L6 | MMAE |
| 9 | R | L6 | MMAE |
| 9 | S | L7 | MMAE |
| 9 | R | L7 | MMAE |
| 9 | S | L8 | MMAE |
| 9 | R | L8 | MMAE |
| 9 | S | L9 | MMAE |
| 9 | R | L9 | MMAE |
| 9 | S | L10 | MMAE |
| 9 | R | L10 | MMAE |
| 9 | S | L15 | MMAE |
| 9 | R | L15 | MMAE |
| 9 | S | L20 | MMAE |
| 9 | R | L20 | MMAE |
| 9 | S | Bond | Eribulin |
| 9 | R | Bond | Eribulin |
| 9 | S | L1 | Eribulin |
| 9 | R | L1 | Eribulin |
| 9 | S | L2 | Eribulin |
| 9 | R | L2 | Eribulin |
| 9 | S | L3 | Eribulin |
| 9 | R | L3 | Eribulin |
| 9 | S | L4 | Eribulin |
| 9 | R | L4 | Eribulin |
| 9 | S | L5 | Eribulin |
| 9 | R | L5 | Eribulin |
| 9 | S | L6 | Eribulin |
| 9 | R | L6 | Eribulin |
| 9 | S | L7 | Eribulin |
| 9 | R | L7 | Eribulin |
| 9 | S | L8 | Eribulin |
| 9 | R | L8 | Eribulin |
| 9 | S | L9 | Eribulin |
| 9 | R | L9 | Eribulin |
| 9 | S | L10 | Eribulin |
| 9 | R | L10 | Eribulin |
| 9 | S | L15 | Eribulin |
| 9 | R | L15 | Eribulin |
| 9 | S | L20 | Eribulin |
| 9 | R | L20 | Eribulin |
| 9 | S | Bond | SN-38 |
| 9 | R | Bond | SN-38 |
| 9 | S | L1 | SN-38 |
| 9 | R | L1 | SN-38 |
| 9 | S | L2 | SN-38 |
| 9 | R | L2 | SN-38 |
| 9 | S | L3 | SN-38 |
| 9 | R | L3 | SN-38 |
| 9 | S | L4 | SN-38 |
| 9 | R | L4 | SN-38 |
| 9 | S | L5 | SN-38 |
| 9 | R | L5 | SN-38 |
| 9 | S | L6 | SN-38 |
| 9 | R | L6 | SN-38 |
| 9 | S | L7 | SN-38 |
| 9 | R | L7 | SN-38 |
| 9 | S | L8 | SN-38 |
| 9 | R | L8 | SN-38 |
| 9 | S | L9 | SN-38 |
| 9 | R | L9 | SN-38 |
| 9 | S | L10 | SN-38 |
| 9 | R | L10 | SN-38 |
| 9 | S | L15 | SN-38 |
| 9 | R | L15 | SN-38 |
| 9 | S | L20 | SN-38 |
| 9 | R | L20 | SN-38 |
| 10 | S | Bond | MMAE |
| 10 | R | Bond | MMAE |
| 10 | S | L1 | MMAE |
| 10 | R | L1 | MMAE |
| 10 | S | L2 | MMAE |
| 10 | R | L2 | MMAE |
| 10 | S | L3 | MMAE |
| 10 | R | L3 | MMAE |
| 10 | S | L4 | MMAE |
| 10 | R | L4 | MMAE |
| 10 | S | L5 | MMAE |
| 10 | R | L5 | MMAE |
| 10 | S | L6 | MMAE |
| 10 | R | L6 | MMAE |
| 10 | S | L7 | MMAE |
| 10 | R | L7 | MMAE |
| 10 | S | L8 | MMAE |
| 10 | R | L8 | MMAE |
| 10 | S | L9 | MMAE |
| 10 | R | L9 | MMAE |
| 10 | S | L10 | MMAE |
| 10 | R | L10 | MMAE |
| 10 | S | L15 | MMAE |
| 10 | R | L15 | MMAE |
| 10 | S | L20 | MMAE |
| 10 | R | L20 | MMAE |
| 10 | S | Bond | Eribulin |
| 10 | R | Bond | Eribulin |
| 10 | S | L1 | Eribulin |
| 10 | R | L1 | Eribulin |
| 10 | S | L2 | Eribulin |
| 10 | R | L2 | Eribulin |
| 10 | S | L3 | Eribulin |
| 10 | R | L3 | Eribulin |
| 10 | S | L4 | Eribulin |
| 10 | R | L4 | Eribulin |
| 10 | S | L5 | Eribulin |
| 10 | R | L5 | Eribulin |
| 10 | S | L6 | Eribulin |
| 10 | R | L6 | Eribulin |
| 10 | S | L7 | Eribulin |
| 10 | R | L7 | Eribulin |
| 10 | S | L8 | Eribulin |
| 10 | R | L8 | Eribulin |
| 10 | S | L9 | Eribulin |
| 10 | R | L9 | Eribulin |
| 10 | S | L10 | Eribulin |
| 10 | R | L10 | Eribulin |
| 10 | S | L15 | Eribulin |
| 10 | R | L15 | Eribulin |
| 10 | S | L20 | Eribulin |
| 10 | R | L20 | Eribulin |
| 10 | S | Bond | SN-38 |
| 10 | R | Bond | SN-38 |
| 10 | S | L1 | SN-38 |
| 10 | R | L1 | SN-38 |
| 10 | S | L2 | SN-38 |
| 10 | R | L2 | SN-38 |
| 10 | S | L3 | SN-38 |
| 10 | R | L3 | SN-38 |
| 10 | S | L4 | SN-38 |
| 10 | R | L4 | SN-38 |
| 10 | S | L5 | SN-38 |
| 10 | R | L5 | SN-38 |
| 10 | S | L6 | SN-38 |
| 10 | R | L6 | SN-38 |
| 10 | S | L7 | SN-38 |
| 10 | R | L7 | SN-38 |
| 10 | S | L8 | SN-38 |
| 10 | R | L8 | SN-38 |
| 10 | S | L9 | SN-38 |
| 10 | R | L9 | SN-38 |
| 10 | S | L10 | SN-38 |
| 10 | R | L10 | SN-38 |
| 10 | S | L15 | SN-38 |
| 10 | R | L15 | SN-38 |
| 10 | S | L20 | SN-38 |
| 10 | R | L20 | SN-38 |
| 11 | S | Bond | MMAE |
| 11 | R | Bond | MMAE |
| 11 | S | L1 | MMAE |
| 11 | R | L1 | MMAE |
| 11 | S | L2 | MMAE |
| 11 | R | L2 | MMAE |
| 11 | S | L3 | MMAE |
| 11 | R | L3 | MMAE |
| 11 | S | L4 | MMAE |
| 11 | R | L4 | MMAE |
| 11 | S | L5 | MMAE |
| 11 | R | L5 | MMAE |
| 11 | S | L6 | MMAE |
| 11 | R | L6 | MMAE |
| 11 | S | L7 | MMAE |
| 11 | R | L7 | MMAE |
| 11 | S | L8 | MMAE |
| 11 | R | L8 | MMAE |
| 11 | S | L9 | MMAE |
| 11 | R | L9 | MMAE |
| 11 | S | L10 | MMAE |
| 11 | R | L10 | MMAE |
| 11 | S | L15 | MMAE |
| 11 | R | L15 | MMAE |
| 11 | S | L20 | MMAE |
| 11 | R | L20 | MMAE |
| 11 | S | Bond | Eribulin |
| 11 | R | Bond | Eribulin |
| 11 | S | L1 | Eribulin |
| 11 | R | L1 | Eribulin |
| 11 | S | L2 | Eribulin |
| 11 | R | L2 | Eribulin |
| 11 | S | L3 | Eribulin |
| 11 | R | L3 | Eribulin |
| 11 | S | L4 | Eribulin |
| 11 | R | L4 | Eribulin |
| 11 | S | L5 | Eribulin |
| 11 | R | L5 | Eribulin |
| 11 | S | L6 | Eribulin |
| 11 | R | L6 | Eribulin |
| 11 | S | L7 | Eribulin |
| 11 | R | L7 | Eribulin |
| 11 | S | L8 | Eribulin |
| 11 | R | L8 | Eribulin |
| 11 | S | L9 | Eribulin |
| 11 | R | L9 | Eribulin |
| 11 | S | L10 | Eribulin |
| 11 | R | L10 | Eribulin |
| 11 | S | L15 | Eribulin |
| 11 | R | L15 | Eribulin |
| 11 | S | L20 | Eribulin |
| 11 | R | L20 | Eribulin |
| 11 | S | Bond | SN-38 |
| 11 | R | Bond | SN-38 |
| 11 | S | L1 | SN-38 |
| 11 | R | L1 | SN-38 |
| 11 | S | L2 | SN-38 |
| 11 | R | L2 | SN-38 |
| 11 | S | L3 | SN-38 |
| 11 | R | L3 | SN-38 |
| 11 | S | L4 | SN-38 |
| 11 | R | L4 | SN-38 |
| 11 | S | L5 | SN-38 |
| 11 | R | L5 | SN-38 |
| 11 | S | L6 | SN-38 |
| 11 | R | L6 | SN-38 |
| 11 | S | L7 | SN-38 |
| 11 | R | L7 | SN-38 |
| 11 | S | L8 | SN-38 |
| 11 | R | L8 | SN-38 |
| 11 | S | L9 | SN-38 |
| 11 | R | L9 | SN-38 |
| 11 | S | L10 | SN-38 |
| 11 | R | L10 | SN-38 |
| 11 | S | L15 | SN-38 |
| 11 | R | L15 | SN-38 |
| 11 | S | L20 | SN-38 |
| 11 | R | L20 | SN-38 |
| 12 | S | Bond | MMAE |
| 12 | R | Bond | MMAE |
| 12 | S | L1 | MMAE |
| 12 | R | L1 | MMAE |
| 12 | S | L2 | MMAE |
| 12 | R | L2 | MMAE |
| 12 | S | L3 | MMAE |
| 12 | R | L3 | MMAE |
| 12 | S | L4 | MMAE |
| 12 | R | L4 | MMAE |
| 12 | S | L5 | MMAE |
| 12 | R | L5 | MMAE |
| 12 | S | L6 | MMAE |
| 12 | R | L6 | MMAE |
| 12 | S | L7 | MMAE |
| 12 | R | L7 | MMAE |
| 12 | S | L8 | MMAE |
| 12 | R | L8 | MMAE |
| 12 | S | L9 | MMAE |
| 12 | R | L9 | MMAE |
| 12 | S | L10 | MMAE |
| 12 | R | L10 | MMAE |
| 12 | S | L15 | MMAE |
| 12 | R | L15 | MMAE |
| 12 | S | L20 | MMAE |
| 12 | R | L20 | MMAE |
| 12 | S | Bond | Eribulin |
| 12 | R | Bond | Eribulin |
| 12 | S | L1 | Eribulin |
| 12 | R | L1 | Eribulin |
| 12 | S | L2 | Eribulin |
| 12 | R | L2 | Eribulin |
| 12 | S | L3 | Eribulin |
| 12 | R | L3 | Eribulin |
| 12 | S | L4 | Eribulin |
| 12 | R | L4 | Eribulin |
| 12 | S | L5 | Eribulin |
| 12 | R | L5 | Eribulin |
| 12 | S | L6 | Eribulin |
| 12 | R | L6 | Eribulin |
| 12 | S | L7 | Eribulin |
| 12 | R | L7 | Eribulin |
| 12 | S | L8 | Eribulin |
| 12 | R | L8 | Eribulin |
| 12 | S | L9 | Eribulin |
| 12 | R | L9 | Eribulin |
| 12 | S | L10 | Eribulin |
| 12 | R | L10 | Eribulin |
| 12 | S | L15 | Eribulin |
| 12 | R | L15 | Eribulin |
| 12 | S | L20 | Eribulin |
| 12 | R | L20 | Eribulin |
| 12 | S | Bond | SN-38 |
| 12 | R | Bond | SN-38 |
| 12 | S | L1 | SN-38 |
| 12 | R | L1 | SN-38 |
| 12 | S | L2 | SN-38 |
| 12 | R | L2 | SN-38 |
| 12 | S | L3 | SN-38 |
| 12 | R | L3 | SN-38 |
| 12 | S | L4 | SN-38 |
| 12 | R | L4 | SN-38 |
| 12 | S | L5 | SN-38 |
| 12 | R | L5 | SN-38 |
| 12 | S | L6 | SN-38 |
| 12 | R | L6 | SN-38 |
| 12 | S | L7 | SN-38 |
| 12 | R | L7 | SN-38 |
| 12 | S | L8 | SN-38 |
| 12 | R | L8 | SN-38 |
| 12 | S | L9 | SN-38 |
| 12 | R | L9 | SN-38 |
| 12 | S | L10 | SN-38 |
| 12 | R | L10 | SN-38 |
| 12 | S | L15 | SN-38 |
| 12 | R | L15 | SN-38 |
| 12 | S | L20 | SN-38 |
| 12 | R | L20 | SN-38 |
| 13 | S | Bond | MMAE |
| 13 | R | Bond | MMAE |
| 13 | S | L1 | MMAE |
| 13 | R | L1 | MMAE |
| 13 | S | L2 | MMAE |
| 13 | R | L2 | MMAE |
| 13 | S | L3 | MMAE |
| 13 | R | L3 | MMAE |
| 13 | S | L4 | MMAE |
| 13 | R | L4 | MMAE |
| 13 | S | L5 | MMAE |
| 13 | R | L5 | MMAE |
| 13 | S | L6 | MMAE |
| 13 | R | L6 | MMAE |
| 13 | S | L7 | MMAE |
| 13 | R | L7 | MMAE |
| 13 | S | L8 | MMAE |
| 13 | R | L8 | MMAE |
| 13 | S | L9 | MMAE |
| 13 | R | L9 | MMAE |
| 13 | S | L10 | MMAE |
| 13 | R | L10 | MMAE |
| 13 | S | L15 | MMAE |
| 13 | R | L15 | MMAE |
| 13 | S | L20 | MMAE |
| 13 | R | L20 | MMAE |
| 13 | S | Bond | Eribulin |
| 13 | R | Bond | Eribulin |
| 13 | S | L1 | Eribulin |
| 13 | R | L1 | Eribulin |
| 13 | S | L2 | Eribulin |
| 13 | R | L2 | Eribulin |
| 13 | S | L3 | Eribulin |
| 13 | R | L3 | Eribulin |
| 13 | S | L4 | Eribulin |
| 13 | R | L4 | Eribulin |
| 13 | S | L5 | Eribulin |
| 13 | R | L5 | Eribulin |
| 13 | S | L6 | Eribulin |
| 13 | R | L6 | Eribulin |
| 13 | S | L7 | Eribulin |
| 13 | R | L7 | Eribulin |
| 13 | S | L8 | Eribulin |
| 13 | R | L8 | Eribulin |
| 13 | S | L9 | Eribulin |
| 13 | R | L9 | Eribulin |
| 13 | S | L10 | Eribulin |
| 13 | R | L10 | Eribulin |
| 13 | S | L15 | Eribulin |
| 13 | R | L15 | Eribulin |
| 13 | S | L20 | Eribulin |
| 13 | R | L20 | Eribulin |
| 13 | S | Bond | SN-38 |
| 13 | R | Bond | SN-38 |
| 13 | S | L1 | SN-38 |
| 13 | R | L1 | SN-38 |
| 13 | S | L2 | SN-38 |
| 13 | R | L2 | SN-38 |
| 13 | S | L3 | SN-38 |
| 13 | R | L3 | SN-38 |
| 13 | S | L4 | SN-38 |
| 13 | R | L4 | SN-38 |
| 13 | S | L5 | SN-38 |
| 13 | R | L5 | SN-38 |
| 13 | S | L6 | SN-38 |
| 13 | R | L6 | SN-38 |
| 13 | S | L7 | SN-38 |
| 13 | R | L7 | SN-38 |
| 13 | S | L8 | SN-38 |
| 13 | R | L8 | SN-38 |
| 13 | S | L9 | SN-38 |
| 13 | R | L9 | SN-38 |
| 13 | S | L10 | SN-38 |
| 13 | R | L10 | SN-38 |
| 13 | S | L15 | SN-38 |
| 13 | R | L15 | SN-38 |
| 13 | S | L20 | SN-38 |
| 13 | R | L20 | SN-38 |
| 14 | S | Bond | MMAE |
| 14 | R | Bond | MMAE |
| 14 | S | L1 | MMAE |
| 14 | R | L1 | MMAE |
| 14 | S | L2 | MMAE |
| 14 | R | L2 | MMAE |
| 14 | S | L3 | MMAE |
| 14 | R | L3 | MMAE |
| 14 | S | L4 | MMAE |
| 14 | R | L4 | MMAE |
| 14 | S | L5 | MMAE |
| 14 | R | L5 | MMAE |
| 14 | S | L6 | MMAE |
| 14 | R | L6 | MMAE |
| 14 | S | L7 | MMAE |
| 14 | R | L7 | MMAE |
| 14 | S | L8 | MMAE |
| 14 | R | L8 | MMAE |
| 14 | S | L9 | MMAE |
| 14 | R | L9 | MMAE |
| 14 | S | L10 | MMAE |
| 14 | R | L10 | MMAE |
| 14 | S | L15 | MMAE |
| 14 | R | L15 | MMAE |
| 14 | S | L20 | MMAE |
| 14 | R | L20 | MMAE |
| 14 | S | Bond | Eribulin |
| 14 | R | Bond | Eribulin |
| 14 | S | L1 | Eribulin |
| 14 | R | L1 | Eribulin |
| 14 | S | L2 | Eribulin |
| 14 | R | L2 | Eribulin |
| 14 | S | L3 | Eribulin |
| 14 | R | L3 | Eribulin |
| 14 | S | L4 | Eribulin |
| 14 | R | L4 | Eribulin |
| 14 | S | L5 | Eribulin |
| 14 | R | L5 | Eribulin |
| 14 | S | L6 | Eribulin |
| 14 | R | L6 | Eribulin |
| 14 | S | L7 | Eribulin |
| 14 | R | L7 | Eribulin |
| 14 | S | L8 | Eribulin |
| 14 | R | L8 | Eribulin |
| 14 | S | L9 | Eribulin |
| 14 | R | L9 | Eribulin |
| 14 | S | L10 | Eribulin |
| 14 | R | L10 | Eribulin |
| 14 | S | L15 | Eribulin |
| 14 | R | L15 | Eribulin |
| 14 | S | L20 | Eribulin |
| 14 | R | L20 | Eribulin |
| 14 | S | Bond | SN-38 |
| 14 | R | Bond | SN-38 |
| 14 | S | L1 | SN-38 |
| 14 | R | L1 | SN-38 |
| 14 | S | L2 | SN-38 |
| 14 | R | L2 | SN-38 |
| 14 | S | L3 | SN-38 |
| 14 | R | L3 | SN-38 |
| 14 | S | L4 | SN-38 |
| 14 | R | L4 | SN-38 |
| 14 | S | L5 | SN-38 |
| 14 | R | L5 | SN-38 |
| 14 | S | L6 | SN-38 |
| 14 | R | L6 | SN-38 |
| 14 | S | L7 | SN-38 |
| 14 | R | L7 | SN-38 |
| 14 | S | L8 | SN-38 |
| 14 | R | L8 | SN-38 |
| 14 | S | L9 | SN-38 |
| 14 | R | L9 | SN-38 |
| 14 | S | L10 | SN-38 |
| 14 | R | L10 | SN-38 |
| 14 | S | L15 | SN-38 |
| 14 | R | L15 | SN-38 |
| 14 | S | L20 | SN-38 |
| 14 | R | L20 | SN-38 |
| 15 | S | Bond | MMAE |
| 15 | R | Bond | MMAE |
| 15 | S | L1 | MMAE |
| 15 | R | L1 | MMAE |
| 15 | S | L2 | MMAE |
| 15 | R | L2 | MMAE |
| 15 | S | L3 | MMAE |
| 15 | R | L3 | MMAE |
| 15 | S | L4 | MMAE |
| 15 | R | L4 | MMAE |
| 15 | S | L5 | MMAE |
| 15 | R | L5 | MMAE |
| 15 | S | L6 | MMAE |
| 15 | R | L6 | MMAE |
| 15 | S | L7 | MMAE |
| 15 | R | L7 | MMAE |
| 15 | S | L8 | MMAE |
| 15 | R | L8 | MMAE |
| 15 | S | L9 | MMAE |
| 15 | R | L9 | MMAE |
| 15 | S | L10 | MMAE |
| 15 | R | L10 | MMAE |
| 15 | S | L15 | MMAE |
| 15 | R | L15 | MMAE |
| 15 | S | L20 | MMAE |
| 15 | R | L20 | MMAE |
| 15 | S | Bond | Eribulin |
| 15 | R | Bond | Eribulin |
| 15 | S | L1 | Eribulin |
| 15 | R | L1 | Eribulin |
| 15 | S | L2 | Eribulin |
| 15 | R | L2 | Eribulin |
| 15 | S | L3 | Eribulin |
| 15 | R | L3 | Eribulin |
| 15 | S | L4 | Eribulin |
| 15 | R | L4 | Eribulin |
| 15 | S | L5 | Eribulin |
| 15 | R | L5 | Eribulin |
| 15 | S | L6 | Eribulin |
| 15 | R | L6 | Eribulin |
| 15 | S | L7 | Eribulin |
| 15 | R | L7 | Eribulin |
| 15 | S | L8 | Eribulin |
| 15 | R | L8 | Eribulin |
| 15 | S | L9 | Eribulin |
| 15 | R | L9 | Eribulin |
| 15 | S | L10 | Eribulin |
| 15 | R | L10 | Eribulin |
| 15 | S | L15 | Eribulin |
| 15 | R | L15 | Eribulin |
| 15 | S | L20 | Eribulin |
| 15 | R | L20 | Eribulin |
| 15 | S | Bond | SN-38 |
| 15 | R | Bond | SN-38 |
| 15 | S | L1 | SN-38 |
| 15 | R | L1 | SN-38 |
| 15 | S | L2 | SN-38 |
| 15 | R | L2 | SN-38 |
| 15 | S | L3 | SN-38 |
| 15 | R | L3 | SN-38 |
| 15 | S | L4 | SN-38 |
| 15 | R | L4 | SN-38 |
| 15 | S | L5 | SN-38 |
| 15 | R | L5 | SN-38 |
| 15 | S | L6 | SN-38 |
| 15 | R | L6 | SN-38 |
| 15 | S | L7 | SN-38 |
| 15 | R | L7 | SN-38 |
| 15 | S | L8 | SN-38 |
| 15 | R | L8 | SN-38 |
| 15 | S | L9 | SN-38 |
| 15 | R | L9 | SN-38 |
| 15 | S | L10 | SN-38 |
| 15 | R | L10 | SN-38 |
| 15 | S | L15 | SN-38 |
| 15 | R | L15 | SN-38 |
| 15 | S | L20 | SN-38 |
| 15 | R | L20 | SN-38 |
| 16 | S | Bond | MMAE |
| 16 | R | Bond | MMAE |
| 16 | S | L1 | MMAE |
| 16 | R | L1 | MMAE |
| 16 | S | L2 | MMAE |
| 16 | R | L2 | MMAE |
| 16 | S | L3 | MMAE |
| 16 | R | L3 | MMAE |
| 16 | S | L4 | MMAE |
| 16 | R | L4 | MMAE |
| 16 | S | L5 | MMAE |
| 16 | R | L5 | MMAE |
| 16 | S | L6 | MMAE |
| 16 | R | L6 | MMAE |
| 16 | S | L7 | MMAE |
| 16 | R | L7 | MMAE |
| 16 | S | L8 | MMAE |
| 16 | R | L8 | MMAE |
| 16 | S | L9 | MMAE |
| 16 | R | L9 | MMAE |
| 16 | S | L10 | MMAE |
| 16 | R | L10 | MMAE |
| 16 | S | L15 | MMAE |
| 16 | R | L15 | MMAE |
| 16 | S | L20 | MMAE |
| 16 | R | L20 | MMAE |
| 16 | S | Bond | Eribulin |
| 16 | R | Bond | Eribulin |
| 16 | S | L1 | Eribulin |
| 16 | R | L1 | Eribulin |
| 16 | S | L2 | Eribulin |
| 16 | R | L2 | Eribulin |
| 16 | S | L3 | Eribulin |
| 16 | R | L3 | Eribulin |
| 16 | S | L4 | Eribulin |
| 16 | R | L4 | Eribulin |
| 16 | S | L5 | Eribulin |
| 16 | R | L5 | Eribulin |
| 16 | S | L6 | Eribulin |
| 16 | R | L6 | Eribulin |
| 16 | S | L7 | Eribulin |
| 16 | R | L7 | Eribulin |
| 16 | S | L8 | Eribulin |
| 16 | R | L8 | Eribulin |
| 16 | S | L9 | Eribulin |
| 16 | R | L9 | Eribulin |
| 16 | S | L10 | Eribulin |
| 16 | R | L10 | Eribulin |
| 16 | S | L15 | Eribulin |
| 16 | R | L15 | Eribulin |
| 16 | S | L20 | Eribulin |
| 16 | R | L20 | Eribulin |
| 16 | S | Bond | SN-38 |
| 16 | R | Bond | SN-38 |
| 16 | S | L1 | SN-38 |
| 16 | R | L1 | SN-38 |
| 16 | S | L2 | SN-38 |
| 16 | R | L2 | SN-38 |
| 16 | S | L3 | SN-38 |
| 16 | R | L3 | SN-38 |
| 16 | S | L4 | SN-38 |
| 16 | R | L4 | SN-38 |
| 16 | S | L5 | SN-38 |
| 16 | R | L5 | SN-38 |
| 16 | S | L6 | SN-38 |
| 16 | R | L6 | SN-38 |
| 16 | S | L7 | SN-38 |
| 16 | R | L7 | SN-38 |
| 16 | S | L8 | SN-38 |
| 16 | R | L8 | SN-38 |
| 16 | S | L9 | SN-38 |
| 16 | R | L9 | SN-38 |
| 16 | S | L10 | SN-38 |
| 16 | R | L10 | SN-38 |
| 16 | S | L15 | SN-38 |
| 16 | R | L15 | SN-38 |
| 16 | S | L20 | SN-38 |
| 16 | R | L20 | SN-38 |
| 17 | S | Bond | MMAE |
| 17 | R | Bond | MMAE |
| 17 | S | L1 | MMAE |
| 17 | R | L1 | MMAE |
| 17 | S | L2 | MMAE |
| 17 | R | L2 | MMAE |
| 17 | S | L3 | MMAE |
| 17 | R | L3 | MMAE |
| 17 | S | L4 | MMAE |
| 17 | R | L4 | MMAE |
| 17 | S | L5 | MMAE |
| 17 | R | L5 | MMAE |
| 17 | S | L6 | MMAE |
| 17 | R | L6 | MMAE |
| 17 | S | L7 | MMAE |
| 17 | R | L7 | MMAE |
| 17 | S | L8 | MMAE |
| 17 | R | L8 | MMAE |
| 17 | S | L9 | MMAE |
| 17 | R | L9 | MMAE |
| 17 | S | L10 | MMAE |
| 17 | R | L10 | MMAE |
| 17 | S | L15 | MMAE |
| 17 | R | L15 | MMAE |
| 17 | S | L20 | MMAE |
| 17 | R | L20 | MMAE |
| 17 | S | Bond | Eribulin |
| 17 | R | Bond | Eribulin |
| 17 | S | L1 | Eribulin |
| 17 | R | L1 | Eribulin |
| 17 | S | L2 | Eribulin |
| 17 | R | L2 | Eribulin |
| 17 | S | L3 | Eribulin |
| 17 | R | L3 | Eribulin |
| 17 | S | L4 | Eribulin |
| 17 | R | L4 | Eribulin |
| 17 | S | L5 | Eribulin |
| 17 | R | L5 | Eribulin |
| 17 | S | L6 | Eribulin |
| 17 | R | L6 | Eribulin |
| 17 | S | L7 | Eribulin |
| 17 | R | L7 | Eribulin |
| 17 | S | L8 | Eribulin |
| 17 | R | L8 | Eribulin |
| 17 | S | L9 | Eribulin |
| 17 | R | L9 | Eribulin |
| 17 | S | L10 | Eribulin |
| 17 | R | L10 | Eribulin |
| 17 | S | L15 | Eribulin |
| 17 | R | L15 | Eribulin |
| 17 | S | L20 | Eribulin |
| 17 | R | L20 | Eribulin |
| 17 | S | Bond | SN-38 |
| 17 | R | Bond | SN-38 |
| 17 | S | L1 | SN-38 |
| 17 | R | L1 | SN-38 |
| 17 | S | L2 | SN-38 |
| 17 | R | L2 | SN-38 |
| 17 | S | L3 | SN-38 |
| 17 | R | L3 | SN-38 |
| 17 | S | L4 | SN-38 |
| 17 | R | L4 | SN-38 |
| 17 | S | L5 | SN-38 |
| 17 | R | L5 | SN-38 |
| 17 | S | L6 | SN-38 |
| 17 | R | L6 | SN-38 |
| 17 | S | L7 | SN-38 |
| 17 | R | L7 | SN-38 |
| 17 | S | L8 | SN-38 |
| 17 | R | L8 | SN-38 |
| 17 | S | L9 | SN-38 |
| 17 | R | L9 | SN-38 |
| 17 | S | L10 | SN-38 |
| 17 | R | L10 | SN-38 |
| 17 | S | L15 | SN-38 |
| 17 | R | L15 | SN-38 |
| 17 | S | L20 | SN-38 |
| 17 | R | L20 | SN-38 |
| 18 | S | Bond | MMAE |
| 18 | R | Bond | MMAE |
| 18 | S | L1 | MMAE |
| 18 | R | L1 | MMAE |
| 18 | S | L2 | MMAE |
| 18 | R | L2 | MMAE |
| 18 | S | L3 | MMAE |
| 18 | R | L3 | MMAE |
| 18 | S | L4 | MMAE |
| 18 | R | L4 | MMAE |
| 18 | S | L5 | MMAE |
| 18 | R | L5 | MMAE |
| 18 | S | L6 | MMAE |
| 18 | R | L6 | MMAE |
| 18 | S | L7 | MMAE |
| 18 | R | L7 | MMAE |
| 18 | S | L8 | MMAE |
| 18 | R | L8 | MMAE |
| 18 | S | L9 | MMAE |
| 18 | R | L9 | MMAE |
| 18 | S | L10 | MMAE |
| 18 | R | L10 | MMAE |
| 18 | S | L15 | MMAE |
| 18 | R | L15 | MMAE |
| 18 | S | L20 | MMAE |
| 18 | R | L20 | MMAE |
| 18 | S | Bond | Eribulin |
| 18 | R | Bond | Eribulin |
| 18 | S | L1 | Eribulin |
| 18 | R | L1 | Eribulin |
| 18 | S | L2 | Eribulin |
| 18 | R | L2 | Eribulin |
| 18 | S | L3 | Eribulin |
| 18 | R | L3 | Eribulin |
| 18 | S | L4 | Eribulin |
| 18 | R | L4 | Eribulin |
| 18 | S | L5 | Eribulin |
| 18 | R | L5 | Eribulin |
| 18 | S | L6 | Eribulin |
| 18 | R | L6 | Eribulin |
| 18 | S | L7 | Eribulin |
| 18 | R | L7 | Eribulin |
| 18 | S | L8 | Eribulin |
| 18 | R | L8 | Eribulin |
| 18 | S | L9 | Eribulin |
| 18 | R | L9 | Eribulin |
| 18 | S | L10 | Eribulin |
| 18 | R | L10 | Eribulin |
| 18 | S | L15 | Eribulin |
| 18 | R | L15 | Eribulin |
| 18 | S | L20 | Eribulin |
| 18 | R | L20 | Eribulin |
| 18 | S | Bond | SN-38 |
| 18 | R | Bond | SN-38 |
| 18 | S | L1 | SN-38 |
| 18 | R | L1 | SN-38 |
| 18 | S | L2 | SN-38 |
| 18 | R | L2 | SN-38 |
| 18 | S | L3 | SN-38 |
| 18 | R | L3 | SN-38 |
| 18 | S | L4 | SN-38 |
| 18 | R | L4 | SN-38 |
| 18 | S | L5 | SN-38 |
| 18 | R | L5 | SN-38 |
| 18 | S | L6 | SN-38 |
| 18 | R | L6 | SN-38 |
| 18 | S | L7 | SN-38 |
| 18 | R | L7 | SN-38 |
| 18 | S | L8 | SN-38 |
| 18 | R | L8 | SN-38 |
| 18 | S | L9 | SN-38 |
| 18 | R | L9 | SN-38 |
| 18 | S | L10 | SN-38 |
| 18 | R | L10 | SN-38 |
| 18 | S | L15 | SN-38 |
| 18 | R | L15 | SN-38 |
| 18 | S | L20 | SN-38 |
| 18 | R | L20 | SN-38 |
| 19 | S | Bond | MMAE |
| 19 | R | Bond | MMAE |
| 19 | S | L1 | MMAE |
| 19 | R | L1 | MMAE |
| 19 | S | L2 | MMAE |
| 19 | R | L2 | MMAE |
| 19 | S | L3 | MMAE |
| 19 | R | L3 | MMAE |
| 19 | S | L4 | MMAE |
| 19 | R | L4 | MMAE |
| 19 | S | L5 | MMAE |
| 19 | R | L5 | MMAE |
| 19 | S | L6 | MMAE |
| 19 | R | L6 | MMAE |
| 19 | S | L7 | MMAE |
| 19 | R | L7 | MMAE |
| 19 | S | L8 | MMAE |
| 19 | R | L8 | MMAE |
| 19 | S | L9 | MMAE |
| 19 | R | L9 | MMAE |
| 19 | S | L10 | MMAE |
| 19 | R | L10 | MMAE |
| 19 | S | L15 | MMAE |
| 19 | R | L15 | MMAE |
| 19 | S | L20 | MMAE |
| 19 | R | L20 | MMAE |
| 19 | S | Bond | Eribulin |
| 19 | R | Bond | Eribulin |
| 19 | S | L1 | Eribulin |
| 19 | R | L1 | Eribulin |
| 19 | S | L2 | Eribulin |
| 19 | R | L2 | Eribulin |
| 19 | S | L3 | Eribulin |
| 19 | R | L3 | Eribulin |
| 19 | S | L4 | Eribulin |
| 19 | R | L4 | Eribulin |
| 19 | S | L5 | Eribulin |
| 19 | R | L5 | Eribulin |
| 19 | S | L6 | Eribulin |
| 19 | R | L6 | Eribulin |
| 19 | S | L7 | Eribulin |
| 19 | R | L7 | Eribulin |
| 19 | S | L8 | Eribulin |
| 19 | R | L8 | Eribulin |
| 19 | S | L9 | Eribulin |
| 19 | R | L9 | Eribulin |
| 19 | S | L10 | Eribulin |
| 19 | R | L10 | Eribulin |
| 19 | S | L15 | Eribulin |
| 19 | R | L15 | Eribulin |
| 19 | S | L20 | Eribulin |
| 19 | R | L20 | Eribulin |
| 19 | S | Bond | SN-38 |
| 19 | R | Bond | SN-38 |
| 19 | S | L1 | SN-38 |
| 19 | R | L1 | SN-38 |
| 19 | S | L2 | SN-38 |
| 19 | R | L2 | SN-38 |
| 19 | S | L3 | SN-38 |
| 19 | R | L3 | SN-38 |
| 19 | S | L4 | SN-38 |
| 19 | R | L4 | SN-38 |
| 19 | S | L5 | SN-38 |
| 19 | R | L5 | SN-38 |
| 19 | S | L6 | SN-38 |
| 19 | R | L6 | SN-38 |
| 19 | S | L7 | SN-38 |
| 19 | R | L7 | SN-38 |
| 19 | S | L8 | SN-38 |
| 19 | R | L8 | SN-38 |
| 19 | S | L9 | SN-38 |
| 19 | R | L9 | SN-38 |
| 19 | S | L10 | SN-38 |
| 19 | R | L10 | SN-38 |
| 19 | S | L15 | SN-38 |
| 19 | R | L15 | SN-38 |
| 19 | S | L20 | SN-38 |
| 19 | R | L20 | SN-38 |
| 20 | S | Bond | MMAE |
| 20 | R | Bond | MMAE |
| 20 | S | L1 | MMAE |
| 20 | R | L1 | MMAE |
| 20 | S | L2 | MMAE |
| 20 | R | L2 | MMAE |
| 20 | S | L3 | MMAE |
| 20 | R | L3 | MMAE |
| 20 | S | L4 | MMAE |
| 20 | R | L4 | MMAE |
| 20 | S | L5 | MMAE |
| 20 | R | L5 | MMAE |
| 20 | S | L6 | MMAE |
| 20 | R | L6 | MMAE |
| 20 | S | L7 | MMAE |
| 20 | R | L7 | MMAE |
| 20 | S | L8 | MMAE |
| 20 | R | L8 | MMAE |
| 20 | S | L9 | MMAE |
| 20 | R | L9 | MMAE |
| 20 | S | L10 | MMAE |
| 20 | R | L10 | MMAE |
| 20 | S | L15 | MMAE |
| 20 | R | L15 | MMAE |
| 20 | S | L20 | MMAE |
| 20 | R | L20 | MMAE |
| 20 | S | Bond | Eribulin |
| 20 | R | Bond | Eribulin |
| 20 | S | L1 | Eribulin |
| 20 | R | L1 | Eribulin |
| 20 | S | L2 | Eribulin |
| 20 | R | L2 | Eribulin |
| 20 | S | L3 | Eribulin |
| 20 | R | L3 | Eribulin |
| 20 | S | L4 | Eribulin |
| 20 | R | L4 | Eribulin |
| 20 | S | L5 | Eribulin |
| 20 | R | L5 | Eribulin |
| 20 | S | L6 | Eribulin |
| 20 | R | L6 | Eribulin |
| 20 | S | L7 | Eribulin |
| 20 | R | L7 | Eribulin |
| 20 | S | L8 | Eribulin |
| 20 | R | L8 | Eribulin |
| 20 | S | L9 | Eribulin |
| 20 | R | L9 | Eribulin |
| 20 | S | L10 | Eribulin |
| 20 | R | L10 | Eribulin |
| 20 | S | L15 | Eribulin |
| 20 | R | L15 | Eribulin |
| 20 | S | L20 | Eribulin |
| 20 | R | L20 | Eribulin |
| 20 | S | Bond | SN-38 |
| 20 | R | Bond | SN-38 |
| 20 | S | L1 | SN-38 |
| 20 | R | L1 | SN-38 |
| 20 | S | L2 | SN-38 |
| 20 | R | L2 | SN-38 |
| 20 | S | L3 | SN-38 |
| 20 | R | L3 | SN-38 |
| 20 | S | L4 | SN-38 |
| 20 | R | L4 | SN-38 |
| 20 | S | L5 | SN-38 |
| 20 | R | L5 | SN-38 |
| 20 | S | L6 | SN-38 |
| 20 | R | L6 | SN-38 |
| 20 | S | L7 | SN-38 |
| 20 | R | L7 | SN-38 |
| 20 | S | L8 | SN-38 |
| 20 | R | L8 | SN-38 |
| 20 | S | L9 | SN-38 |
| 20 | R | L9 | SN-38 |
| 20 | S | L10 | SN-38 |
| 20 | R | L10 | SN-38 |
| 20 | S | L15 | SN-38 |
| 20 | R | L15 | SN-38 |
| 20 | S | L20 | SN-38 |
| 20 | R | L20 | SN-38. |

The following compounds are still alternative:
wherein Rx is:

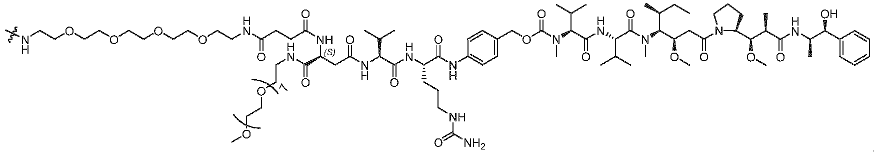

According to the embodiments of the present disclosure, the conjugate is selected from the following specific compounds, or tautomers, stereoisomers, pharmaceutically acceptable salts or isotopic variants thereof, wherein the compounds are represented by the structure shown in formula (VII): 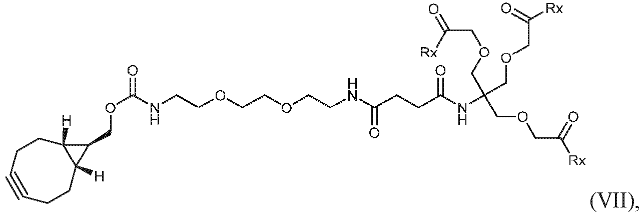 wherein Rx is 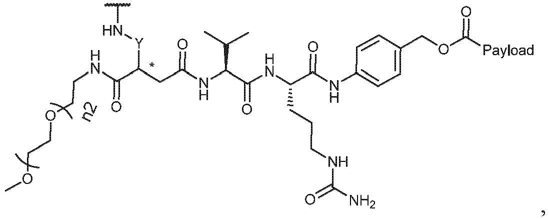 and n2, *, Y and payload are as defined in Table 1 above.

The following compounds are still alternative:
wherein Rx is:

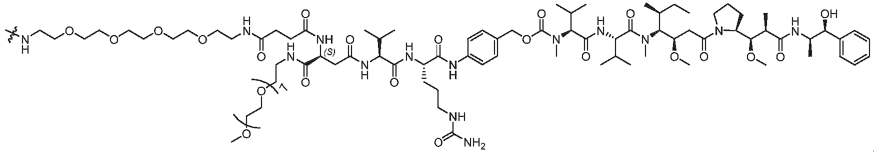

According to the embodiments of the present disclosure, the conjugate is selected from the following specific compounds, or tautomers, stereoisomers, pharmaceutically acceptable salts or isotopic variants thereof, wherein the compounds are represented by the structure shown in formula (VIII): 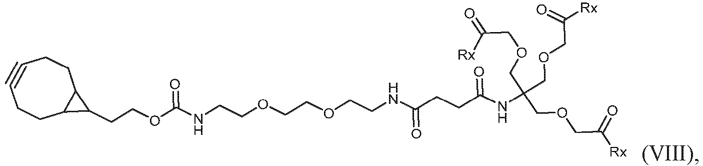 wherein Rx is 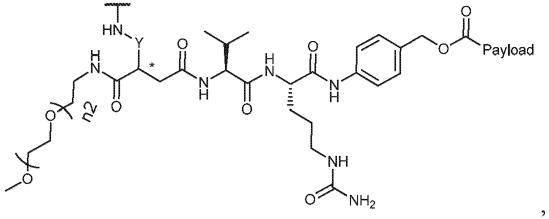 and n2, *, Y and payload are as defined in Table 1 above.

The following compounds are still alternative:
wherein Rx is:

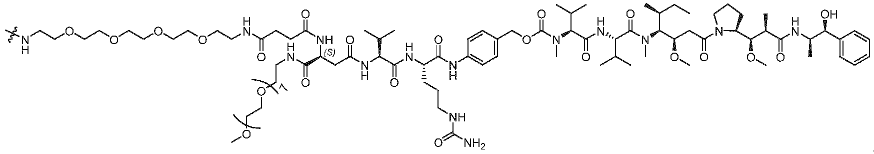

According to the embodiments of the present disclosure, the conjugate is selected from the following specific compounds, or tautomers, stereoisomers, pharmaceutically acceptable salts or isotopic variants thereof, wherein the compounds are represented by the structure shown in formula (IX): 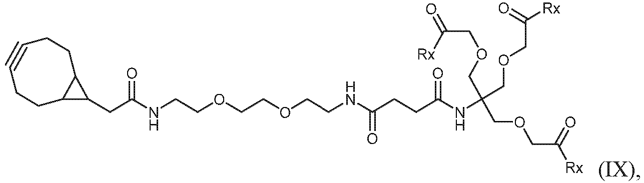 wherein, Rx is 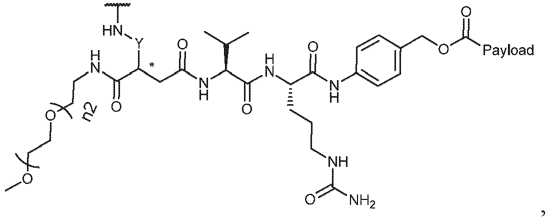 and n2, *, Y and payload are as defined in Table 1 above.

The following compounds are still alternative:
wherein Rx is:

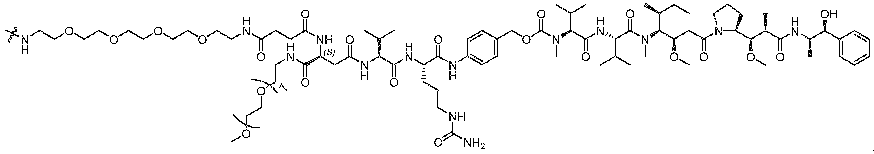

According to the embodiments of the present disclosure, the targeting moiety is a protein-based recognition molecule (PBRM).

According to the embodiments of the present disclosure, the recognition molecule is an internalizing antibody or an internalizing antigen-binding fragment thereof targeting tumor cells. In turn, the conjugate of the second aspect is covalently bonded to an internalizing antibody or an internalizing antigen-binding fragment thereof targeting tumor cells via an A group to form an antibody-drug conjugate (ADC).

According to the embodiments of the present disclosure, the antibody or antigen-binding fragment binds to anti-human epidermal growth factor receptor (HER2) antibody, EGFR, GPNMB, CD56, TACSTD2 (TROP2), CEACAM5, folate receptor-a, mesothelin, ENPP3, guanylate cyclase C, SLC44A4, NaPi2b, CD70, mucin 1, STEAP1, connexin 4, 5T4, SLTRK6, SC-16, LIV-1, P-cadherin, PSMA, extra domain B of fibronectin, endothelin receptor ETB, tenascin c, collagen IV, VEGFR2, periostin, CD30, CD79b, CD19, CD22, CD138, CD37, CD33, CD74, etc.

In the fourth aspect of the present disclosure, provided herein is a pharmaceutical composition. According to the embodiments of the present disclosure, the pharmaceutical composition comprises the above conjugate and a pharmaceutically acceptable carrier, wherein the conjugate comprises a targeting moiety, the compound of the first aspect, and a drug moiety, wherein the targeting moiety is covalently linked to one or more of the compounds, and the compound is covalently linked to the drug moiety via an R group.

In the fifth aspect of the present disclosure, provided herein is use of the above conjugate or the above pharmaceutical composition in the manufacture of a medicament for treating a patient having or at risk of having a cancer expressing a target antigen, wherein the conjugate comprises a targeting moiety, the compound of the first aspect, and a drug moiety, wherein the targeting moiety is covalently linked to one or more of the compounds, and the compound is covalently linked to the drug moiety via an R group.

According to the embodiments of the present disclosure, the target antigen is human epidermal growth factor receptor 2.

According to the embodiments of the present disclosure, the cancer expresses a high level of human epidermal growth factor receptor 2.

According to the embodiments of the present disclosure, the cancer is breast cancer, gastric cancer, bladder cancer, or urothelial carcinoma.

In the sixth aspect of the present disclosure, provided herein is a method of treating a patient having or at risk of having a cancer expressing a target antigen. According to the embodiments of the present disclosure, the method comprises administering to the patient a therapeutically effective amount of the above conjugate, which is an ADC comprising a targeting moiety, the compound of the first aspect and a drug moiety.

In the seventh aspect of the present disclosure, provided herein is a method of reducing or inhibiting the growth of a tumor expressing a target antigen, comprising administering a therapeutically effective amount of the above conjugate, which is an ADC comprising a targeting moiety, the compound of the first aspect and a drug moiety.

In the eighth aspect of the present disclosure, provided herein is use of the above conjugate in treating a cancer expressing a target antigen, wherein the conjugate is an ADC comprising a targeting moiety, the compound of the first aspect and a drug moiety.

In the ninth aspect of the present disclosure, provided herein is use of the above conjugate in the manufacture of a medicament for treating a cancer expressing a target antigen, wherein the conjugate is an ADC comprising a targeting moiety, the compound of the first aspect and a drug moiety.

In the tenth aspect of the present disclosure, provided herein is a method of producing the above conjugate, which comprises reacting an antibody or an antigen-binding fragment with the above linker compound bonded to a small molecule drug under conditions that allow the conjugation.

In the eleventh aspect of the present disclosure, provided herein is a method of determining whether a patient will respond to the treatment with the above conjugate, comprising providing a biological sample from the patient and contacting the biological sample with the above conjugate, wherein the conjugate is an ADC comprising a targeting moiety, the compound of the first aspect and a drug moiety.

According to the embodiments of the present disclosure, the biological sample is a tumor biopsy derived from a patient having or at risk of having a cancer expressing human epidermal growth factor receptor 2, wherein the cancer is breast cancer, gastric cancer, bladder cancer, or urothelial carcinoma.

### DETAILED DESCRIPTION

The following embodiments are provided to further illustrate the present disclosure. It should be understood that these embodiments are only for illustrating the present disclosure, but are not intended to limit the scope of the present disclosure.

### Terminology used herein

Unless defined otherwise, all technical and scientific terms used herein have the same meanings as commonly understood by those skilled in the art.

In the description of the present specification, descriptions with reference to terms "embodiment", "embodiments", "example", "specific embodiment", or "examples" etc. mean that a specific feature, structure, material, or characteristic described in conjunction with the embodiment or example is included in at least one embodiment or example of the present disclosure. In this specification, exemplary descriptions of the above terms do not necessarily refer to the same embodiment or example. Moreover, the described specific feature, structure, material or characteristic can be combined in any one or more embodiments or examples in a suitable manner. In addition, those skilled in the art can combine and incorporate different embodiments or examples and features of different embodiments or examples described in this specification without contradicting each other.

As used herein, the articles "a", "an" and "the" are intended to include "at least one" or "one or more" unless otherwise stated or clearly contradicted by context. Therefore, these articles used herein refer to articles of one or more than one (i.e., at least one) object. For example, "a component" refers to one or more components, that is, more than one component may be considered to be adopted or used in an implementation of the described embodiment.

As used herein, the term "subject" refers to an animal. Generally, the animal is a mammal. The subject, for example, also refers to primates (e.g. humans, males or females), bovines, sheep, goats, horses, canines, cats, rabbits, rats, mice, fish, birds, and the like. In some embodiments, the subject is a primate. In other embodiments, the subject is a human being.

As used herein, the term "patient" refers to human beings (including adults and children) or other animals. In some embodiments, "patient" refers to a human being.

The term "include" or "comprise" is an open-ended expression, i.e., including the content specified in the present disclosure but not excluding the content in other aspects.

"Stereoisomer" refers to a compound that has the same chemical structure but differs in the spatial arrangement of atoms or moieties. Stereoisomers include enantiomers, diastereomers, conformational isomers (rotamers), geometric isomers (*cis*/*trans* isomers), atropisomers, etc.

"Chirality" means that a molecule has the property that the molecule and its mirror image are non-superimposable; and "achirality" means that a molecule has the property that the molecule and its mirror image are superimposable.

"Enantiomer" refers to two isomers of a compound that are each a mirror image of the other one but are non-superimposable with each other.

"Diastereomer" refers to stereoisomers that have two or more chiral centers and whose molecules are not mirror images of each other. Diastereomers have different physical properties such as melting point, boiling point, spectral property, and reactivity. A mixture of diastereomers can be separated by high-resolution analytical operations, for example, electrophoresis and chromatography such as HPLC.

The definitions and rules of stereochemistry used in the present disclosure generally follow S. P. Parker, Ed., "McGraw-Hill Dictionary of Chemical Terms (1984)", McGraw-Hill Book Company, New York; and Eliel, E. and Wilen, S., "Stereochemistry of Organic Compounds", John Wiley & Sons, Inc., New York, 1994.

Many organic compounds exist in optically active forms, i.e., they have the ability to rotate the plane of plane polarized light. When describing optically active compounds, the prefixes D and L, or *R* and *S* are used to denote the absolute configurations of the molecule with respect to one or more chiral centers. The prefixes *d* and *l*, or (+) and (-) are symbols used to designate the rotation of plane polarized light due to a compound, wherein (-) or *1* indicates that the compound is levorotatory, and (+) or *d* indicates that the compound is dextrorotatory. One specific stereoisomer is an enantiomer, and a mixture of such isomers is called an enantiomeric mixture. A mixture of enantiomers in 50:50 is called a racemic mixture or a racemate, which may occur when a chemical reaction or process is not stereoselective or stereospecific.

Any asymmetric atom (e.g., carbon, etc.) of the compound of the present disclosure can be present in a racemic or enantiomer-enriched form, for example, present in (*R*)-, (*S*)-, or (*R*, *S*)-configuration. In some embodiments, in terms of (*R*)- or (*S*)-configuration, each asymmetric atom has an enantiomeric excess of at least 50%, an enantiomeric excess of at least 60%, an enantiomeric excess of at least 70%, an enantiomeric excess of at least 80%, an enantiomeric excess of at least 90%, an enantiomeric excess of at least 95%, or an enantiomeric excess of at least 99%.

In accordance with the selection of starting materials and methods, the compounds of the present disclosure may be present as one of the possible isomers or a mixture thereof, such as a racemate and a mixture of diastereomers, depending on the number of asymmetric carbon atoms. Optically active (*R*)- or (*S*)-isomers can be prepared using chiral synthons or chiral reagents, or resolved using conventional techniques. If a compound contains a double bond, the substituents may be in an E or Z configuration; and if a compound contains disubstituted cycloalkyl, the substituents of the cycloalkyl may have a *cis-* or *trans*-configuration.

Any obtained mixture of stereoisomers can be separated into pure or substantially pure geometric isomers, enantiomers, or diastereomers according to the differences in physical and chemical properties of components, for example, by chromatography and/or fractional crystallization.

A racemate of any obtained end-product or intermediate can be resolved into optical enantiomers by methods known to those skilled in the art, for example, by separating salts of the obtained diastereomers. Racemic products can also be separated by chiral chromatography, such as high-performance liquid chromatography (HPLC) using chiral adsorbents. Particularly, enantiomers can be prepared by asymmetric synthesis, for example, referring to Jacques, et al., Enantiomers, Racemates and Resolutions (Wiley Interscience, New York, 1981); Principles of Asymmetric Synthesis (2nd Ed. Robert E. Gawley, Jeffrey Aubé, Elsevier, Oxford, UK, 2012); Eliel, E.L. Stereochemistry of Carbon Compounds (McGraw-Hill, NY, 1962); Wilen, S.H. Tables of Resolving Agents and Optical Resolutions p. 268 (E.L. Eliel, Ed., Univ. of Notre Dame Press, Notre Dame, IN 1972); Chiral Separation Techniques: A Practical Approach (Subramanian, G. Ed., Wiley-VCH Verlag GmbH & Co. KGaA, Weinheim, Germany, 2007).

The term "tautomer" or "tautomeric form" refers to structural isomers that have different energies and can be interconverted by crossing a low energy barrier. If tautomerism is possible (e.g., in solution), a chemical equilibrium of tautomers can be reached. For example, protontautomer (also known as prototropic tautomer) includes interconversion through proton migration, such as keto-enol isomerization and imine-enamine isomerization. Valence tautomer includes interconversion through recombination of some bonding electrons. A specific example of keto-enol tautomerism is interconversion of pentane-2,4-dione and 4-hydroxy-3-penten-2-one tautomers. Another example of tautomerism is phenol-keto tautomerism. A specific example of phenol-keto tautomerism is interconversion of 4-hydroxypyridine and pyridin-4(1*H*)-one tautomers. Unless otherwise indicated, all tautomeric forms of the compound of the present disclosure shall fall within the scope of the present disclosure.

The present disclosure also comprises compounds that are labeled with isotopes (isotopic variants), which are equivalent to those described in formula (I), but one or more atoms are replaced by atoms having an atom mass or mass number that are different from that of atoms that are common in nature. Examples of isotopes which may be introduced into the compounds of the present disclosure include isotopes of hydrogen, carbon, nitrogen, oxygen, phosphorus, sulfur, fluorine and chlorine, such as ²H, ³H , ¹³C, ¹¹C, ¹⁴C, ¹⁵N, ¹⁸O, ¹⁷O, ³¹P, ³²P, ³⁵S, ¹⁸F and ³⁶Cl, respectively. Compounds of the present disclosure that comprise the above isotopes and/or other isotopes of other atoms and pharmaceutically acceptable salts thereof are all within the scope of the present disclosure. Certain isotope-labeled compounds of the present disclosure, such as those incorporating radioactive isotopes (e.g., ³H and ¹⁴C), can be used for the measurement of the distribution of drug and/or substrate in tissue. Tritium, which is ³H and carbon-14, which is ¹⁴C isotope, are yet alternative, because they are easy to prepare and detect. Furthermore, replaced by heavier isotopes, such as deuterium, which is ²H, may provide therapeutic benefits due to the higher metabolic stability, such as prolonging the half-life in vivo or decreasing the dosage requirements, and thus may be alternative in some cases. Isotope-labeled compounds of the present disclosure can be prepared generally by using readily available isotope-labeled reagents to replace non-isotope-labeled reagents in the following schemes and/or the procedures disclosed in the examples and preparation examples.

As described in the present disclosure, the compound of the present disclosure may be optionally substituted with one or more substituents, for example, compounds represented by the above general formulas, or specific example compounds in the examples, and a class of compounds contained in the present disclosure.

The term "protecting group" or "PG" refers a substituent which, when reacted with other functional groups, is usually used to block or protect specific functionality. For example, "amino-protecting group" refers to a substituent that is connected to an amino group to block or protect the functionality of the amino group in a compound. Suitable amino-protecting groups include acetyl, trifluoroacetyl, tert-butoxycarbonyl (BOC, Boc), benzyloxycarbonyl (CBZ, Cbz), and 9-fluorenylmethyleneoxycarbonyl (Fmoc). Similarly, "hydroxyl-protecting group" refers to a substituent of a hydroxyl group, which is used to block or protect the functionality of the hydroxyl group. Suitable hydroxyl-protecting groups include acetyl and silyl groups. "Carboxy-protecting group" refers to a substituent of a carboxyl group, which is used to block or protect the functionality of the carboxyl group. The carboxyl-protecting groups generally include - CH₂CH₂SO₂Ph, cyanoethyl, 2-(trimethylsilyl)ethyl, 2-(trimethylsilyl)ethoxymethyl, 2-(p-toluenesulfonyl)ethyl, 2-(p-nitrobenzenesulfonyl)ethyl, 2-(diphenylphosphino)ethyl, nitroethyl, etc. For a general description of protecting groups, please refer to: T W. Greene, Protective Groups in Organic Synthesis, John Wiley&Sons, New York, 1991; and P. J. Kocienski, Protecting Groups, Thieme, Stuttgart, 2005.

As used herein, "pharmaceutically acceptable salt" refers to an organic or inorganic salt of a compound of the present disclosure. Pharmaceutically acceptable salts are well known in the art, as described in the literature: S. M. Berge et al. describe pharmaceutically acceptable salts in detail in J. Pharmaceutical Sciences, 1977, 66: 1-19. Salts formed by pharmaceutically acceptable non-toxic acids include, but are not limited to: inorganic acid salts formed through the reaction with amino groups, such as hydrochloride, hydrobromide, phosphate, sulfate, and perchlorate; organic acid salts such as acetate, oxalate, maleate, tartrate, citrate, succinate, and malonate; or salts obtained by other methods such as ion exchange described in books and literatures. Other pharmaceutically acceptable salts include adipate, alginate, ascorbate, aspartate, benzenesulfonate, benzoate, bisulfate, borate, butyrate, camphorate, camphorsulfonate, cyclopentyl propionate, digluconate, lauryl sulfate, ethanesulfonate, formate, fumarate, gluceptate, glycerophosphate, gluconate, hemisulfate, heptanoate, caproate, hydroiodide, 2-hydroxy-ethanesulfonate, lactobionate, lactate, laurate, lauryl sulfate, malate, malonate, methanesulfonate, 2-naphthalenesulfonate, nicotinate, nitrate, oleate, palmitate, pamoate, pectinate, persulfate, 3-phenylpropionate, picrate, pivalate, propionate, stearate, thiocyanate, p-toluenesulfonate, undecanoate, valerate, etc. Salts obtained from appropriate bases include salts of alkali metals, alkaline earth metals, ammonium, and N⁺(C₁-C₄ alkyl)₄. The present disclosure also intends to contemplate any quaternary ammonium salts formed by compounds containing a group containing N. Water-soluble or oil-soluble or dispersed products can be obtained by quaternization. Salts of alkali metals or alkaline earth metals include salts of sodium, lithium, potassium, calcium, magnesium, etc. Pharmaceutically acceptable salts further include suitable non-toxic ammonium, and amine cations formed by quaternary ammonium salts and counterions such as halides, hydroxides, carboxylates, sulfates, phosphates, nitrates, C₁₋₈ sulfonates and aromatic sulfonates.

The term "conjugate" refers to a substance formed by covalently bonding two or more compounds, such as a conjugate formed by covalently bonding a compound of formula (I) of this application to a small molecule drug, or a conjugate formed by covalently bonding the compound of formula (I) to an antibody or an antigen-binding fragment, or a conjugate formed by covalently bonding the compound of formula (I) to an antibody or an antigen-binding fragment and a small molecule drug, in which case the conjugate is an antibody-drug conjugate (ADC).

The terms "antibody-drug conjugate", "antibody conjugate", "conjugate", "immunoconjugate", and "ADC" are used interchangeably, and refer to a compound that is linked to an antibody (e.g., an anti-HER2 antibody), or a derivative thereof.

The term "antibody" is used in the broadest sense to refer to an immunoglobulin molecule that recognizes and specifically binds to a target, such as a protein, polypeptide, carbohydrate, polynucleotide, lipid, or a combination thereof through antigen recognition sites within the variable region of the immunoglobulin molecule. The heavy chain of an antibody is composed of a heavy chain variable domain (VH) and a heavy chain constant domain (CH). The light chain is composed of a light chain variable domain (VL) and a light chain constant domain (CL). For the purposes of this application, mature heavy chain and light chain variable domains each comprise three complementarity determining regions (CDR1, CDR2 and CDR3) within four framework regions (FR1, FR2, FR3 and FR4) arranged from N-terminus to C-terminus: FR1, CDR1, FR2, CDR2, FR3, CDR3 and FR4. An "antibody" can be naturally occurring or man-made, such as monoclonal antibodies produced by conventional hybridoma technology. The term "antibody" includes full-length monoclonal antibodies and full-length polyclonal antibodies, as well as antibody fragments such as Fab, Fab', F(ab')2, Fv, and single chain antibodies. An antibody can be any one of the five major classes of immunoglobulins: IgA, IgD, IgE, IgG, and IgM, or a subclass thereof (e.g., isotypes IgG1, IgG2, IgG3, and IgG4). The term further encompasses human antibodies, chimeric antibodies, humanized antibodies and any modified immunoglobulin molecule containing an antigen recognition site, so long as it demonstrates the desired biological activity.

As used herein, the term "antigen-binding fragment" or "antigen-binding portion" of an antibody refers to one or more fragments of an antibody that retain the ability to specifically bind to an antigen (e.g., HER2). Antigen-binding fragments alternatively also retain the ability to internalize into an antigen-expressing cell. In some embodiments, antigen-binding fragments also retain immune effector activity. It has been shown that fragments of a full-length antibody can perform the antigen-binding function of a full-length antibody. Examples of binding fragments encompassed within the term "antigen-binding fragment" or "antigen-binding portion" of an antibody include (i) a Fab fragment, a monovalent fragment consisting of the VL, VH, CL, and CH1 domains; (ii) a F(ab')2 fragment, a bivalent fragment comprising two Fab fragments linked by a disulfide bond at the hinge region; (iii) a Fd fragment consisting of the VH and CH1 domains; (iv) a Fv fragment consisting of the VL and VH domains of a single arm of an antibody; (v) a dAb fragment, which comprises a single variable domain, e.g., a VH domain (see, e.g., Ward et al. (1989) Nature 341:544-6; and Winter et al., WO 90/05144); and (vi) an isolated complementarity determining region (CDR). Furthermore, although the two domains of the Fv fragment, VL and VH, are coded by separate genes, they can be joined, using recombinant methods, by a synthetic linker that enables them to be made as a single protein chain in which the VL and VH domains pair to form a monovalent molecule (known as single chain Fv(scFv)). See, e.g., Bird et al. (1988) Science 242:423-6; and Huston et al. (1988) Proc. Natl. Acad. Sci. USA 85:5879-83. Such single chain antibodies are also intended to be encompassed within the term "antigen-binding fragment" or "antigen-binding portion" of an antibody, and are known in the art as an exemplary type of binding fragment that can internalize into cells upon binding. See, e.g., Zhu et al. (2010) 9:2131-41; He et al. (2010) J. Nucl. Med. 51:427-32; and Fitting et al. (2015) MAbs 7:390-402. In certain embodiments, scFv molecules may be incorporated into a fusion protein. Other forms of single chain antibodies, such as bifunctional antibodies are also encompassed. Bifunctional antibodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see e.g., Holliger et al. (1993) Proc. Natl. Acad. Sci. USA 90:6444-8; and Poljak et al. (1994) Structure 2:1121-3). Antigen-binding fragments are obtained using conventional techniques known to those skilled in the art, and the binding fragments are screened for utility (e.g., binding affinity, internalization) in the same manner as intact antibodies. Antigen-binding fragments may be prepared by cleavage of the intact protein, e.g., by protease or chemical cleavage.

As used herein in reference to an antibody or antigen-binding fragment, "internalizing" means that an antibody or antigen-binding fragment is capable of being taken through the cell's lipid bilayer membrane into an internal compartment (i.e., "internalized") upon binding to the cell, alternatively into a degradative compartment in the cell. For example, an internalizing anti-HER2 antibody is one that is capable of being taken into the cell after binding to HER2 on the cell membrane.

The term "human epidermal growth factor receptor 2", "her2", or "her2/neu" refers to any native form of human her2. The term encompasses full-length her2 (e.g., NCBI Reference Sequence: NP_004439.2; SEQ ID NO: 21), as well as any form of human her2 that results from cellular processing. The term also encompasses naturally occurring variants of her2, including but not limited to splice variants, allelic variants, and isoforms. Her2 can be isolated from human, or may be produced recombinantly or by synthetic methods.

The term "anti-her2 antibody" or "antibody that specifically binds her2" refers to any form of antibody or fragment thereof that specifically binds her2, and encompasses monoclonal antibodies (including full length monoclonal antibodies), polyclonal antibodies, and biologically functional antibody fragments so long as they specifically bind her2. U.S. Pat. No. 5,821,337 (incorporated herein by reference) provides exemplary her2-binding sequences, including exemplary anti-her2 antibody sequences. Alternatively, the anti-her2 antibody used in the ADCs disclosed herein is an internalizing antibody or internalizing antibody fragment. Trastuzumab is an exemplary internalizing anti-human her2 antibody.

The term "kon" or "ka" refers to the on-rate constant for association of an antibody to an antigen to form an antibody/antigen complex. The rate can be determined using standard assays, such as Biacore assay or ELISA.

The term "koff" or "kd" refers to the off-rate constant for dissociation of an antibody from an antibody/antigen complex. The rate can be determined using standard assays, such as Biacore assay or ELISA.

The term "K_{D}" refers to the equilibrium dissociation constant of a particular antibody-antigen interaction. K_{D} is calculated by ka/kd. The rate can be determined using standard assays, such as Biacore assay or ELISA. The antibody or antigen-binding fragment in the conjugate of the present application can bind to the target antigen at an equilibrium dissociation constant (K_{D} ≤ 1 mM, ≤ 100 nM or ≤ 10 nM, or any amount therebetween). In certain embodiments, the K_{D} is between 1 pM and 500 pM. In some embodiments, the K_{D} is between 500 pM and 1 µM.

The term "antibody:drug ratio" or "drug-to-antibody ratio" or "DAR" refers to the number of attached drug moieties per antibody moiety, i.e., drug loading.

The term "therapeutic agent", "drug" or "drug moiety" refers to an agent capable of modulating a biological process and/or having a biological activity.

The term "cytotoxic agent" refers to a substance that causes cell death primarily by interfering with a cell's expression activity and/or functioning. Examples of cytotoxic agents include, but are not limited to, anti-mitotic agents, such as eribulin, auristatins (e.g., monomethyl auristatin E (MMAE)).

The term "cancer" refers to the physiological condition in mammals in which a population of cells is characterized by unregulated cell growth.

A "pharmaceutical composition" refers to a formulation which is in such form as to permit administration and subsequently provide the intended biological activity of the active ingredient (s) and/or to achieve a therapeutic effect, and which contains no additional components which are unacceptably toxic to a subject to which the formulation would be administered. The pharmaceutical composition may be sterile.

A "pharmaceutical excipient" comprises a substance such as an adjuvant, a carrier, a pH-adjusting and buffering agent, a tonicity adjusting agent, a wetting agent, a preservative, and the like.

"Pharmaceutically acceptable" means, within the scope of sound medical judgment, suitable for contact with human and lower animal tissues without undue toxicity, irritation, allergic reaction, etc., and commensurate with a reasonable benefit/risk ratio.

An "effective amount" of an ADC as disclosed herein is an amount sufficient to perform a specifically stated purpose, for example to produce a therapeutic effect after administration, such as a reduction in tumor growth rate or tumor volume, a reduction in a symptom of cancer, or some other indicia of treatment efficacy. An effective amount can be determined in a routine manner in relation to the stated purpose. The term "therapeutically effective amount" refers to an amount of an ADC effective to treat a disease or condition in a subject. In the case of cancer, a therapeutically effective amount of ADC can reduce the number of cancer cells, reduce tumor size, inhibit (e.g., slow or stop) tumor metastasis, inhibit (e.g., slow or stop) tumor growth, and/or relieve one or more symptoms. A "prophylactically effective amount" refers to a required amount effective, at dosages and for periods of time necessary, to achieve the prophylactic result. Typically, since a prophylactic dose is used in a subject prior to or at an earlier stage of a disease, the prophylactically effective amount will be less than the therapeutically effective amount.

An "effective amount" of an ADC as disclosed herein is an amount sufficient to perform a specifically stated purpose, for example to produce a therapeutic effect after administration, such as a reduction in tumor growth rate or tumor volume, a reduction in a symptom of cancer, or some other indicia of treatment efficacy. An effective amount can be determined in a routine manner in relation to the stated purpose. The term "therapeutically effective amount" refers to an amount of an ADC effective to treat a disease or condition in a subject. In the case of cancer, a therapeutically effective amount of ADC can reduce the number of cancer cells, reduce tumor size, inhibit (e.g., slow or stop) tumor metastasis, inhibit (e.g., slow or stop) tumor growth, and/or relieve one or more symptoms. A "prophylactically effective amount" refers to a required amount effective, at dosages and for periods of time necessary, to achieve the prophylactic result. Typically, since a prophylactic dose is used in a subject prior to or at an earlier stage of a disease, the prophylactically effective amount will be less than the therapeutically effective amount.

Now some embodiments of the present disclosure are described in more detail, examples of which are illustrated by the accompanying structural formulas and chemical formulas. The present disclosure is intended to cover all alternatives, modifications and equivalent technical solutions falling within the scope of the present disclosure as defined by the claims. Those skilled in the art should recognize that many methods and materials similar or equivalent to those described herein can be used to implement the present disclosure. The present disclosure is not limited to the methods and materials described herein. In the event that one or more of the incorporated literatures, patents, and similar materials differ from or contradict this application (including but not limited to defined terms, term applications, described technologies, etc.), this application shall prevail.

It should be further noted that some features of the present disclosure, for the sake of clarity, have been described in multiple independent embodiments, but may alternatively be provided in combination in a single embodiment. On the contrary, the various features of the present disclosure are described in a single embodiment for the sake of brevity, but they can alternatively be provided individually or in any suitable sub-combination.

Unless otherwise specified, all scientific and technological terms used in the present disclosure have the same meanings as commonly understood by those skilled in the art to which the present disclosure belongs. All patents and publications involved in the present disclosure are incorporated into the present disclosure in their entirety by reference.

Unless otherwise stated, the following definitions used herein shall apply. For the purposes of the present disclosure, the chemical elements correspond to the Periodic Table of the Elements, CAS Edition, and Handbook of Chemistry and Physics, 75th Edition, 1994. In addition, general principles of organic chemistry can be found in the descriptions of "Organic Chemistry", Thomas Sorrell, University Science Books, Sausalito: 1999, and "March's Advanced Organic Chemistry" by Michael B. Smith and Jerry March, John Wiley & Sons, New York: 2007, the entire contents of which are incorporated herein by reference.

All features disclosed in this specification may be combined in any combination. Each feature disclosed in this specification may be replaced by an alternative feature serving the same, equivalent or similar purpose. Thus, unless expressly stated otherwise, each feature disclosed is only an example of a series of equivalent or similar features.

From the above description, one skilled in the art can easily ascertain the essential features of the present disclosure, and can make various changes and modifications to the present disclosure without departing from the spirit and scope of the present disclosure to adapt this disclosure to various uses and conditions. Accordingly, other examples are also within the scope of the appended claims.

### Preparation example

### Example 1: Synthesis of MS-1

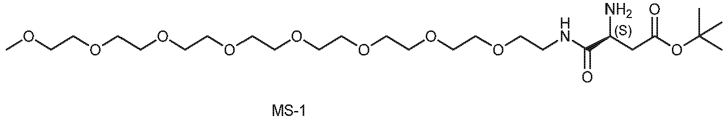

### Step 1: Synthesis of Cbz-Ms-1

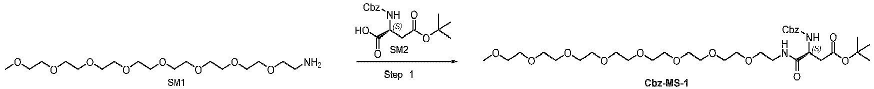

SM2 (8 g, 23.5 mmol) and 150 mL of DMF were added to a 250 mL three-neck flask, and the mixture was cooled to 0 °C under argon in an ice salt bath. SM1 (9 g, 23.5 mmol) and HBTU (13.3 g, 35.2 mmol) were added, and then DIPEA(4.5 g, 35.2 mmol) was added dropwise. After the addition was completed, the mixture was reacted at room temperature (20 °C) for 1h. The reaction was monitored by TLC (DCM:MeOH=10:1), and SM2 disappeared.

500 mL of water was added to the reaction solution, and the mixture was extracted 3 times with 200 mL of EA. The organic phase was dried over anhydrous sodium sulfate and concentrated. The crude product was purified via a silica gel column, and eluted with MeOH/DCM (2%-3%) to give 11 g (15.9 mmol, 68.0%) of product as a colorless oil.

¹H NMR (300 MHz, DMSO-d₆) δ 7.95-7.91 (m, 1H), 7.53 (d, J=8.7Hz, 1H), 737-7.34 (m, 5H), 5.10-4.98 (m, 2H), 4.38-4.30 (m, 7H), 3.51 - 3.44 (m, 26H), 3.43 - 3.38 (m, 4H), 3.24 - 3.18 (m, 5H), 2.67 - 2.60 (m, 1H), 2.48 - 2.43 (m, 1H), 1.40 (s, 9H).

### Step 2: Synthesis of Ms-1

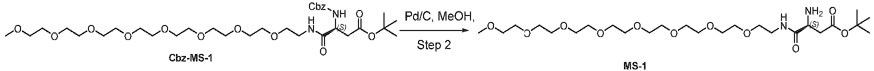

Palladium on carbon (0.2 g) was added to a solution of Cbz-Ms-1 (1.0 g, 1.5 mmol, 1.0 eq.) in methanol, and the atmosphere in the reaction system was replaced with hydrogen 5 times. The mixture was hydrogenated with stirring at 25 °C for 4 hours. The reaction solution was filtered and concentrated to dryness by rotary evaporation under reduced pressure to give 650 mg of Ms-1 (81%) as a colorless oil. This product can be directly used in the next step without further purification.

MS m/z [M+H]+(ESI): 555.45.

### Example 2: Synthesis of CS-2

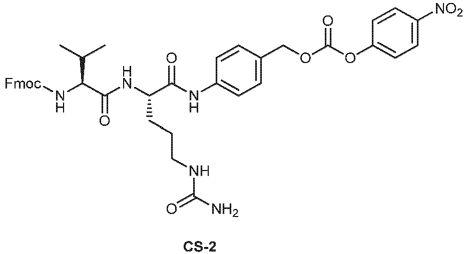

### Step 1: Synthesis of INA

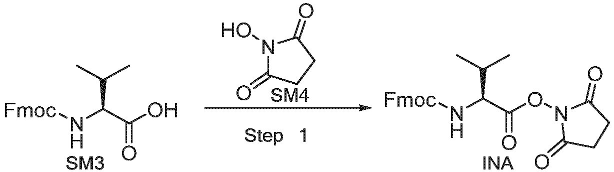

300 mL of DCM was added to a three-neck flask, then SM3 (30 g, 0.0885 mol), SM4 (13.2 g, 0.115 mol) and EDCI (22.1 g, 0.115 mol) were added, and the mixture was stirred at room temperature for 6 h. The reaction was monitored by TLC with DCM/MeOH = 10:1, and the raw material was completely converted. The reaction solution was diluted to 600 mL with dichloromethane, then washed twice with 200 mL of 0.25 M dilute hydrochloric acid and once with saturated brine, dried over anhydrous sodium sulfate, and filtered. The filtrate was rotary evaporated to dryness to give 35 g (0.08 mol, 90.39%) of white foamy solid, which was directly used in the next step without purification.

### Step 2: Synthesis of INB

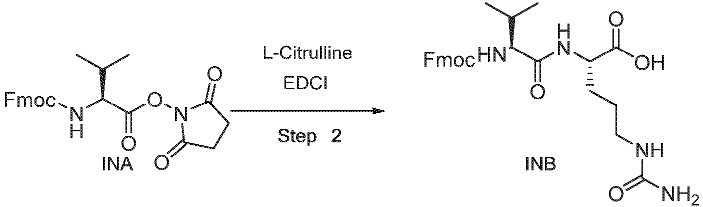

INA (35 g, 0.08 mmol) was dissolved in 400 mL of DMF, and L-citrulline (30.9 g, 0.177 mol) and sodium bicarbonate (18.5 g, 0.221 mol) were added. Then, 200 mL of water was added, and the mixture was reacted at 25 °C for 16 hours. The reaction was monitored by TLC with DCM/MeOH=10/1, and there was no raw material remaining as the reaction was completed. DMF was removed by rotary evaporation, and 500 mL of water and 50 g of citric acid were added. The mixture was slurried for 1 hour, and filtered. The solid was rotary evaporated to dryness. The solid was slurried with 500 mL of dichloromethane for 1 hour, and filtered with suction. The solid was sucked with an oil pump to dryness to give 34.5 g (0.07 mmol, 87.5%) of the target product as a white solid.

¹H NMR (300 MHz, DMSO-d₆) δ 12.40 (s, 1H), 8.15 (d, *J* = 7.3 Hz, 1H), 7.89 (d, *J* = 7.5 Hz, 2H), 7.80 - 7.69 (m, 2H), 7.46 - 7.29 (m, 6H), 6.00 - 5.88 (m, 1H), 5.37 (s, 2H), 4.30 - 4.14 (m, 4H), 3.95 - 3.89 (m, 1H), 2.98 - 2.89 (m, 2H), 2.05 - 1.87 (m, 1H), 1.83 - 1.50 (m, 2H), 1.45 - 1.31 (m, 2H), 0.87 (dd, *J =* 9.3, 6.7 Hz, 6H).

### Step 3: Synthesis of INC

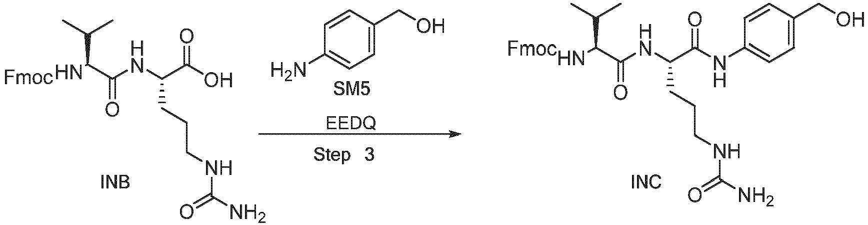

INB (26.9 g, 54.23 mmol) was added to a 3L single-neck flask, and a mixed solvent of DCM/MeOH (2L, 2:1) was added. The mixture was stirred to be dissolved until clear. SM5 (8 g, 65.08 mmol) and 2-ethoxy-1-ethoxycarbonyl-1,2-dihydroquinoline (EEDQ) (24.1 g, 97.6 mmol) were added, and the mixture was reacted at room temperature (20 °C) in the dark for 16 h. Then, the mixture was transferred to an oil bath at 30 °C and stirred for 5 h. The reaction was monitored by TLC with DCM/MeOH=5:1, and the raw material was completely converted. Most of the reaction solution was removed by rotary evaporation. The residue was transferred to a 1L single-neck flask and rotary evaporated to dryness. The residue was slurried with 500 mL of methyl tert-butyl ether for 1 h, filtered, and then slurried with 300 mL of tetrahydrofuran for 16 h. The mixture was filtered to give 20 g of white solid (33.2 mmol, 61.3%).

¹H NMR (300 MHz, DMSO-d₆) δ 9.97 (s, 1H), 8.10 (d, *J* = 7.1 Hz, 1H), 7.89 (d,*J* = 7.3 Hz, 2H), 7.78 - 7.65 (m, 2H), 7.54 (d, *J* = 8.1 Hz, 2H), 7.42 (t, *J* = 7.6 Hz, 3H), 7.32 (t, *J* = 7.3 Hz, 2H), 7.23 (d, *J =* 8.0 Hz, 2H), 6.04 - 5.88 (m, 1H), 5.47 - 5.30 (m, 2H), 5.15 - 5.00 (m, 1H), 4.50 - 4.17 (m, 6H), 3.93 (t, *J* = 7.5 Hz, 1H), 3.10 - 2.88 (m, 2H), 2.05 - 1.95 (m, 1H), 1.76 - 1.57 (m, 2H), 1.50 - 1.34 (m, 2H), 0.96 - 0.81 (m, 6H).

### Step 4: Synthesis of CS-2

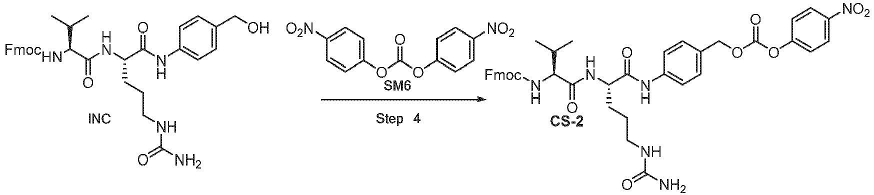

INC (20 g, 33 mmol) and 1L of DMF were added to a single-neck flask, and the mixture was stirred until completely dissolved. SM6 (63 g, 209 mmol) was added. The mixture was cooled to 0-5 °C in an ice bath, and DIPEA (27 g, 209 mmol) was added dropwise. Then, the mixture was reacted under an ice bath for 3h.

The reaction was monitored by TLC with dichloromethane:methanol = 10:1, and INC was completely converted. The reaction was stopped. The mixture was sucked with an oil pump to dryness at 55 °C. The solid was crushed, slurried with 100 mL of methyl tert-butyl ether for 1 hour, and filtered. The filter cake was slurried with 300 mL of DCM/PE=1: 1 for 1h and filtered. The filter cake was slurried again with 300mL of DCM/PE=1:1, and filtered to give 15.5g (20.2 mmol, 61.3%) of a khaki solid.

1H NMR (300 MHz, DMSO-d₆) δ 10.14 (s, 1H), 8.37 - 8.26 (m, 2H), 8.14 (d, J = 7.4 Hz, 1H), 7.89 (d, J = 7.3 Hz, 2H), 7.74 (t, J = 6.6 Hz, 2H), 7.65 (d, J = 8.4 Hz, 2H), 7.60 - 7.52 (m, 2H), 7.47 - 7.37 (m, 5H), 7.32 (t, J = 7.1 Hz, 2H), 6.02 - 5.90 (m, 1H), 5.41 (s, 2H), 5.25 (s, 2H), 4.49 - 4.37 (m, 1H), 4.34 - 4.18 (m, 3H), 3.97 - 3.88 (m, 1H), 3.09 - 2.91 (m, 2H), 2.07 - 1.92 (m, 1H), 1.76 - 1.54 (m, 2H), 1.52 - 1.30 (m, 2H), 0.93 - 0.79 (m, 6H).

### Example 3: Synthesis of FS-1

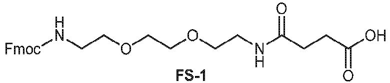

### Step 1: Synthesis of IND

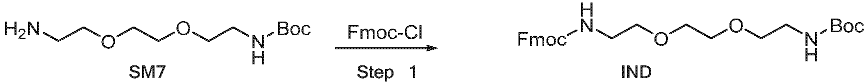

SM7 (9 g, 36.24 mmol), 1,4-dioxane (45 mL) and Na₂CO₃ aqueous solution (10%, 90 mL) were added to a single-neck flask. A solution of Fmoc-Cl (10.3 g, 39.87 mmol) in 1,4-dioxane (45 mL) was added dropwise under an ice bath, and then the mixture was reacted under an ice bath. The reaction was monitored by TLC (DCM:MeOH=10:1), and the raw material was completely converted. 300 mL of EA was added to the reaction solution. The mixture was washed twice with 100 mL of water and twice with 100 mL of saturated brine, and concentrated. The crude product was purified via a silica gel column, and eluted with EA/PE (30%-50%) to give 16.5g (35.09 mmol, 96.8%) of product as a colorless oil.

¹H NMR (300 MHz, DMSO-d₆) δ 8.00 - 7.76 (m, 3H), 7.72 - 7.65 (m, 2H), 7.44 - 7.30 (m, 5H), 4.47 - 4.10 (m, 3H), 3.52 - 3.42 (m, 4H), 3.42 - 3.32 (m, 4H), 3.23 - 3.02 (m, 4H), 1.37 (s, 9H).

### Step 2: Synthesis of INE

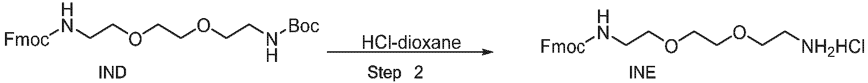

IND (16.5 g, 35.09 mmol) was added to a 250 mL single-neck flask, and HCl-dioxane solution (4 N, 50 mL) was added with stirring. The mixture was reacted at room temperature for 1h. The reaction was monitored by TLC with PE:EA=1:1, and the raw material was completely converted. The solvent was removed by rotary evaporation to give 14 g (34.4 mmol, 98.05%) of product as a colorless oil.

¹H NMR (300 MHz, DMSO-d₆) δ 8.05 (s, 3H), 7.90 (d, *J =* 7.4 Hz, 2H), 7.73 - 7.61 (m, 2H), 7.38 (dt, *J =* 13.8, 7.3 Hz, 5H), 4.44 - 4.14 (m, 3H), 3.56 - 3.51 (m, 4H), 3.49 - 3.26 (m, 4H), 3.15 (dd, *J =* 11.7, 5.8 Hz, 2H), 2.94 (dd, *J =* 10.1, 5.0 Hz, 2H).

### Step 3: Synthesis of FS-1

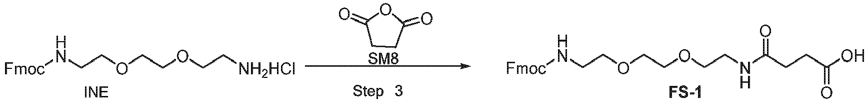

DCM (250 mL), INE (14 g, 34.4 mmol), and succinic anhydride (11.4 g, 114.2 mmol) were added to a 500 mL single-neck flask, and the mixture was cooled in an ice bath. Triethylamine (3.86 g, 38.1 mmol) was added dropwise, and then the mixture was reacted at room temperature for 3 hours. The reaction was monitored by TLC with DCM/MeOH=10/1, and the raw material was completely converted. The reaction solution was diluted with 800 mL of dichloromethane, washed twice with water (200 mL) and once with saturated brine (200 mL), dried over anhydrous sodium sulfate, and filtered. The filtrate was concentrated. The crude product was purified via a silica gel column using DCM/MeOH=20:1 as an eluent to give 15.5 g (32.9 mmol, 95.9%) of light yellow oil. ¹H NMR (300 MHz, DMSO-d₆) δ 12.08 (s, 1H), 7.98 - 7.80 (m, 3H), 7.73 - 7.60 (m, 2H), 7.37 (dt, *J* = 13.9, 7.3 Hz, 5H), 4.46 - 4.14 (m, 3H), 3.55 - 3.43 (m, 4H), 3.42 - 3.34 (m, 4H), 3.25 - 3.06 (m, 4H), 2.44 - 2.39 (m, 2H), 2.37 - 2.26 (m, 2H).

### Example 4: Synthesis of PH-HG-001-1

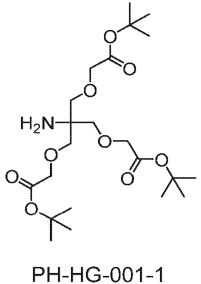

### Step 1: Synthesis of INF

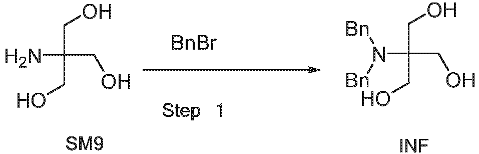

DMF (450 mL) was added to a 1L single-neck flask, and then SM9 (50 g, 0.413 mol), benzyl bromide (155 g, 0.908 mol) and K₂CO₃ powder (143 g, 1.038 mol) were added successively. The mixture was refluxed at 155 °C in an oil bath for 20 hours. The reaction was monitored by LCMS. The solvent was removed by rotary evaporation, and then 1L of dichloromethane was added. The mixture was washed twice with water (300 mL) and twice with saturated brine (200 mL), dried over anhydrous sodium sulfate, and filtered. The filtrate was rotary evaporated to dryness. 40 mL of ethyl acetate was added to the crude product. The mixture was heated to reflux until completely dissolved, then cooled and crystallized overnight. The solid was filtered, and dried in vacuum to give 55 g (0.182 mmol, 44.07%) of off-white crystals.

¹H NMR (300 MHz, DMSO-d₆) δ 7.31 - 7.00 (m, 10H), 4.29 (t, *J =* 5.1 Hz, 3H), 3.97 (s, 4H), 3.52 (d, *J* = 5.1 Hz, 6H).

### Step 2: Synthesis of Bn-LS-1

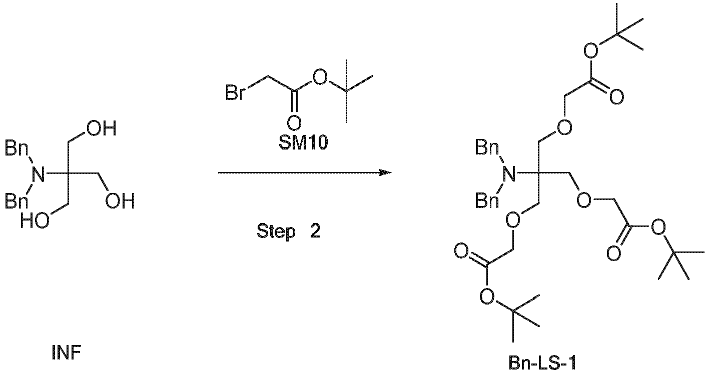

80 mL of water and sodium hydroxide (80 g, 1.99 mol) were added to a 500 mL three-neck flask. Then, DCM (160 mL), tetrabutylammonium bromide (3.2 g, 10 mmol), and INF (20 g, 66.36 mmol) were added. The mixture was stirred mechanically. The mixture was cooled to below 10 °C in an ice bath, and tert-butyl bromoacetate (52 g, 265.44 mmol) was added dropwise. After the addition was completed, the mixture was naturally warmed to 20 °C and stirred at room temperature overnight (16 h). The reaction was monitored by TLC (PE:EA=1:1), and there was no raw material remaining. 1L of DCM was added to the reaction system, and the mixture was washed twice with water and twice with saturated sodium bicarbonate aqueous solution, and concentrated. The crude product was purified via a silica gel column, and eluted with PE:EA=20:1 to give 17g of product Bn-LS-1 (26.40 mmol, 39.78%).

¹H NMR (300 MHz, DMSO-d₆) δ 7.29 - 7.19 (m, 4H), 7.17 - 7.00 (m, 6H), 3.98 (s, 4H), 3.88 (s, 6H), 3.63 (s, 6H), 1.41 (s, 27H).

### Step 3: Synthesis of PH-HG-001-1

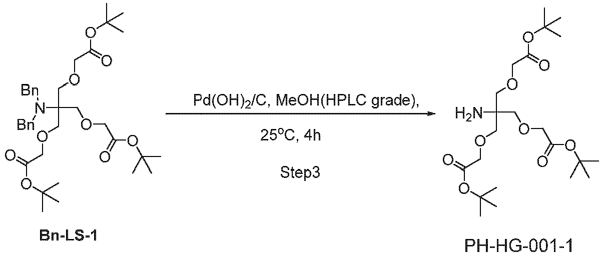

Palladium hydroxide on carbon (0.2 g) was added to a solution of Bn-LS-1 (1.0 g, 1.6 mmol, 1.0 eq.) in methanol (20 mL, chromatography grade). The atmosphere in the reaction system wasreplaced with hydrogen 5 times, and the mixture was stirred for hydrogenation at 25 °C for 4 hours. The reaction solution was filtered and concentrated to dryness by rotary evaporation under reduced pressure to give 650 mg of PH-HG-001-1 (90%) as a colorless oil. This product can be directly used in the next step without further purification.

MS m/z [M+H]⁺(ESI): 464.30.

### Example 5: Synthesis of PH-HG-001-8

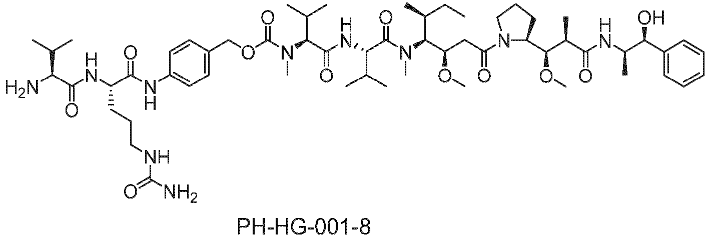

### Step 1: Synthesis of PH-HG-001-7

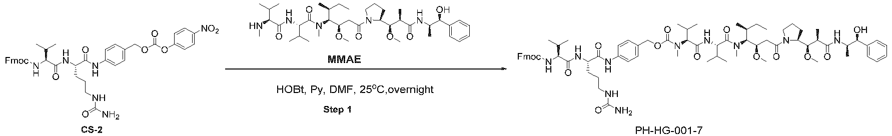

MMAE (740.2 mg, 1.05 mmol, 1.5 eq.), HOBt (18.6 mg, 0.14 mmol, 0.2 eq.) and pyridine (16.3 mg, 0.21 mmol, 0.3 eq.) were added in batches to a solution of CS-2 (527.0 mg, 0.7 mmol, 1.0 eq.) in DMF (10.0 mL). The reaction solution was stirred at 25 °C under nitrogen overnight. 100 mL of water was added to the reaction system, and the mixture was extracted with dichloromethane (3×100mL). The combined organic phase was washed with saturated brine (3× 100 mL), dried over anhydrous sodium sulfate, and filtered. The filtrate was concentrated under reduced pressure. The resulting crude product was purified by preparative HPLC (X Bridge Prep OBD C18 column; mobile phase, water (10 mmol ammonium bicarbonate) and acetonitrile (from 42.0% acetonitrile to 72.0% acetonitrile, 10 min); detector. UV 254 nm) to give 550 mg (59%) of PH-HG-001-7 as a white solid.

MS m/z [M+H]⁺(ESI): 1345.45.

### Step 2: Synthesis of PH-HG-001-8

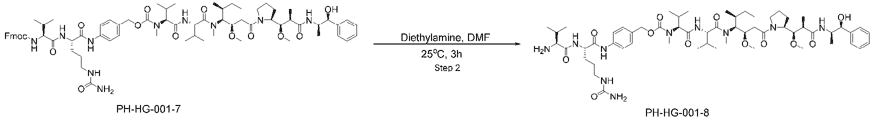

Diethylamine (4 mL) was added to PH-HG-001-7 (600.0 mg, 0.4 mmol, 1.0 eq.) in DMF (8.0 mL) under nitrogen. The reaction solution was stirred at 25 °C for 3 hours and then concentrated by rotary evaporation. The crude product was purified by Flash-Prep-HPLC (C8 column, mobile phase: water (10 mmol ammonium bicarbonate) and acetonitrile (10% to 80%), detector UV 210 nm) to give 340 mg (68%) of PH-HG-001-8 as a white solid.

MS m/z [M+H]⁺(ESI): 1123.75. ¹HNMR (300 MHz, DMSO-d₆) δ: 0.78-0.92 (m, 24H), 0.99-1.06 (m, 7H), 1.21-1.27 (m, 1H), 1.31-1.37 (m, 3H), 1.49-1.86 (m, 7H), 1.90-2.21 (m, 4H), 2.37-2.45 (m, 1H), 2.81-3.01 (m, 6H), 3.10-3.22 (m, 9H), 3.51-3.81 (m, 3H), 3.90-4.08 (m, 2H), 4.14-4.28 (m, 2H), 4.34-4.80 (m, 4H), 4.84-5.17 (m, 2H), 5.39-5.58 (m, 2H), 6.01-6.35 (m, 1H), 7.10-7.37 (m, 7H), 7.55-7.92 (m, 3H), 7.99-8.41 (m, 1H), 10.12-10.52 (m, 1H).

### Example 6: Synthesis of PH-HG-002-6

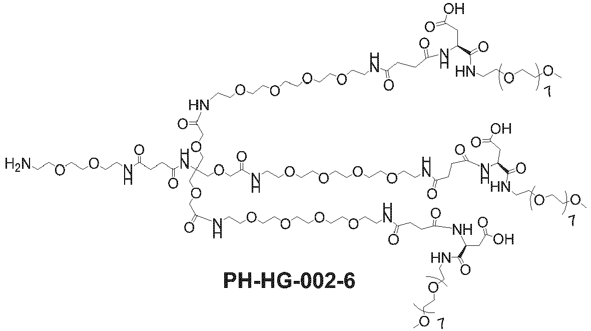

### Step 1: Synthesis of PH-HG-001-2

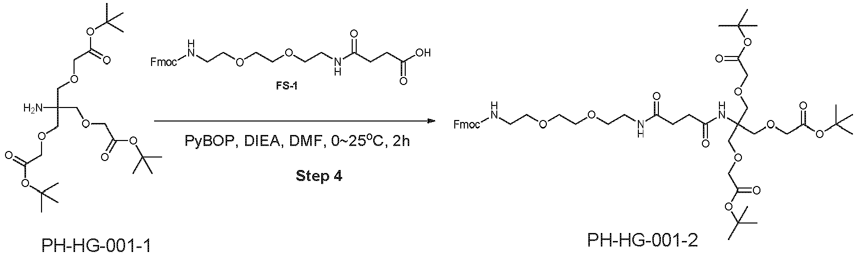

FS-1 (650.3 mg, 1.4 mmol, 1.1 eq.), N,N-diisopropylethylamine (494.4 mg, 3.8 mmol, 3.0 eq.), and 1H-benzotriazol-1-yloxytripyrrolidinophosphonium hexafluorophosphate (PyBOP) (995.4 mg, 1.9 mmol, 1.5 eq.) were added to a solution of PH-HG-001-1 (600.0 mg, 1.3 mmol, 1.0 eq.) in DMF (10.0 mL) under nitrogen at 0 °C. The reaction solution was stirred at 25 °C for 2 hours, and then the reaction was quenched with 100 mL of water. The mixture was extracted with ethyl acetate (3 × 100 mL). The combined organic phase was washed with saturated brine (3 × 100 mL), dried over anhydrous sodium sulfate, and filtered. The filtrate was concentrated by rotary evaporation. The crude product was purified via a silica gel column, and eluted with dichloromethane:methanol=30:1 to give 900 mg (77%) of PH-HG-001-2 as a colorless oil.

MS m/z [M+H]⁺(ESI): 916.50.

### Step 2: Synthesis of PH-HG-001-3

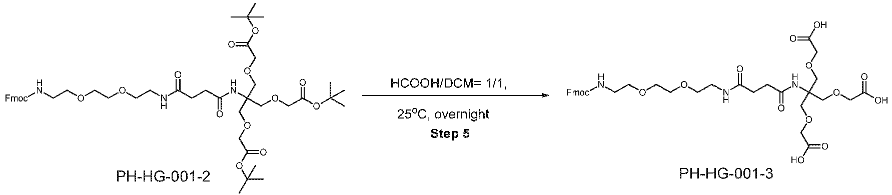

Formic acid (5.0 mL) was added to a solution of PH-HG-001-2 (1.1 g, 1.2 mmol, 1.0 eq.) in dichloromethane (5.0 mL) under nitrogen. The reaction solution was stirred at 25 °C overnight and concentrated by rotary evaporation. The crude product was purified by Flash-Prep-HPLC (conditions: C18 silica gel column; mobile phase water and acetonitrile (10% to 80% acetonitrile, 20 min), detector UV 210 nm) to give 720 mg (80%) of PH-HG-001-3 as a colorless oil.

MS m/z [M+H]⁺(ESI): 748.15.¹H NMR (400 MHz, Methanol-d₄) δ: 2.42-2.58 (m, 4H), 3.36-3.40 (m, 4H), 3.47-3.55 (m, 4H), 3.57-3.64 (m, 4H), 3.89 (s, 6H), 4.09 (s, 6H), 4.20-4.24 (m, 1H), 4.37-4.39 (m, 2H), 7.30-7.32 (m, 2H), 7.38-7.42 (m, 2H), 7.66 (d, *J =* 7.2 Hz, 2H), 7.81 (d, *J* = 7.2 Hz, 2H).

### Step 3: Synthesis of PH-HG-002-1

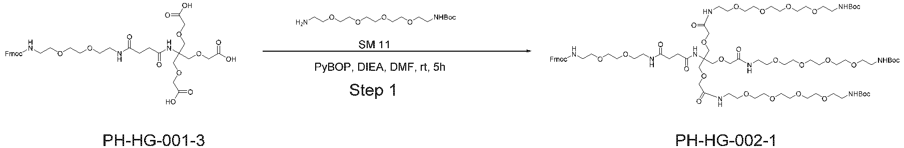

N,N-diisopropylethylamine (3.0 g, 23.2 mmol, 4.5 eq.), SM11 (5.7 g, 17.0 mmol, 3.3 eq.), and 1H-benzotriazol-1-yloxytripyrrolidinophosphonium hexafluorophosphate (PyBOP) (12.1 g, 23.3 mmol, 4.5 eq.) were added successively to a solution of PH-HG-001-3 (3.8 g, 5.1 mmol, 1.0 eq.) in DMF (60 mL) under nitrogen. The reaction solution was stirred at 25 °C for 5 hours, then diluted with 300 mL of water, and extracted with ethyl acetate (3×300 mL). The combined organic phase was washed with saturated brine (3×300mL), dried over anhydrous sodium sulfate, and filtered. The filtrate was concentrated by rotary evaporation. The crude product was purified by Flash-Prep-HPLC (conditions: C18 silica gel column; mobile phase water and acetonitrile (10% to 100% acetonitrile, 20 minutes), detector UV 210 nm) to give 6.1 g (70%) of PH-HG-002-1 as a colorless oil.

MS m/z [M+H]⁺(ESI): 1702.93. ¹H NMR (400 MHz, DMSO-d₆) δ: 1.30-1.42 (s, 27H), 2.25-2.40 (m, 4H), 3.03-3.08 (m, 6H), 3.12-3.20 (m, 4H), 3.25-3.29 (m, 6H), 3.35-3.44 (m, 18H), 3.49-3.50 (m, 38H), 3.71 (s, 6H), 3.88 (s, 6H), 4.21-4.30 (m, 3H), 6.71-6.73 (m, 3H), 7.33 (t, *J* = 7.6 Hz, 3H), 7.42 (t, *J =* 7.6 Hz, 2H), 7.68-7.75 (m, 6H), 7.88-7.90 (m, 3H).

### Step 4: Synthesis of PH-HG-002-2

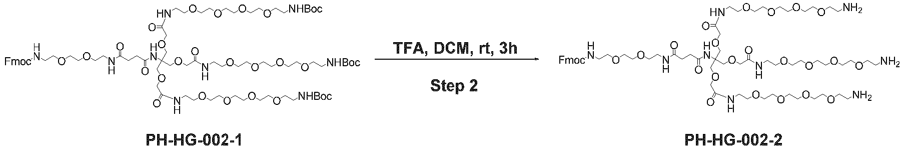

Trifluoroacetic acid (15 mL) was added to a solution of PH-HG-002-1 (3.1 g, 1.8 mmol, 1.0 eq.) in dichloromethane (15 mL) under nitrogen. The reaction solution was stirred at 25 °C for 3 hours, and then concentrated by rotary evaporation. The crude product was purified by Flash-Prep-HPLC (conditions: C18 silica gel column; mobile phase water and acetonitrile (10% to 80% acetonitrile, 20 minutes), detector UV 210 nm) to give 1.6 g (64%) of PH-HG-002-2 as a colorless oil.

MS m/z [M/2+H]+ (ESI): 1402.77.

### Step 5: Synthesis of PH-HG-002-3

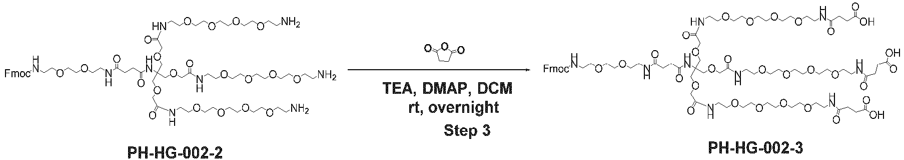

Succinic anhydride (1.9 g, 19.3 mmol, 9.0 eq.), triethylamine (2.6 g, 25.7 mmol, 12.0 eq.), and 4-dimethylaminopyridine (0.8 g, 6.4 mmol, 3.0 eq.) were added to a solution of PH-HG-002-2 (3.0 g, 2.1 mmol, 1.0 eq.) in dichloromethane (45 mL) under nitrogen at 0 °C. The reaction solution was stirred at 25 °C for 12 hours, and then concentrated by rotary evaporation. The crude product was purified by Flash-Prep-HPLC (conditions: C18 silica gel column; mobile phase water and acetonitrile (10% to 80% acetonitrile, 30 minutes), detector UV 210 nm) to give 1.6 g (44%) of PH-HG-002-3 as a colorless oil.

MS m/z [M/2+H]⁺ (ESI): 1402.77. ¹H NMR (300 MHz, DMSO-d₆) δ: 2.34-2.39 (m, 10H), 2.41-2.44 (m, 6H), 3.13-3.28 (m, 16H), 3.37-3.51 (m, 56H), 3.72 (s, 6H), 3.89 (s, 6H), 4.19-4.31 (m, 3H), 7.32 (t, *J* = 7.5 Hz, 3H), 7.43 (t, *J* = 7.5 Hz, 2H), 7.68-7.77 (m, 6H), 7.88-7.91 (m, 6H), 12.00-12.06 (m, 2H).

### Step 6: Synthesis of PH-HG-002-4

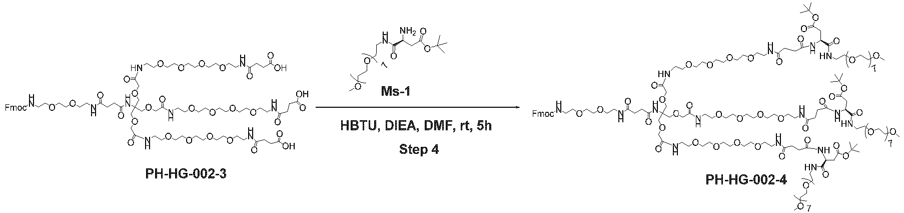

Ms-1 (1.7 g, 3.1 mmol, 3.3 eq.), N,N-diisopropylethylamine (546.5 mg, 4.2 mmol, 4.5 eq.) and O-(1H-benzotriazol-1-yl)-N,N,N',N'-tetramethyluronium hexafluorophosphate (HBTU) (1.6 g, 4.2 mmol, 4.5 eq.) were added to a solution of PH-HG-002-3 (1.6 g, 0.9 mmol, 1.0 eq.) in DMF (30 mL) under nitrogen at 0 °C. The reaction solution was stirred at 25 °C for 5 hours, then diluted with 300 mL of water, and extracted with ethyl acetate (3×300 mL). The combined organic phase was washed with saturated brine (3×300 mL), dried over anhydrous sodium sulfate, and filtered. The filtrate was concentrated by rotary evaporation. The crude product was purified by Flash-Prep-HPLC (conditions: C18 silica gel column; mobile phase water (with 0.1% formic acid) and acetonitrile (10% to 100% acetonitrile, 20 minutes), detector UV 210 nm) to give 1.7 g (55%) of PH-HG-002-4 as a yellow oil.

MS m/z [M/2+H]⁺(ESI): 1657.15.

### Step 7: Synthesis of PH-HG-002-5

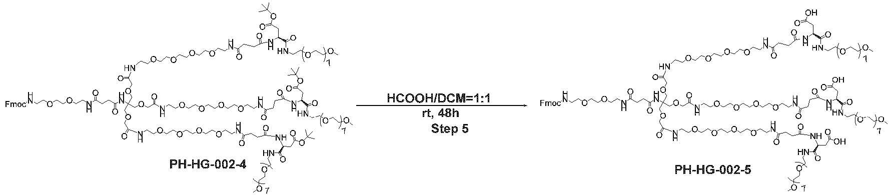

Formic acid (5.0 mL) was added to a solution of PH-HG-002-4 (1.0 g, 0.3 mmol, 1.0 eq.) in dichloromethane (5 mL) under nitrogen. The reaction solution was stirred at 25 °C for 48 hours, and then concentrated by rotary evaporation. The crude product was purified by Flash-Prep-HPLC (conditions: C18 silica gel column; mobile phase water (with 0.1% formic acid) and acetonitrile (10% to 80% acetonitrile, 30 minutes), detector UV 210 nm) to give 700 mg (74%) of PH-HG-002-5 as a colorless oil.

MS m/z [M/2+H]⁺(ESI): 1573.20. ¹H NMR (300 MHz, Methanol-d₄) δ: 2.48-2.58 (m, 16H), 2.65-2.84 (m, 6H), 3.20-3.30 (m, 3H), 3.33-3.47 (m, 30H), 3.53-3.65 (m, 144H), 3.88 (s, 6H), 4.03 (s, 6H), 4.20-4.40 (m, 3H), 4.73-4.85 (m, 3H), 7.34-7.42 (m, 4H), 7.67 (m, 2H), 7.83 (d, *J* = 7.5 Hz, 2H).

### Step 8: Synthesis of PH-HG-002-6

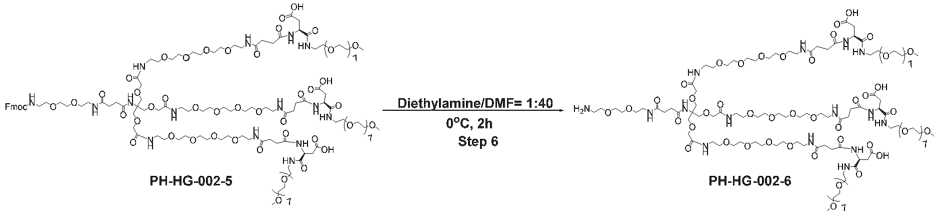

Diethylamine (0.1 mL) was added to a solution of PH-HG-002-5 (300 mg, 0.1 mmol, 1.0 eq.) in DMF (4 mL) under nitrogen at 0 °C. The reaction solution was stirred at 0 °C for 2 hours and then purified by preparative HPLC (conditions: X Select CSH Prep C18 OBD column; size 19 mm×250 mm, 5 µm; mobile phase, water (0.05% formic acid) and acetonitrile (8.0% to 23.0% acetonitrile, 11 min); detector, UV 200 nm) to give 140 mg (50%) of product PH-HG-002-6 as a colorless oil.

MS m/z [M/2+H]⁺ (ESI): 1461.95.

### Example 7: Synthesis of FS-2

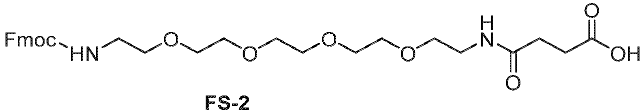

### Step 1: Synthesis of ING

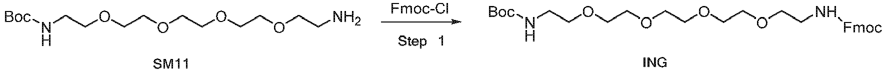

A solution of Fmoc-Cl (4.23 g, 16.0 mmol) in 1,4-dioxane (25 mL) was added dropwise to a solution of SM11 (5.0 g, 14.8 mmol) in 1,4-dioxane (25 mL) and Na₂CO₃ solution (10%, 50 mL) at 0 °C. The reaction solution was stirred at 0 °C for 2 hours, and then extracted with ethyl acetate (200 mL). The organic phase was washed with water (2×200 mL), washed with saturated brine (2×200 mL), and concentrated. The crude product was purified via a silica gel column, eluted with DCM/MeOH=50:1 to 10:1, and concentrated to give 7.0 g (84.5%) of product ING as a colorless oil.

¹H NMR (300 MHz, DMSO) δ 8.00 - 7.80 (m, 3H), 7.76 - 7.61 (m, 2H), 7.44 - 7.30 (m, 5H), 4.47 - 4.10 (m, 3H), 3.66 - 3.56 (m, 4H), 3.53 - 3.46 (m, 12H), 3.34 - 3.14 (m, 4H), 1.46 (s, 9H).

### Step 2: Synthesis of INH

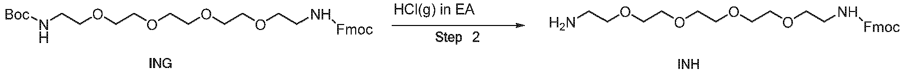

A solution of HCl (g) in ethyl acetate (4 N, 50 mL) was added dropwise to a solution of ING (7.0 g, 12.0 mmol) in ethyl acetate (20 mL) at 0 °C. The reaction solution was stirred at room temperature for 3 h, and then some of the solvent was removed by rotary evaporation. The residue was slurried with n-hexane (15 mL) to give 6.0 g (104%) of product INH as a white solid.

¹H NMR (300 MHz, DMSO) δ 8.03(s, 3H), 7.92 (d, J = 7.4 Hz, 2H), 7.73 - 7.61 (m, 2H), 7.34 (dt, J = 13.8, 7.3 Hz, 4H), 4.65 -4.14 (m, 3H), 3.69-3.58 (m, 4H), 3.52 -3.42 (m,12H), 3.15 (dd, J = 11.7, 5.8 Hz, 2H), 2.96 (dd, J = 10.1, 5.0 Hz, 2H).

### Step 3: Synthesis of FS-2

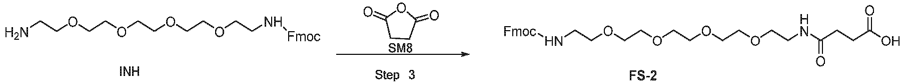

Triethylamine (1.35 g, 13 mmol) was added to a solution of DCM (60 mL) and INH (6.0 g, 10 mmol) in dichloromethane (60 mL) at 0 °C. The mixture was stirred at room temperature for 5 min, and then succinic anhydride (2.4 g, 20 mmol) was added. After the addition was completed, the mixture was heated to 40 °C and reacted for 3 h. The reaction was monitored by TLC (DCM/MeOH=10/1), which showed that the raw material was completely converted. The reaction solution was diluted with 200 mL of dichloromethane, washed twice with water (100 mL), washed once with saturated brine (100 mL), dried over anhydrous sodium sulfate, filtered, and concentrated. The crude product was purified via a silica gel column, and eluted with DCM/MeOH=50:1 to give 5.37 g (73.5%) of FS-2 as a yellow oil.

¹H NMR (300 MHz, DMSO) δ 12.04 (s, 1H), 7.98 - 7.80 (m, 3H), 7.73 - 7.62 (m, 2H), 7.37 (dt, *J =* 13.9, 7.3 Hz, 5H), 4.46 - 4.14 (m, 3H), 3.55 - 3.43 (m, 12H), 3.45 - 3.34 (m, 4H), 3.25 - 3.15 (m, 4H), 2.48 - 2.38 (m,2H), 2.37 - 2.28 (m, 2H).

### Example 8: Synthesis of BCN-EtOSu

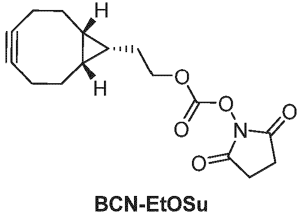

### Step 1: Synthesis of BCN-EtOH-1

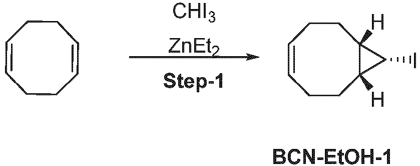

A solution of diethylzinc in n-hexane (1.0 M, 35.0 mL, 35.4 mmol, 1.4 eq.) was slowly added dropwise to a mixture of iodoform (10.0 g, 25.4 mmol, 1.0 eq.) and 1,5-cyclooctadiene (27.5 g, 254 mmol, 10 eq.) under argon at 0 °C. After the addition was completed, the mixture was stirred until the mixture was naturally warmed to room temperature. The mixture was reacted overnight for 16 hours, and then the reaction was quenched with dilute hydrochloric acid (1M, 35 mL). The layers were separated. The aqueous phase was extracted with dichloromethane (35 mL×2). The combined organic phase was dried over anhydrous sodium sulfate, filtered, and concentrated by rotary evaporation. The crude product was distilled under reduced pressure using an oil pump to give 2.55 g (39.4%) of BCN-EtOH-1 as a black oil.

¹H NMR (400 MHz, Chloroform-*d*) δ 5.69 - 5.58 (m, 2H), 3.08 (t, *J =* 7.8 Hz, 1H), 2.48 - 2.35 (m, 2H), 2.22 - 2.10 (m, 2H), 2.08 - 1.96 (m, 2H), 1.71 - 1.58 (m, 2H), 1.00 - 0.87 (m, 2H).

### Step 2: Synthesis of BCN-EtOH-2

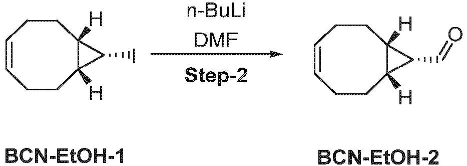

An n-butyllithium solution (2.5 M 8.2 mL, 20 mmol, 2.0 eq.) was added dropwise to a solution of BCN-EtOH-1 (2.55 g, 10 mmol, 1.0 eq.) in tetrahydrofuran (20 mL) under argon at - 70 °C. The reaction solution was stirred at -78 °C for 30 minutes and then DMF (4.51 g, 60 mmol, 6.0 eq.) was added dropwise. After the addition was completed, the reaction solution was naturally warmed to room temperature and stirred for 16 hours. The reaction was quenched with saturated ammonium chloride (40 mL), and the layers were separated. The aqueous phase was extracted with ethyl acetate (40 mL×2). The combined organic phase was dried over anhydrous sodium sulfate, filtered, and concentrated by rotary evaporation. The crude product was purified by silica gel column chromatography, and eluted with PE/EA=20/1 to give 1.1 g (7.32 mmol) of product BCN-EtOH-2.

MS m/z [M+H]⁺ (ESI): 151.

### Step 3: Synthesis of BCN-EtOH-3

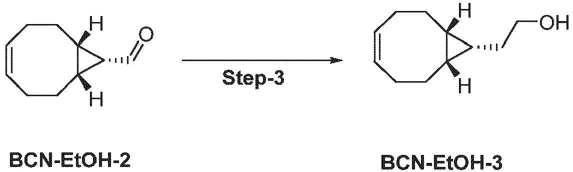

Potassium tert-butoxide (1.23 g, 10.1 mmol, 1.5 eq.) was added to a solution of (methoxymethyl)triphenylphosphonium chloride (3.76 g 10.1 mmol 1.5 eq.) in tetrahydrofuran (11 mL) under argon at 0 °C. The reaction solution was stirred at 0 °C for 30 minutes, and then a solution of compound BCN-EtOH-2 (1.1 g, 7.32 mmol, 1.0 eq.) in THF (5 mL) was added dropwise. The above mixture was stirred at 25 °C for 30 minutes and then poured into a mixed solvent of dichloromethane/water (22 mL/22 mL). The layers were separated, and the aqueous phase was extracted with dichloromethane (10 mL×2). The combined organic phase was dried over anhydrous sodium sulfate, filtered, and concentrated. The above obtained substance was dissolved in tetrahydrofuran (22 mL) and dilute hydrochloric acid (1M, 11 mL), and the mixture was heated to 70 °C and stirred for 40 minutes. The reaction solution was cooled and then poured into a mixture of water/ethyl acetate (22 mL/22 mL). The layers were separated, and the aqueous phase was extracted with ethyl acetate (10 mL×2). The combined organic phase was dried over anhydrous sodium sulfate, filtered, and concentrated. Sodium borohydride (420 mg, 10.1 mmol, 1.5 eq.) was added to a solution of the above obtained substance in methanol (22 mL) under argon at 0 °C. The mixture was reacted at 0 °C for 20 minutes, and then quenched with saturated ammonium chloride solution (10 mL). The reaction solution was poured into water/EA (22 mL/22 mL). The layers were separated, and the aqueous phase was extracted with ethyl acetate (10 mL×2). The combined organic phase was dried over anhydrous sodium sulfate, filtered, and concentrated. The crude product was purified via a silica gel column (with an eluent ratio of PE/EA=10/1) to give 700 mg (57.4%) of BCN-EtOH-3 as a colorless oil.
MS m/z [M+H]⁺ (ESI): 167;
¹H NMR (400 MHz, Chloroform-d) δ 5.67 - 5.57 (m, 2H), 3.70 (t, J = 6.9 Hz, 2H), 2.39 - 2.27 (m, 2H), 2.16 - 2.03 (m, 2H), 1.97 - 1.84 (m, 2H), 1.59 - 1.41 (m, 5H), 0.95 - 0.70 (m, 3H).

### Step 4: Synthesis of BCN-EtOH-4

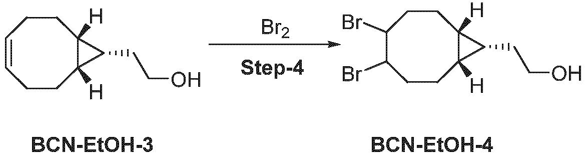

Liquid bromine (705 mg, 4.41 mmol, 1.05 eq.) was added dropwise to a solution of BCN-EtOH-3 (700 mg, 4.2 mmol, 1.0 eq.) in dichloromethane (25 mL) at 0 °C. The reaction solution was stirred at 0 °C for 20 minutes and then quenched with saturated sodium sulfite (20 mL). The organic phase was washed with water (20 mL) and saturated brine (20 mL), dried over anhydrous sodium sulfate, filtered, and concentrated to give 1.3 g (94.9%) of BCN-EtOH-4 as a colorless oil.

MS m/z [M+H]⁺ (ESI): 327.

### Step 5: Synthesis of BCN-EtOH

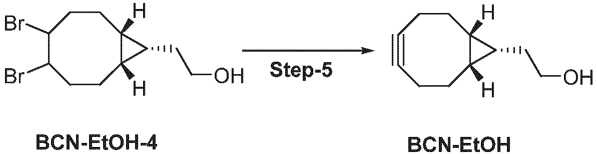

A solution of potassium tert-butoxide (1.48 g, 13.2 mmol, 3.3 eq.) in tetrahydrofuran (15 mL) was added dropwise to a solution of BCN-EtOH-4 (1.3 g, 3.99 mmol, 1.0 eq.) in tetrahydrofuran (15 mL) under argon at 0 °C. The reaction solution was stirred at 70 °C for 5 hours, and then diluted with 50 mL of water. The mixture was extracted with ethyl acetate (3×30 mL). The combined organic phase was washed with saturated brine (3×30 mL), dried over anhydrous sodium sulfate, filtered, and concentrated by rotary evaporation. The crude product was purified via a silica gel column with an eluent ratio of PE/EA= 10/1 to give 490 mg (74.8%) of BCN-EtOH as a colorless oil.

¹H NMR (400 MHz, Chloroform-d) δ 3.71 (t, J = 6.9 Hz, 2H), 2.39 - 2.16 (m, 6H), 1.70 (s, 1H), 1.65 - 1.47 (m, 4H), 1.06 - 0.97 (m, 1H), 0.90 - 0.76 (m, 2H).

### Step 6: Synthesis of BCN-EtOSu

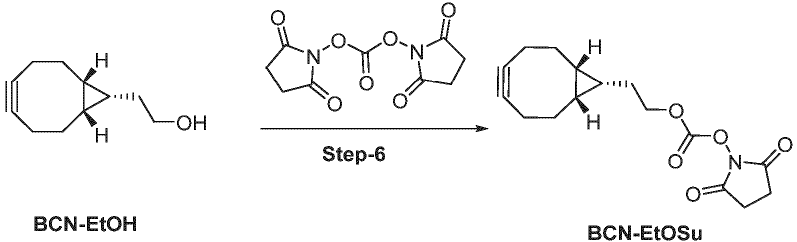

A solution of triethylamine and N,N'-disuccinimidyl carbonate (1.53 g, 5.98 mmol, 2.0 eq.) in acetonitrile (15 mL) was added to a solution of BCN-EtOH (490 mg, 2.98 mmol, 1.0 eq.) in acetonitrile (15 mL) at 0 °C. The reaction solution was stirred at 0 °C for 2 hours, then diluted with 50 mL of water, and extracted with ethyl acetate (3×30 mL). The combined organic phase was washed with saturated brine (3 ×30 mL), dried over anhydrous sodium sulfate, filtered, and concentrated by rotary evaporation. The crude product was purified via a silica gel column, and eluted with DCM as an eluent to give 150 mg (16.5%) of BCN-EtOSu as a white solid.
MS m/z [M+H]⁺(ESI):306;
¹H NMR (400 MHz, Chloroform-*d*) δ 4.41 (t, *J* = 6.9 Hz, 2H), 2.87 (s, 4H), 2.44 - 2.17 (m, 6H), 1.87 - 1.76 (m, 2H), 1.66 - 1.52 (m, 2H), 1.09 - 1.00 (m, 1H), 0.94 - 0.87 (m, 2H).

### Example 9: Synthesis of PH-HG-021-0

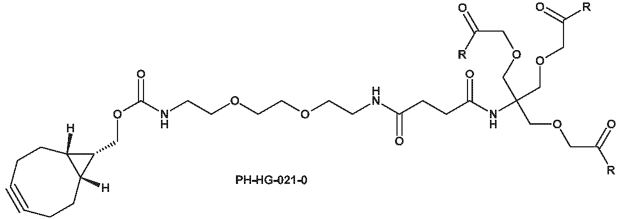

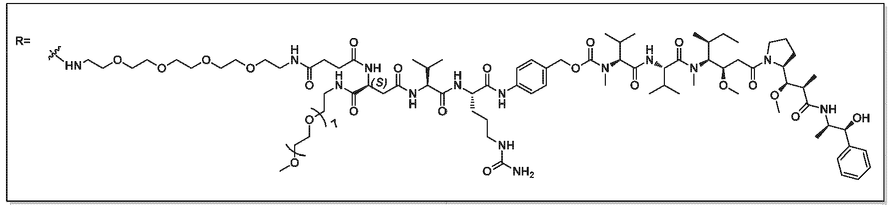

### Step 1: Synthesis of PH-HG-021-1

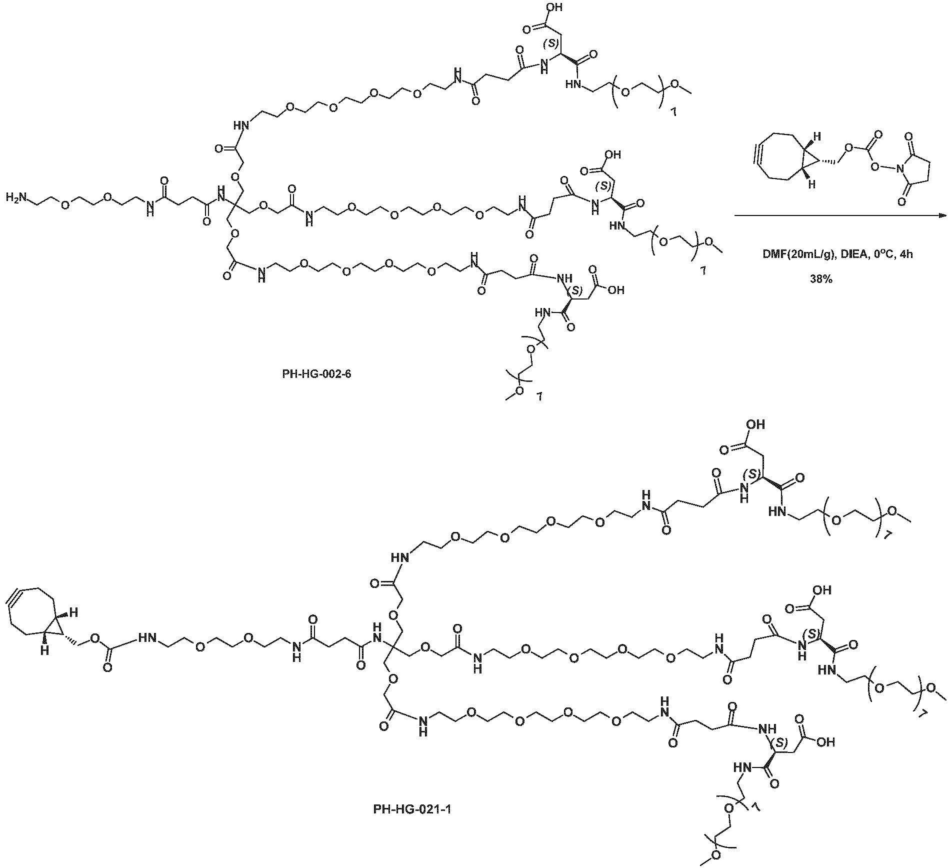

(1R,8S,9S)-Bicyclo[6.1.0]non-4-yn-9-ylmethyl succinimidyl carbonate (BCN-MeOSuendo) (32.4 mg, 0.111 mmol, 1.3 eq.) and N,N-diisopropylethylamine (33.2 mg, 0.258 mmol, 3.0 eq.) were added to a solution of PH-HG-002-6 (250.0 mg, 0.086 mmol, 1.0 eq.) in DMF (5.0 mL) under nitrogen at 0 °C. The reaction solution was stirred at 0 °C for 4 hours and then purified by preparative HPLC (conditions: Xselect CSH OBD C18 column; size 30 mm × 150 mm, 5 µm; mobile phase, water (with 0.1% formic acid) and acetonitrile (20.0% to 38.0% acetonitrile, 7 min); detector, UV 220 nm) to give 110 mg (38%) of PH-HG-021-1 as a colorless oil.

MS m/z [M/3+H]⁺ (ESI): 1033.85.

### Step 2: Synthesis of PH-HG-021-0

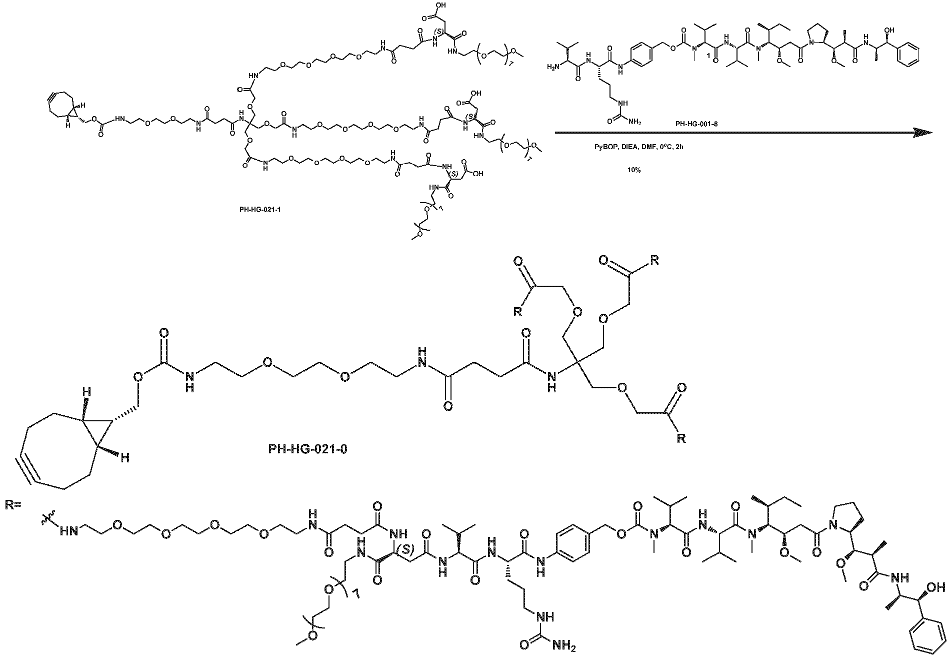

PH-HG-001-8 (273.65 mg, 0.244 mmol, 3.3 eq.), N,N-diisopropylethylamine (28.6 mg, 0.222 mmol, 3.0 eq.) and 1H-benzotriazol-1-yloxytripyrrolidinophosphonium hexafluorophosphate (PyBOP) (172.8 mg, 0.333 mmol, 4.5 eq.) were added to a solution of PH-HG-021-1 (110.0 mg, 0.074 mmol, 1.0 eq.) in DMF (3.0 mL) under nitrogen at 0 °C. The reaction solution was stirred at 0 °C for 2 hours and then purified by preparative HPLC (conditions: Xselect CSH C18 OBD column; size 30 mm × 150 mm, 5 µm; mobile phase, water (with 0.1% formic acid) and acetonitrile (44.0% to 58.0% acetonitrile, 9 min); detector, UV 254 nm) to give 23.6 mg (10%) of PH-HG-021-0 as a white solid.

MS m/z [M/4+H]⁺ (ESI): 1604.60. ¹H NMR (300 MHz, DMSO-d₆) δ: 0.56-0.92 (m, 94H), 0.95-1.06 (m, 21H), 1.23-1.59 (m, 21H), 1.60-1.89 (m, 17H), 1.91-2.18 (m, 20H), 2.20-2.48 (m, 29H), 2.68-2.90 (m, 15H), 2.95-3.05 (m, 12H), 3.10-3.22 (m, 36H), 3.23-3.30 (m, 34H), 3.32-3.45 (m, 51H), 3.49-3.50 (m, 25H), 3.50-3.52 (m, 6H), 3.55-3.60 (m, 3H), 3.71 (s, 8H), 3.75-3.81 (m, 3H), 3.90 (s, 9H), 3.91-4.10 (m, 16H), 4.13-4.79 (m, 29H), 4.94-5.15 (m, 6H), 6.03 (s, 1H), 6.91-7.09 (m, 1H), 7.10-7.32 (m, 25H), 7.60-7.67 (m, 8H), 7.73-8.02 (m, 18H), 8.07-8.38 (m, 10H), 9.71 (s, 3H).

### Example 10: Synthesis of PH-HG-022-0

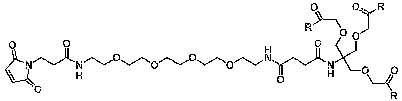

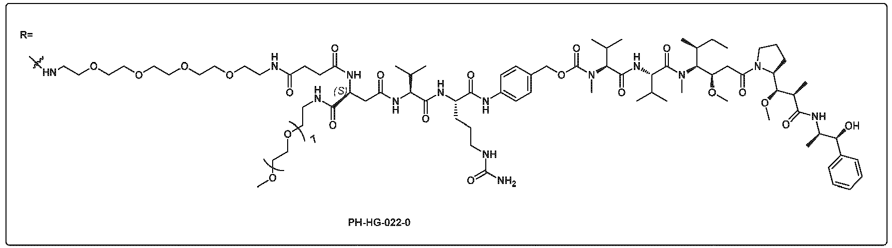

### Step 1: Synthesis of PH-HG-022-1

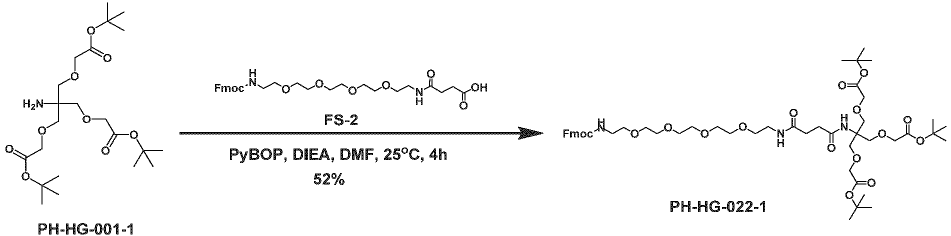

FS-2 (7.2 g, 12.95 mmol, 1.5 eq.), N,N-diisopropylethylamine (1.7 g, 12.95 mmol, 1.5 eq.) and PyBOP (6.7 g, 12.95 mmol, 1.5 eq.) were added to a solution of PH-HG-001-1 (4.0 g, 8.63 mmol, 1.0 eq.) in DMF (40.0 mL) under nitrogen at 0 °C. The reaction solution was stirred at 25 °C for 4 hours, then quenched with 300 mL of water, and extracted with dichloromethane (3 × 500 mL). The combined organic phase was washed with saturated brine (3 × 500 mL), dried over anhydrous sodium sulfate, filtered, and concentrated by rotary evaporation. The crude product was purified by Flash-Prep-HPLC (conditions: C18 silica gel column; mobile phase water (0.1% formic acid) and acetonitrile (10% to 100% acetonitrile, 20 minutes), detector UV 254 nm) to give 4.9 g (52%) of PH-HG-022-1 as a colorless oil.

¹H NMR (300 MHz, DMSO-d₆) δ 1.40 (s, 27H), 2.06 (s, 4H), 2.18-2.39 (m, 4H), 3.05-3.22 (m, 3H), 3.33-3.43 (m, 3H), 3.48 (t, *J =* 2.0 Hz, 10H), 3.70 (s, 6H), 3.95 (s, 6H), 4.15-4.32 (m, 3H), 7.23-7.47 (m, 6H), 7.68 (d, *J =* 7.4 Hz, 2H), 7.78-7.92 (m, 3H).

### Step 2: Synthesis of PH-HG-022-2

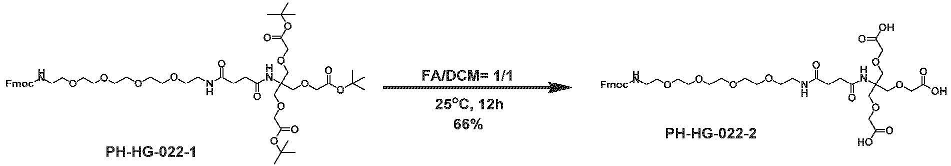

Formic acid (50.0 mL) was added to a solution of PH-HG-022-1 (4.9 g, 4.88 mmol, 1.0 eq.) in dichloromethane (50.0 mL) under nitrogen. The reaction solution was stirred at 25 °C overnight and concentrated by rotary evaporation. The crude product was purified by Flash-Prep-HPLC (conditions: C18 silica gel column; mobile phase water (0.1% trifluoroacetic acid) and acetonitrile (10% to 100% acetonitrile, 15 minutes), detector UV 254 nm) to give 2.7 g (66%) of PH-HG-022-2 as a yellow oil.

MS m/z [M+H]⁺(ESI): 836.40.

### Step 3: Synthesis of PH-HG-022-3

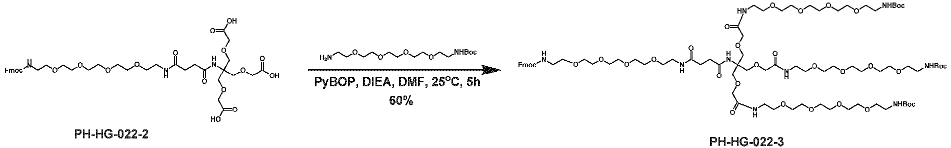

N,N-diisopropylethylamine (1.7 g, 12.92 mmol, 4.0 eq.), tert-butyl (14-amino-3,6,9,12-tetraoxatetradecyl)carbamate (3.6 g, 10.66 mmol, 3.3 eq.) and PyBOP (6.7 g, 12.92 mmol, 4.0 eq.) were added successively to a solution of PH-HG-022-2 (2.7 g, 3.23 mmol, 1.0 eq.) in DMF (30 mL) under nitrogen. The reaction solution was stirred at 25 °C for 5 hours, then diluted with 300 mL of added water, and extracted with dichloromethane (3×300 mL). The combined organic phase was washed with saturated brine (3×300 mL), dried over anhydrous sodium sulfate, filtered, and concentrated by rotary evaporation. The crude product was purified by Flash-Prep-HPLC (conditions: C18 silica gel column; mobile phase water (0.1% formic acid) and acetonitrile (10% to 100% acetonitrile, 20 minutes), detector UV 210 nm) to give 3.45 g (60%) of PH-HG-022-3 as a colorless oil.

MS m/z [M+H]⁺ (ESI): 1791.20.

### Step 4: Synthesis of PH-HG-022-4

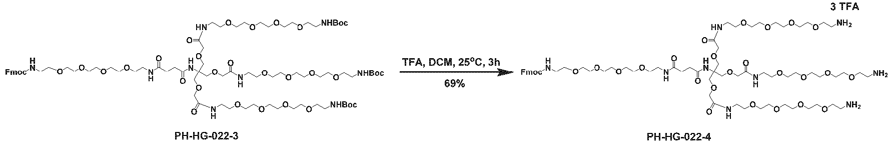

Trifluoroacetic acid (25.0 mL) was added to a solution of PH-HG-022-3 (2.8 g, 1.56 mmol, 1.0 eq.) in dichloromethane (25 mL) under nitrogen. The reaction solution was stirred at 25 °C for 3 hours and then concentrated by rotary evaporation. The crude product was purified by Flash-Prep-HPLC (conditions: C18 silica gel column; mobile phase water (0.1% trifluoroacetic acid) and acetonitrile (10% to 100% acetonitrile, 20 minutes), detector UV 254 nm) to give 1.6 g (69%) of PH-HG-022-4 as a light yellow oil. MS m/z [M+H]⁺ (ESI): 1491.05.

### Step 5: Synthesis of PH-HG-022-5

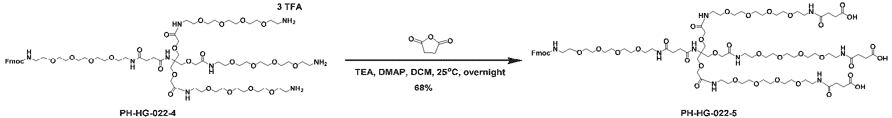

Succinic anhydride (0.43 g, 4.28 mmol, 4.0 eq.), triethylamine (0.49 g, 4.82 mmol, 4.5 eq.), and 4-dimethylaminopyridine (66 mg, 0.54 mmol, 0.5 eq.) were added to a solution of PH-HG-022-4 (1.6 g, 1.07 mmol, 1.0 eq.) in dichloromethane (20 mL) under nitrogen at 0 °C. The reaction solution was stirred at 25 °C for 12 hours and then concentrated by rotary evaporation. The crude product was purified by Flash-Prep-HPLC (conditions: C18 silica gel column; mobile phase: water (0.1% formic acid) and acetonitrile (10% to 100% acetonitrile, 18 minutes), detector UV 254 nm) to give 1.3 g (68%) of PH-HG-022-5 as a colorless oil.

MS m/z [M+H]⁺(ESI): 1791.00.

### Step 5: Synthesis of PH-HG-022-6

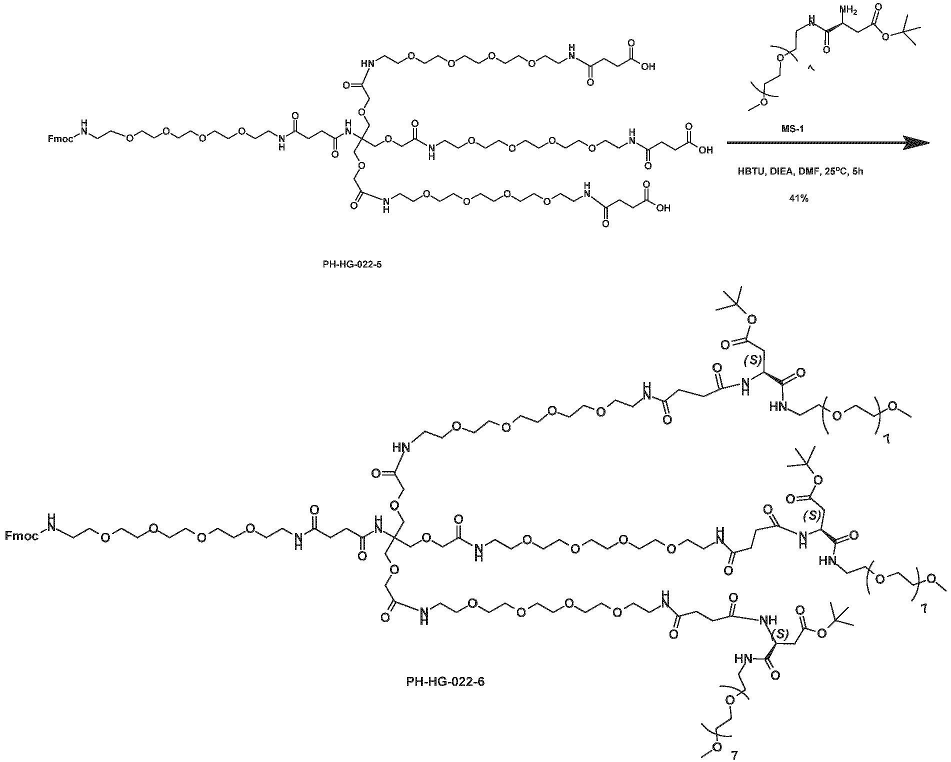

MS-1 (1.34 g, 2.41 mmol, 3.3 eq.), N,N-diisopropylethylamine (420 mg, 3.29 mmol, 4.5 eq.) and O-(1H-benzotriazol-1-yl)-N,N,N',N'-tetramethyluronium hexafluorophosphate (HBTU) (1.25 g, 3.29 mmol, 4.5 eq.) were added to a solution of PH-HG-022-5 (1.30 g, 0.73 mmol, 1.0 eq.) in DMF (15 mL) under nitrogen at 25 °C. The reaction solution was stirred at 25 °C for 5 hours, then diluted with 300 mL of water, and extracted with dichloromethane (3×500 mL). The combined organic phase was washed with saturated brine (3×500 mL), dried over anhydrous sodium sulfate, filtered, and concentrated by rotary evaporation. The crude product was purified by Flash-Prep-HPLC (conditions: C18 silica gel column; mobile phase water (with 0.1% formic acid) and acetonitrile (10% to 100% acetonitrile, 20 minutes), detector UV 210 nm) to give 1.0 g (41%) of PH-HG-022-6 as a white solid.

MS m/z [M/2+H]⁺ (ESI): 1701.10.

### Step 6: Synthesis of PH-HG-022-7

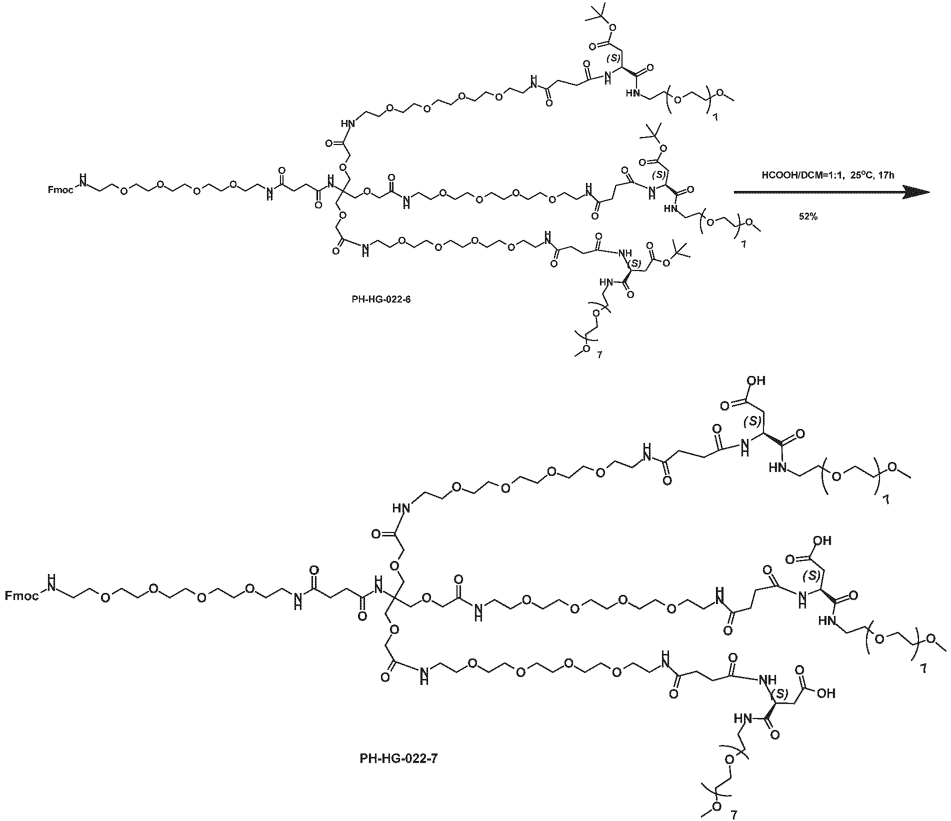

Formic acid (7.0 mL) was added to a solution of PH-HG-022-6 (1.0 g, 0.29 mmol, 1.0 eq.) in dichloromethane (7.0 mL) under nitrogen at 25 °C. The reaction solution was stirred at 25 °C for 17 hours, and then concentrated by rotary evaporation. The crude product was purified by Flash-Prep-HPLC (conditions: C18 silica gel column; mobile phase water (with 0.1% formic acid) and acetonitrile (10% to 80% acetonitrile, 30 minutes), detector UV 210 nm) to give 630 mg (52%) of PH-HG-022-7 as a white solid. MS m/z [M/2+H]⁺(ESI): 1616.95.

### Step 7: Synthesis of PH-HG-022-8

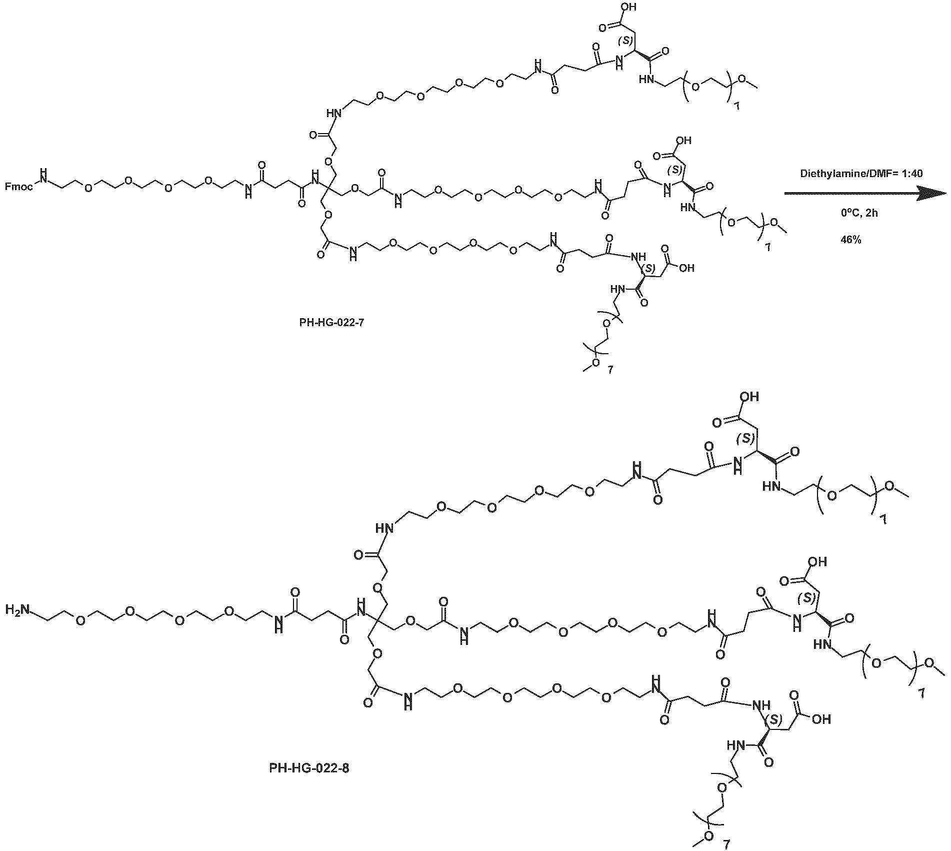

Diethylamine (0.1 mL) was added to a solution of PH-HG-022-7 (490 mg, 0.15 mmol, 1.0 eq.) in DMF (4.0 mL) under nitrogen at 0 °C. The reaction solution was stirred at 0 °C for 2 hours and then purified by preparative HPLC (conditions: XBridge Prep C18 OBD column; size 30 mm×150 mm, 5 µm; mobile phase, water (0.01% formic acid) and acetonitrile (35.0% to 65.0% acetonitrile, 7 min); detector, UV 254 nm) to give 210 mg (46%) of PH-HG-022-8 as a white solid. MS m/z [M/2+H]⁺ (ESI): 1505.85.

### Step 8: Synthesis of PH-HG-022-9

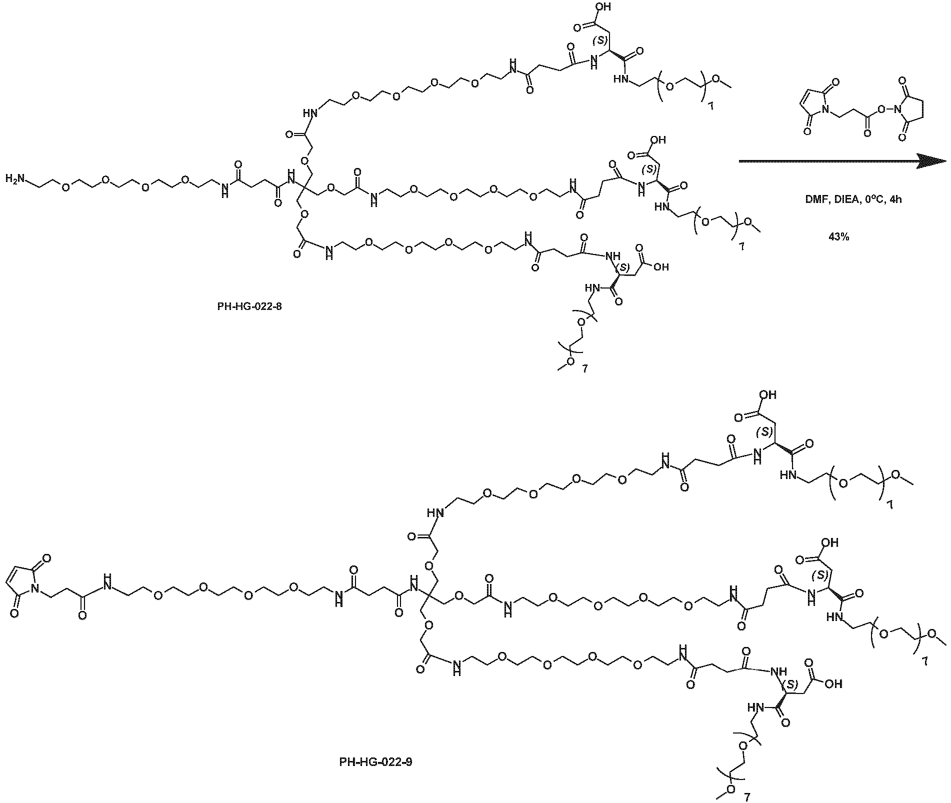

Succinimidyl 3-maleimidopropionate (29.3 mg, 0.11 mmol, 1.5 eq.) and N,N-diisopropylethylamine (27.1 mg, 0.21 mmol, 3.0 eq.) were added to a solution of PH-HG-022-8 (210 mg, 0.070 mmol, 1.0 eq.) in DMF (5.0 mL) under nitrogen at 0 °C. The reaction solution was stirred at 0 °C for 4 hours and then purified by preparative HPLC (conditions: XBridge Prep C18 OBD column; size 30 mm×150 mm, 5 µm; mobile phase, water (0.1% formic acid) and acetonitrile (8.0% to 38.0% acetonitrile, 7 min); detector, UV 220 nm) to give 94 mg (43%) of product PH-HG-022-9 as a colorless oil. MS m/z [M/2+H]⁺(ESI): 1581.45

### Step 9: Synthesis of PH-HG-022-0

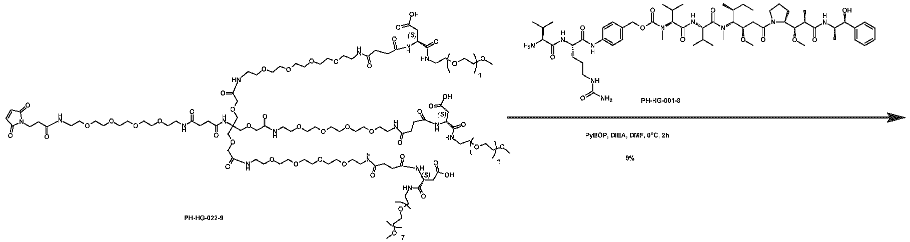

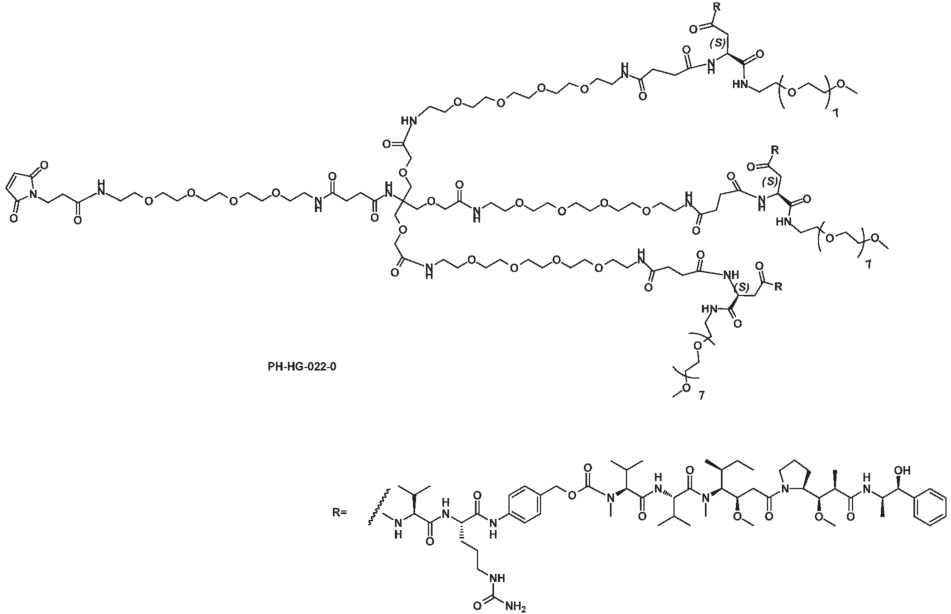

PH-HG-001-8 (108.0 mg, 0.096 mmol, 3.3 eq.), N,N-diisopropylethylamine (11.2 mg, 0.087 mmol, 3.0 eq.), and PyBOP (68.0 mg, 0.13 mmol, 4.5 eq.) were added to a solution of PH-HG-022-9 (94 mg, 0.029 mmol, 1.0 eq.) in DMF (3.0 mL) under nitrogen at 0 °C. The reaction solution was stirred at 0 °C for 2 hours and then purified by preparative HPLC (conditions: XBridge Prep C18 OBD column; size 30 mm × 150 mm, 5 µm; mobile phase, water (0.1% formic acid) and acetonitrile (17.0% to 42.0% acetonitrile, 9 min); detector, UV 220 nm) to give 16.8 mg (9%) of product PH-HG-022-0 as a white solid.

MS m/z [M/4+H]⁺ (ESI): 1619.85.

¹H NMR (300 MHz, DMSO-d₆) δ: 0.65-0.92 (m, 80H), 0.95-1.07 (m, 22H), 1.25-1.59 (m, 18H), 1.66-1.83 (m, 15H), 1.98-2.15 (m, 12H), 2.22-2.46 (m, 26H), 2.68-2.75 (m, 3H), 2.78-2.82 (m, 8H), 2.93-3.03 (m, 10H), 3.06-3.10 (m, 4H), 3.13-3.21 (m, 23H), 3.21-3.27 (m, 25H), 3.39-3.45 (m, 33H), 3.48-3.52 (m, 107H), 3.58-3.61 (m, 7H), 3.68-3.72 (m, 6H), 3.74-3.78 (m, 2H), 3.84-3.91 (m, 7H), 3.95-4.08 (m, 8H), 4.10-4.39 (m, 12H), 4.40-4.59 (m, 9H), 4.61-4.81 (m, 3H), 4.91-5.14 (m, 6H), 5.38-5.44 (m, 9H), 5.93-6.06 (m, 3H), 7.00 (s, 2H), 7.13-7.20 (m, 3H), 7.23-7.35 (m, 20H), 7.60-7.81 (m, 12H), 7.88-8.04 (m, 14H), 8.06-8.13 (m, 2H), 8.16-8.42 (m, 8H), 9.72 (s, 3H).

### Example 11: Synthesis of PH-HG-023-0

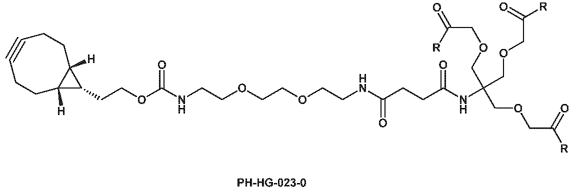

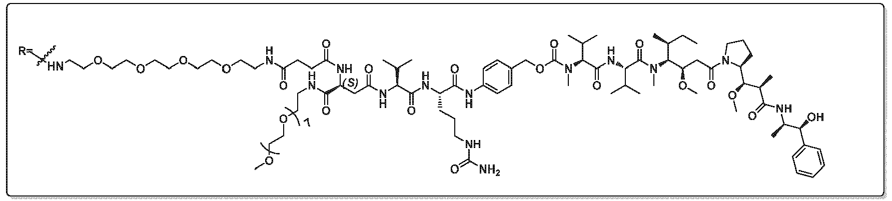

### Step 1: Synthesis of PH-HG-023-1

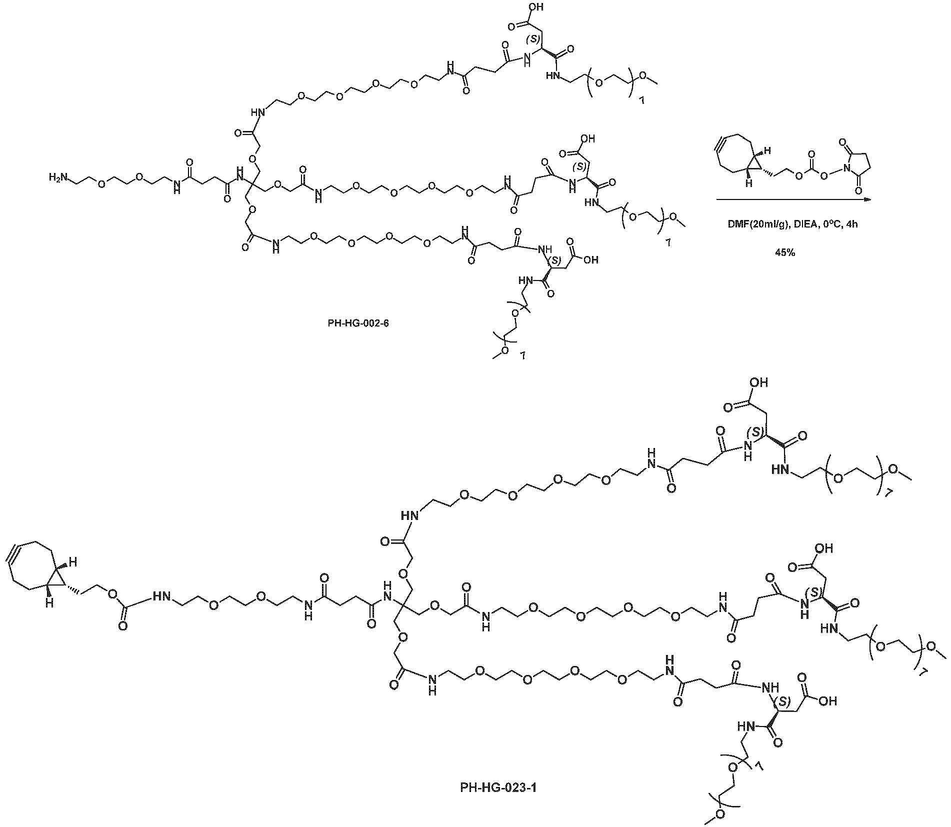

**BCN-EtOSu** (33.6 mg, 0.12 mmol, 1.5 eq.) and N,N-diisopropylethylamine (15.5 mg, 0.12 mmol, 1.5 eq.) were added to a solution of PH-HG-002-6 (230.0 mg, 0.079 mmol, 1.0 eq.) in DMF (3.0 mL) under nitrogen at 0 °C. The reaction solution was stirred at 0 °C for 4 hours and then purified by preparative HPLC (conditions: XBridge Shield RP18 OBD column; size 30 mm × 150 mm, 5 µm; mobile phase, water and acetonitrile (35.0% to 65.0% acetonitrile, 13 min); detector, UV 254 nm) to give 114 mg (45%) of PH-HG-023-1 as a colorless oil.

MS m/z [M/3+H]⁺(ESI): 1038.30

### Step 2: Synthesis of PH-HG-023-0

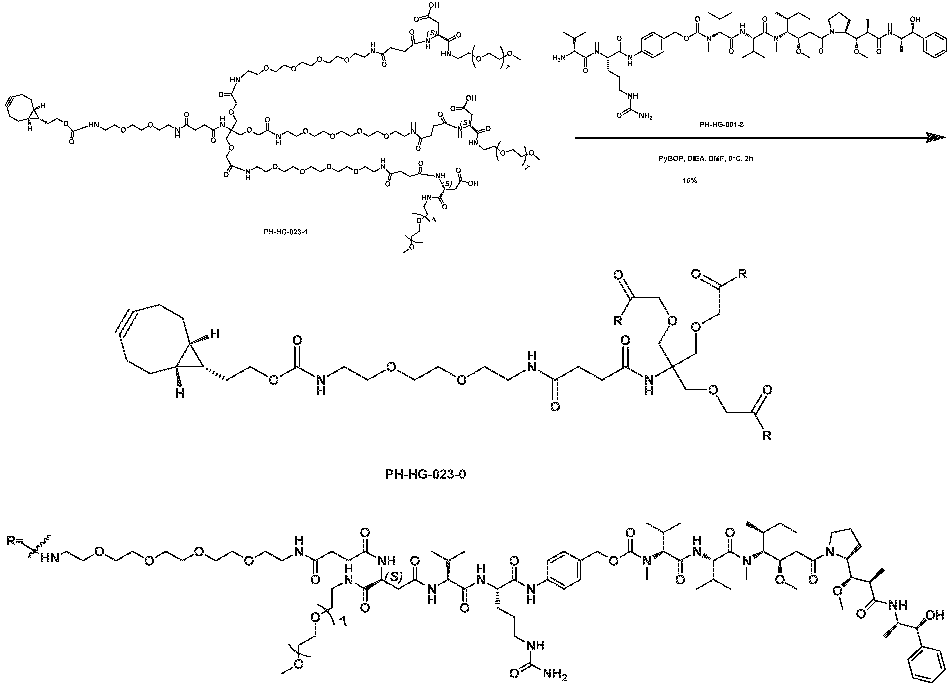

PH-HG-001-8 (134.8 mg, 0.12 mmol, 3.3 eq.), N,N-diisopropylethylamine (20.6 mg, 0.16 mmol, 4.5 eq.) and PyBOP (83.3 mg, 0.16 mmol, 4.5 eq.) were added to a solution of PH-HG-023-1 (110.0 mg, 0.035 mmol, 1.0 eq.) in DMF (3.0 mL) under nitrogen at 0 °C. The reaction solution was stirred at 0 °C for 2 hours and then purified by preparative HPLC (conditions: XBridge Shield RP18 OBD column; size 30 mm × 150 mm, 5 µm; mobile phase, water and acetonitrile (35.0% to 65.0% acetonitrile, 13 min); detector, UV 254 nm) to give 34.1 mg (15%) of PH-HG-023-0 as a white solid.

MS m/z [M/4+H]⁺(ESI): 1608.10.

¹H NMR (400 MHz, DMSO-d6) δ: 0.75-0.96 (m, 85H), 0.97-1.10 (m, 21H), 1.15-1.21 (m, 1H), 1.25-1.60 (m, 21H), 1.61-1.82 (m, 17H), 1.91-2.24 (m, 17H), 2.26-2.47 (m, 29H), 2.62-2.85 (m, 5H), 2.86-2.92 (m, 10H), .95-3.13 (m, 12H), 3.14-3.21 (m, 9H), 3.22-3.37 (m, 25H), 3.38-3.43 (m, 32H), 3.45-3.51 (m, 41H), 3.52-3.58 (m, 36H), 3.60-3.62 (m, 24H), 3.71-3.82 (m, 6H), 3.84-3.91 (m, 18H), 3.93-4.12 (m, 10H), 4.13-4.21 (m, 4H). 4.24-4.38 (m, 8H), 4.40-4.52 (m, 3H), 4.58-4.63 (m, 3H), 4.67-4.74 (m, 4H), 4.82-4.91 (m, 4H), 5.17-5.26 (m, 8H). 5.28-5.67 (m, 6H), 5.92-6.08 (m, 3H), 6.94-7.21 (m, 5H), 7.2-7.35 (m, 19H), 7.52-7.71 (m, 8H), 7.74-7.81 (m, 4H). 7.83-7.95 (m, 9H), 7.97-8.12 (m, 5H), 8.18-8.42 (m, 8H), 9.70 (s, 3H).

### Example 12: Synthesis of PH-HG-024-0

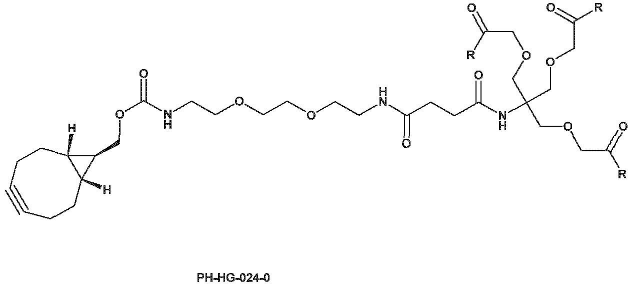

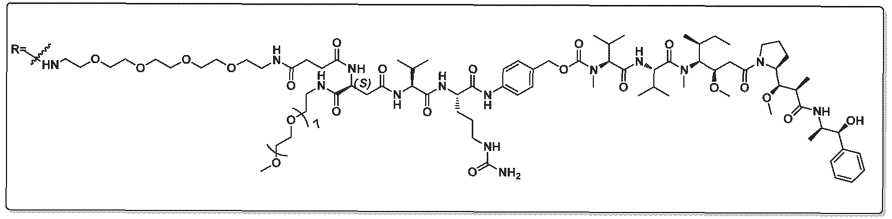

### Step 1: Synthesis of PH-HG-024-1

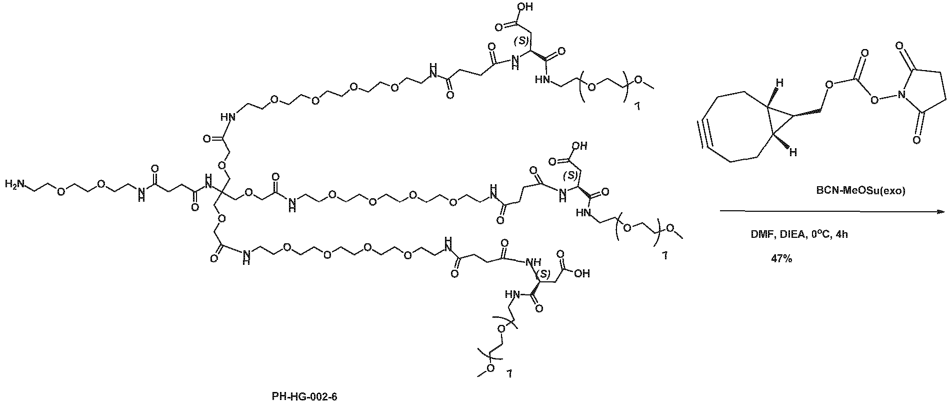

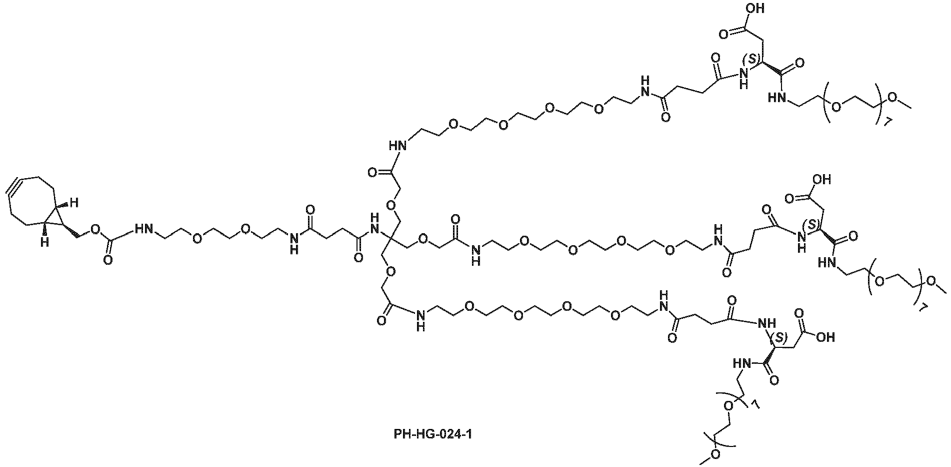

(1R,8S,9R)-Bicyclo[6.1.0]non-4-yn-9-ylmethyl succinimidyl carbonate (BCN-MeOSu-exo) (29.1 mg, 0.10 mmol, 1.5 eq.) and N,N-diisopropylethylamine (33.2 mg, 0.258 mmol, 3.0 eq.) were added to a solution of PH-HG-002-6 (200.0 mg, 0.068 mmol, 1.0 eq.) in DMF (3.0 mL) under nitrogen at 0 °C. The reaction solution was stirred at 0 °C for 4 hours and then purified by preparative HPLC (conditions: XBridge Shield RP18 OBD C18 column; size 30 mm × 150 mm, 5 µm; mobile phase, water and acetonitrile (35.0% to 65.0% acetonitrile, 13 min); detector, UV 254 nm) to give 100 mg (47%) of PH-HG-024-1 as a white solid.

MS m/z [M/3+H]⁺ (ESI): 1033.60.

### Step 2: Synthesis of PH-HG-024-0

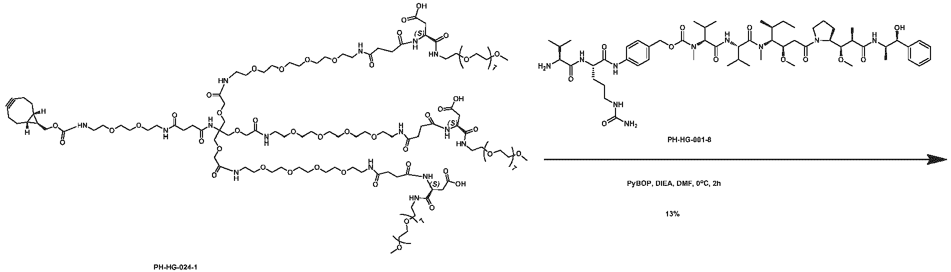

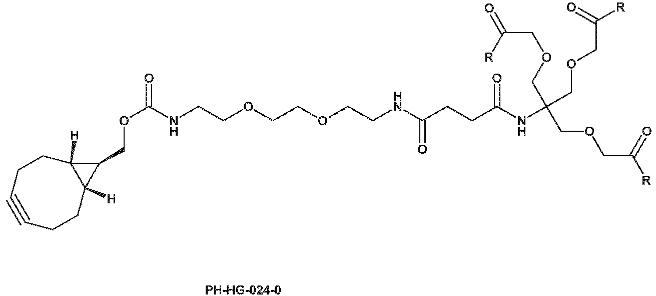

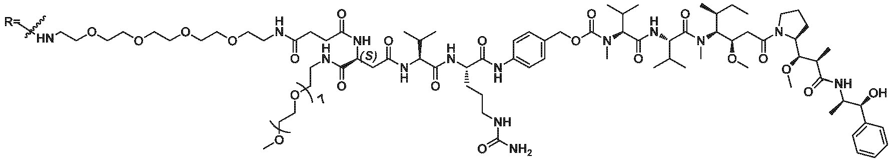

PH-HG-001-8 (149.6 mg, 0.13 mmol, 3.3 eq.), N,N-diisopropylethylamine (23.5 mg, 0.18 mmol, 4.5 eq.) and PyBOP (94.5 mg, 0.18 mmol, 4.5 eq.) were added to a solution of PH-HG-024-1 (125.0 mg, 0.04 mmol, 1.0 eq.) in DMF (3.0 mL) under nitrogen at 0 °C. The reaction solution was stirred at 0 °C for 2 hours and then purified by preparative HPLC (conditions: XBridge Shield RP18 OBD column; size 30 mm × 150 mm, 5 µm; mobile phase, water and acetonitrile (35.0% to 65.0% acetonitrile, 13 min); detector, UV 254 nm) to give 36.8 mg (13%) of PH-HG-024-0 as a white solid.

MS m/z [M/4+H]⁺ (ESI): 1604.55. ¹H NMR (400 MHz, DMSO-d₆) δ: 0.75-0.93 (m, 83H), 0.98-1.11 (m, 23H), 1.16-1.21 (m, 1H), 1.25-1.63 (m, 9H), 1.64-1.83 (m, 11H), 1.89-2.21 (m, 17H), 2.24-2.49 (m, 16H), 2.52-2.84 (m, 32H), 2.86-2.93 (m, 4H), 2.94-2.96 (m, 10H), 2.98-3.12 (m, 12H), 3.14-3.23 (m, 7H), 3.25-3.38 (m, 24H), 3.39-3.42 (m, 29H), 3.45-3.52 (m, 18H), 3.53-3.57 (m, 18H), 3.60-3.62 (m, 20H), 3.71-3.83 (m, 38H), 3.84-3.93 (m, 12H), 3.95-4.17 (m, 12H). 4.18-4.23 (m, 9H), 4.25-4.36 (m, 8H), 4.41-4.53 (m, 4H), 4.58-4.64 (m, 8H), 4.67-4.78 (m, 10H), 4.83-4.95 (m, 4H). 5.18-5.29 (m, 8H), 5.31-5.69 (m, 8H), 6.08 (s, 3H), 6.95-7.24 (m, 26H), 7.58-7.72 (m, 8H), 7.81-7.85 (m, 4H), 7.86-8.03 (m, 12H), 8.05-8.40 (m, 10H), 9.71 (s, 3H).

### Biological example

The following abbreviations are used throughout the present disclosure:

| | |
|---|---|
| ADC | Antibody-drug conjugate |
| BSA | Bovine serum albumin |
| DAR | Drug-to-antibody ratio |
| EC₅₀ | Half maximal effective concentration |
| FACS | Fluorescence-activated cell sorting |
| FBS | Fetal bovine serum |
| IC₅₀ | Half maximal inhibitory concentration |
| MFI | Mean fluorescence intensity |
| NA | Not Available |
| PBS | Phosphate buffered saline |

### Example 13: Trastuzumab conjugate Her-HG021 (DAR1) using PH-HG-021-0 as a scaffold, trastuzumab conjugate Her-HG023 (DAR1) using PH-HG-023-0 as a scaffold, and trastuzumab conjugate Her-HG024 (DAR1) using PH-HG-024-0 as a scaffold.

In PB buffer (40 mM PB, 2 mM EDTA, pH 7.0), NHS-PEG₄-N₃ solution (5 mM NHS-PEG₄-Azide solution in dimethyl sulfoxide, 3 eq.) was added to react with antibody Herceptin. After mixing well, the reaction solution was allowed to stand at 22 °C for 0.5 h.

1/20 volume of the reaction solution of Tris.HCl buffer (1 M, pH 8.0) was added to quench the excess NHS-PEG₄-N₃. After quenching for 10 minutes, the reaction solution was purified using an Amicon ultrafiltration tube (0.5 mL, 10K MWCO).

PB buffer, dimethyl sulfoxide (DMSO) and linker-payload solution (4 mM solution in dimethyl sulfoxide, 15 eq.) were added to the above reaction solution. After mixing well, the reaction solution was shaken and reacted at 37 °C on a shaker for 70.5 hours (with a shaking speed of 60 rpm).

After the reaction was completed, the ADC solution was concentrated into a storage buffer (20 mM sodium succinate, 6% sucrose, pH 5.0) by solution replacement using an Amicon ultrafiltration tube (4 mL, 50 K MWCO), and finally filtered with a 0.2 µm PVDF syringe filter to give the ADC product, which was sampled for evaluation.

### Example 14: Trastuzumab conjugate Her-HG022 (DAR1) using PH-HG-022-0 as a scaffold.

In PB buffer (40 mM PB, 2 mM EDTA, pH 7.0), the antibody Herceptin was reduced with 1.0 eq. of tris(2-carboxyethyl)phosphine (TCEP), and the reaction solution was shaken and reacted at 22 °C on a shaker for 3 hours (with a shaking speed of 60 rpm). Without removing excess TCEP, the reaction solution was directly used for the next coupling reaction.

PB buffer, dimethylacetamide (DMA) and a solution of linker-payload PH-HG-022-0 (3 eq., 5 mM solution in DMA) were slowly added to the reduced antibody in an ice bath. After mixing well, the coupling reaction solution was allowed to stand and reacted at 4 °C for 2 hours.

After the reaction was completed, the ADC solution was first purified using a desalting column (2 mL, 40 K MWCO), and then concentrated into a storage buffer (20 mM histidine, pH 5.5) by solution replacement using an Amicon ultrafiltration tube (4 mL, 50 K MWCO), and finally filtered with a 0.2 µm PVDF syringe filter to give the ADC product, which was sampled for evaluation.

### Data summary of antibody-drug conjugates

### (1) Antibody-drug conjugate [Her-HG021]

| **MS-DAR** | **Concentration (mg/mL)** | **Aggregation (%)** | **Amount (mg)** | **Free drug (mol%)** |
|---|---|---|---|---|
| **1.29** | **2.957** | **3** | **1.3** | **<5%** |

### (2) Antibody-drug conjugate [Her-HG022]

| **HIC-DAR** | **Concentration (mg/mL)** | **Aggregation (%)** | **Amount (mg)** | **Free drug (mol%)** |
|---|---|---|---|---|
| **1.16** | **1.31** | **3** | **0.8** | **<2%** |

### (3) Antibody-drug conjugate [Her-HG023]

| **MS-DAR** | **Concentration (mg/mL)** | **Aggregation (%)** | **Amount (mg)** | **Free drug (mol%)** |
|---|---|---|---|---|
| **1.35** | **2.854** | **1** | **0.9** | **<2%** |

### (4) Antibody-drug conjugate [Her-HG024]

| **MS-DAR** | **Concentration (mg/mL)** | **Aggregation (%)** | **Amount (mg)** | **Free drug (mol%)** |
|---|---|---|---|---|
| **1.29** | **3.609** | **2** | **1.3** | **<2%** |

### SEC analysis method

| | | |
|---|---|---|
| Chromatography column | TOSON - G3000SWXL, 300*7.8mm, 5µm | |
| Column temperature | 22°C | |
| Wavelength | 280 nm, 252 nm, 370 nm | |
| Injection amount | 30 to 50 µg | |
| Mobile phase system | 0.2 M dipotassium phosphate/monopotassium phosphate, 0.25 M potassium chloride, 15% (v/v) isopropyl alcohol, pH 7.0 | |
| Gradient | Time (min) | Flow rate (mL/min) |
| | 0.0 | 0.75 |
| | 18.0 | 0.75 |

### HIC analysis method

| | | | |
|---|---|---|---|
| Chromatography column | TSKgel Butyl-NPR, 4.6 mm *3.5cm, 2.5µm | | |
| Column temperature | 25°C | | |
| Wavelength | 280 nm | | |
| Injection volume | 8 µL | | |
| Mobile phase system | Mobile phase A: 1.5 M ammonium sulfate, 25 mM sodium phosphate, pH 7.0 | | |
| | Mobile phase B: 50 mM sodium phosphate, 25% (V/V) isopropyl alcohol | | |
| Gradient | Time (min) | % B | Flow rate (mL/min) |
| | 0.0 | 0.0 | 0.6 |
| | 2.0 | 0.0 | |
| | 15.0 | 100.0 | |
| | 16.0 | 100.0 | |
| | 17.0 | 0.0 | |
| | 20.0 | 0.0 | |

### Assay method for the level of free linker-payload (Free drug):

The level of free linker-payload in an ADC product was assayed by reversed-phase high-performance liquid chromatography (RP-HPLC).
1) The linker-payload was diluted with pure water and a corresponding organic solvent to 2 X mol/L and 0.8 X mol/L, with the final solution containing 10% organic solvent. $5 / 100 = \frac{X mol / L}{X + DAR ∗ \frac{1 mg / mL}{{MW}_{protein} + DAR ∗ {MW}_{drug}}}$
2) The antibody was diluted with pure water and the same organic solvent to 2 mg/mL, with the final solution containing 10% organic solvent.
3) 12.5 µL of the solution of 2) was added to 12.5 µL of the solution of 1), respectively, resulting in a standard solution with an antibody concentration of 1 mg/mL and a linker-payload concentration of 5% or 2% (molar percentage), respectively.
4) The ADC was diluted with pure water and the same organic solvent to 1 mg/mL, with the final solution containing 10% organic solvent.
5) The prepared ADC solution and the 5% or 2% (molar percentage) linker-payload standard solution were detected by LC-MS.

| | | | |
|---|---|---|---|
| Chromatography column | Agilent, PLRP-S, 2.1*50mm, 8µm | | |
| Wavelength | 252 nm | | |
| Column temperature | 80 °C | | |
| Flow rate | 0.4 mL/min | | |
| Time | 13 min | | |
| Injection volume | 10 µL | | |
| Mobile phase system | Mobile phase A: 0.025% trifluoroacetic acid and 0.1% formic acid in water | | |
| | Mobile phase B: 0.025% trifluoroacetic acid and 0.1% formic acid in acetonitrile | | |
| | Time (min) | A(%) | B (%) |
| | 0.0 | 95 | 5 |
| | 0.5 | 95 | 5 |
| | 2.0 | 70 | 30 |
| Gradient | 8.0 | 20 | 80 |
| | 8.5 | 10 | 90 |
| | 10.0 | 10 | 90 |
| | 10.01 | 95 | 5 |
| | 13.0 | 95 | 5 |

### Assay method for concentration

The concentration of ADC was assayed using a BCA kit.

### Assay method for endotoxin

The level of endotoxin in the sample was assayed using a kinetic turbidimetric assay according to the standard procedures.

### Example 15: Example of in vitro characterization

| **Abbreviation** | **Full name** |
|---|---|
| Her2 | Human epidermal growth factor receptor 2 (also known as erbB-2) |
| FACS | Fluorescence-activated cell sorting |
| MFI | Mean fluorescence intensity |
| EC₅₀ | Half maximal effective concentration |
| IC₅₀ | Half maximal inhibitory concentration |
| FBS | Fetal bovine serum |
| Penicillin/Streptomycin | Penicillin/Streptomycin |
| CTG | Cell Titer Glo luminescent cell viability assay |

### Sample information:

| **Sample name** | **Concentration (mg/ml)** | **Buffer** | **Storage condition** |
|---|---|---|---|
| Herceptin | 20.98 | 40 mM PB, 2 mM EDTA, pH 7.0 | -80 °C |
| DS-8201 | 2.5 | 150 mg of trastuzumab, lyophilized with 3.36 mg of L-histidine hydrochloride, 2.16 mg of L-histidine, 136.2 mg of trehalose dihydrate and 0.6 mg of polysorbate 20 | -80 °C |
| Her-HG022 | 1.313 | 20mM histidine, pH 5.5 | -80 °C |
| Her-HG021 | 2.957 | 20 mM sodium succinate, 6% sucrose, pH 5.2 | -80 °C |
| Her-HG023 | 2.854 | 20 mM sodium succinate, 6% sucrose, pH 5.2 | -80 °C |
| Her-HG024 | 3.609 | 20 mM sodium succinate, 6% sucrose, pH 5.2 | -80 °C |
| Human IgG1 subtype control | 13.6 | 20 mM histidine, 5% sucrose, pH 6.0 | -80 °C |

### Assay materials

| **Material name** | **Supplier** | **Item No.** |
|---|---|---|
| NCI-N87 cells | ATCC | CRL-5822 |
| RPMI1640 medium | Gibco | 22400-089 |
| FBS | Ausgenex | FND500 |
| Penicillin/streptomycin solution | Cytiva | SV30010 |
| CTG | Promega | G7573 |
| Goat anti-human IgG Fc-Alexa 647 | Jackson | 109-605-098 |

The flow cytometer was purchased from BD (model: Canto II with HTS module).

### Assay method

### Preparation of DS-8201

In PB buffer (40 mM PB, 2 mM EDTA, pH 7.0), the antibody Herceptin was reduced with 7.5 eq. of TCEP, and the reaction solution was shaken and reacted at 32 °C on a shaker for 3 hours (with a shaking speed of 60 rpm). Without removing excess TCEP, the reaction solution was directly used for the next coupling reaction. DMA (dimethylacetamide) and Deruxtecan solution (10 mg/mL, dissolved in DMA, 20 eq.) were slowly added to the reaction solution. After mixing well, the reaction solution was reacted at 22 °C for 2 hours. After the reaction was completed, the reaction solution was transferred into 20 mM histidine buffer by solution replacement with a desalting column (40 K), and filtered with a 0.2 µm PVDF syringe filter to give the ADC product, which was sampled for evaluation.

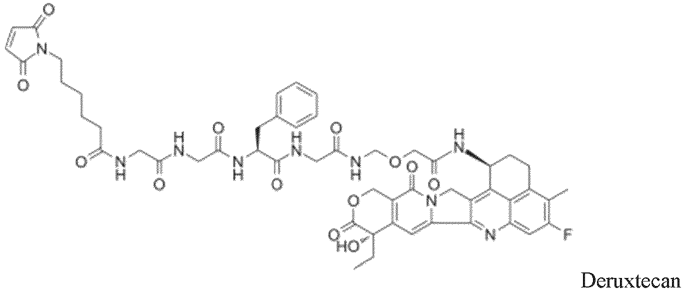

### Binding assay of ADC antibody and target cells

FACS assay was used to detect the binding specificity of an ADC antibody to NCI-N87 cells. This method can quantitatively analyze and specifically detect the target proteins on the surface of living cells. Unlabeled cells were used as a blank control to determine the threshold of the signal detection. A signal above this threshold was considered as a specific signal generated after the antibody bound to the target protein. NCI-N87 tumor cells (1× 10⁵ cells/well) expressing Her2 proteins and different concentrations of ADC antibodies (starting from 800 nM, 4-fold serial dilution) were mixed together (making the total volume to 100 µL/well) and incubated in a refrigerator (with the temperature set to 4 °C) for 1 hour. Anti-Her2 antibody (Herceptin) was used as a positive control. Human IgG1 subtype antibody was used as a negative control. The cells were washed with 1×PBS/1%BSA, and then A647-labeled goat anti-human antibody (1:500 diluted with 1×PBS/1%BSA) was added. The cells were incubated in a refrigerator (with the temperature set to 4 °C) in the dark for 1 hour. The mean fluorescence intensity (MFI) of the cells was detected by flow cytometry, and the data were analyzed using FlowJo. The EC₅₀ value was calculated by four-parameter nonlinear regression analysis using GraphPad Prism 7 software.

### Cytotoxicity assay

Cell viability assay was used to detect the cytotoxicity of an ADC antibody on NCI-N87 cells. This method can quantitatively analyze and specifically detect the cytotoxicity of an ADC antibody on NCI-N87 cells. Cells cultured without addition of ADC antibody were used as a blank control to determine the threshold of the cell viability detection. A signal below this threshold was considered as a specific signal generated after the ADC antibody killed cells. NCI-N87 cells (5×10³ cells/well) were resuspended in a RPMI1640 medium containing 15% FBS and inoculated into a black 96-well plate with transparent bottom in a volume of 50 µL. The 96-well plate was placed in an incubator set at 37 °C and 5% CO₂ overnight. After the overnight incubation, 50 µL of different concentrations of ADC antibodies or MMAE (starting from 400 nM, 4-fold serial dilution) were added to the 96-well plate (making the total volume to 100 µL/well), and the 96-well plate was placed in an incubator set at 37 °C and 5% CO₂ for 144 hours. Cell Titer Glo (50 µL/well) was added to each well and the mixture was thoroughly mixed. The luciferase intensity was read using Envision. The cytotoxic effect was calculated using the following formula: cytotoxicity % = 100*(RFU_{cell only}-RFU ₛₐₘₚₗₑ)/RFU_{cell only}. The IC₅₀ value was calculated by four-parameter nonlinear regression analysis using GraphPad Prism 7 software.

### Assay results

### Assay results of the binding of ADC antibody to target cells

The ADC antibodies showed similar binding abilities to the control antibodies (Herceptin and DS-8201). The human IgG1 subtype control antibody was used as a negative control in the assay and did not show specific binding to NCI-N87. The analysis results of each ADC antibody to NCI-N87 cells can be seen in Table 2.

**Table 2 FACS data analysis of ADC antibody to NCI-N87**

| | **Concentration (mg/ml)** | **EC₅₀ (nM)** | **Max MFI** | **Negative/Blank MFI** |
|---|---|---|---|---|
| **Sample name** Herceptin | 20.98 | 1.28 | 67100 | 10.3/8.71 |
| DS-8201 | 2.5 | 1.07 | 60900 | 10.7/8.71 |
| Human IgG1 subtype control | 13.6 | NA | 29.9 | NA/9.92 |
| Her-HG021 | 2.957 | 1.25 | 60100 | 11.1/8.71 |
| Her-HG022 | 1.313 | 1.04 | 61100 | 16.7/8.71 |
| Her-HG023 | 2.854 | 1.29 | 59200 | 11.4/8.71 |
| Her-HG024 | 3.61 | 1.534 | 57800 | 10.7/9.92 |

### Assay results of cytotoxicity of ADC antibody (NCI-N87 cells)

All the ADC antibodies and control antibodies (Herceptin and DS-8201) showed toxicity to NCI-N87 cells. The analysis results of each ADC antibody and NCI-N87 cells can be seen in Table 3.

**Table 3 Data analysis of cytotoxicity of ADC antibody on NCI-N87 cells**

| | **Concentration (mg/ml)** | **IC50 (nM)** | **Max Inh%** |
|---|---|---|---|
| **Sample name** DS-8201 | 2.50 | 0.18 | 87.86 |
| Herceptin | 20.98 | 2.16 | 48.65 |
| Her-HG021 | 2.957 | 0.03 | 90.16 |
| Her-HG022 | 1.31 | 0.04 | 87.57 |
| Her-HG023 | 2.854 | 0.03 | 90.51 |
| Her-HG024 | 3.609 | 0.04 | 91.89 |
| MMAE | 0.72 | 0.12 | 90.41 |

All features disclosed in this specification may be combined in any combination. Each feature disclosed in this specification may be replaced by an alternative feature serving the same, equivalent or similar purpose. Thus, unless expressly stated otherwise, each feature disclosed is only an example of a series of equivalent or similar features.

From the above description, one skilled in the art can easily ascertain the essential features of the present disclosure, and can make various changes and modifications to the present disclosure without departing from the spirit and scope of the present disclosure to adapt this disclosure to various uses and conditions. Accordingly, other examples are also within the scope of the appended claims.

## Claims

1. A compound of formula (I), or a tautomer, a stereoisomer, a pharmaceutically acceptable salt or an isotopic variant thereof, 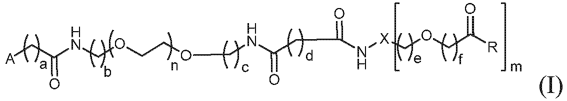 wherein,
a, b, c, d, e, and f are each independently 0, 1, 2, 3, 4, 5, 6, 7, 8, 9 or 10; alternatively 0, 1, 2, 3, 4 or 5; alternatively 0, 1, 2 or 3; alternatively 1 or 2; alternatively a is 0 or 2, b, c and d are 2, and e and f are 1;
n is 1, 2, 3, 4, 5, 6, 7, 8, 9, 10, 11, 12, 13, 14, 15, 16, 17, 18, 19 or 20; alternatively 1, 2, 3, 4, 5, 6, 7, 8, 9 or 10; alternatively 1, 2, 3, 4 or 5; alternatively 1, 2 or 3; alternatively 1;
m is 2 or 3;
X is C, N or Si;
A is -NH₂, -NH-PG1, 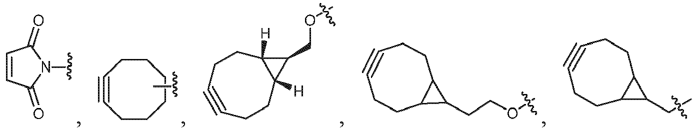
or 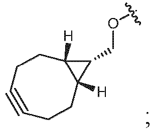
R is -OH, -O-PG2, 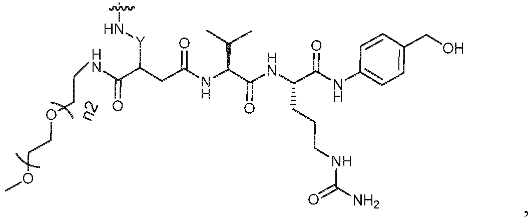 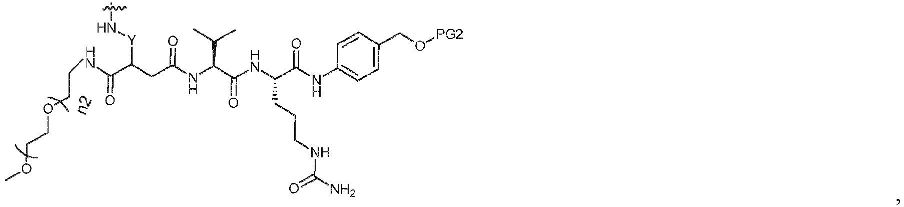 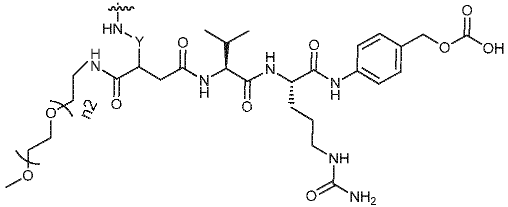 or 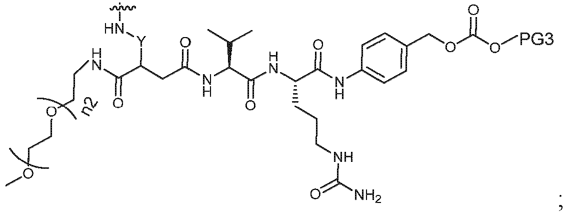
wherein PG1, PG2 and PG3 are protecting groups;
n2 is 5, 6, 7, 8, 9, 10, 11, 12, 13, 14, 15, 16, 17, 18, 19 or 20; alternatively 5, 6, 7, 8, 9, 10, 11, 12, 13, 14 or 15; alternatively 7, 8, 9, 10 or 11;
Y is a bond or 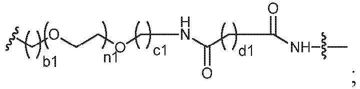
wherein n1 is 1, 2, 3, 4, 5, 6, 7, 8, 9, 10, 11, 12, 13, 14, 15, 16, 17, 18, 19 or 20; alternatively 1, 2, 3, 4, 5, 6, 7, 8, 9 or 10; alternatively 1, 2, 3, 4 or 5; alternatively 1, 2 or 3; alternatively n1 is 3, 4, 5, 6, 7 or 8, alternatively 3;
b1, c1 and d1 are each independently 0, 1, 2, 3, 4, 5, 6, 7, 8, 9 or 10; alternatively 0, 1, 2, 3, 4 or 5; alternatively 0, 1, 2 or 3; alternatively 1 or 2; alternatively b1, c1 and d1 are 2;
when X is C or Si, m is 3;
when X is N, m is 2;
with the proviso that, when A is 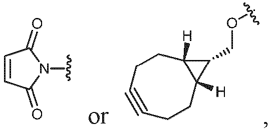 then n is not 1.

2. The compound according to claim 1, which has a structure represented by formula (II), or a tautomer, a stereoisomer, a pharmaceutically acceptable salt or an isotopic variant thereof, 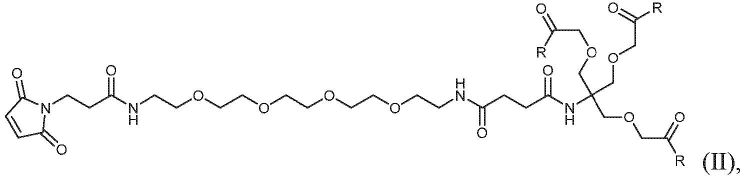 wherein R is as defined in claim 1.

3. The compound according to claim 1, wherein the compound has a structure represented by formula (III), or a tautomer, a stereoisomer, a pharmaceutically acceptable salt or an isotopic variant thereof, 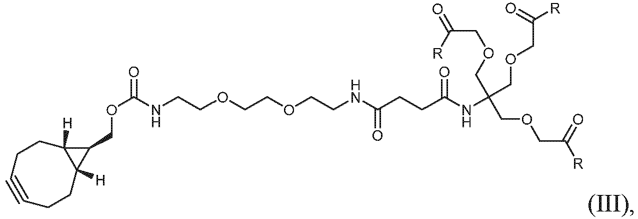 wherein R is as defined in claim 1.

4. The compound according to claim 1, wherein the compound has a structure represented by formula (IV), or a tautomer, a stereoisomer, a pharmaceutically acceptable salt or an isotopic variant thereof, 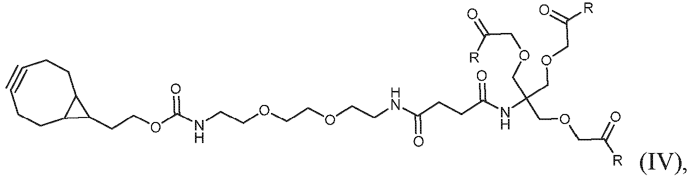 wherein R is as defined in claim 1.

5. The compound according to claim 1, wherein the compound has a structure represented by formula (V), or a tautomer, a stereoisomer, a pharmaceutically acceptable salt or an isotopic variant thereof, 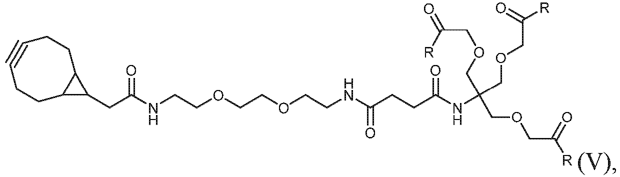 wherein R is as defined in claim 1.

6. The compound according to any one of claims 1 to 5, wherein, R is -OH, -O-PG2, 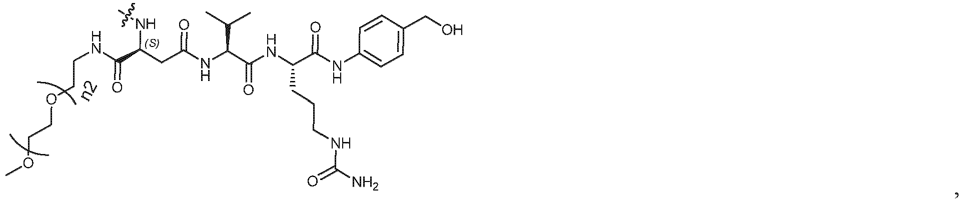 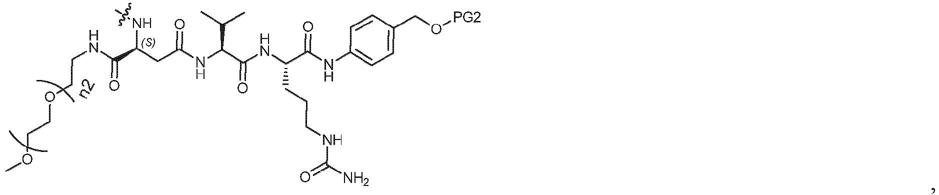 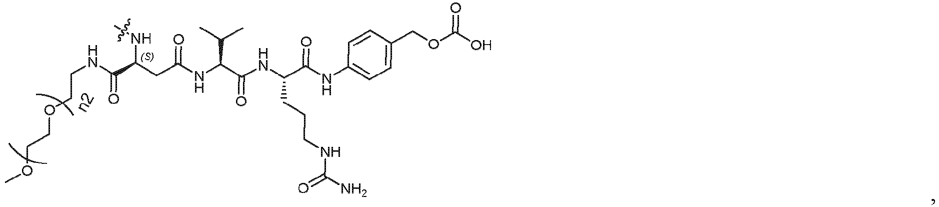 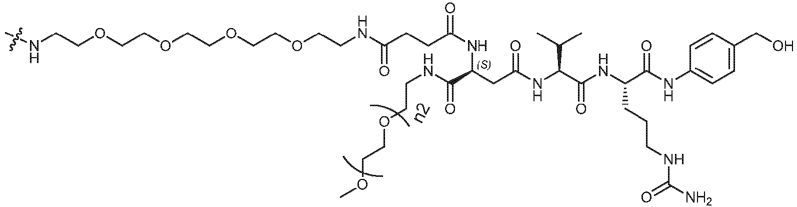 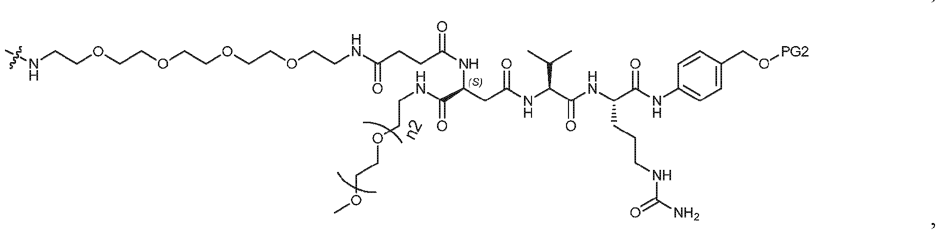 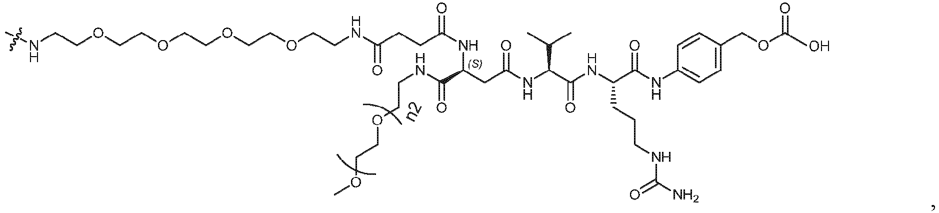 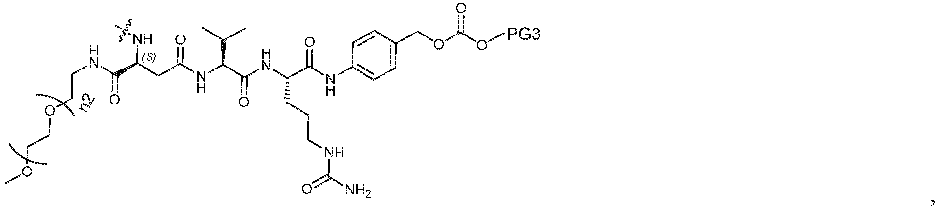 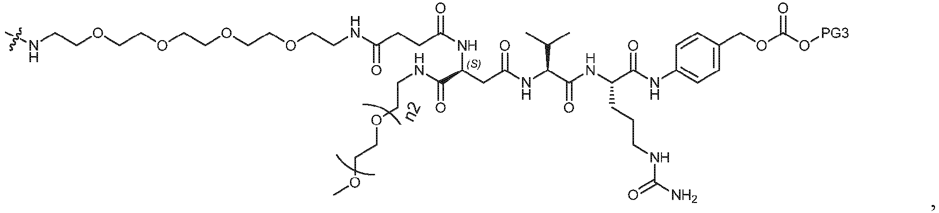 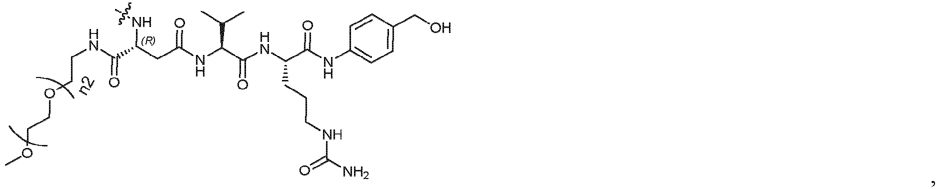 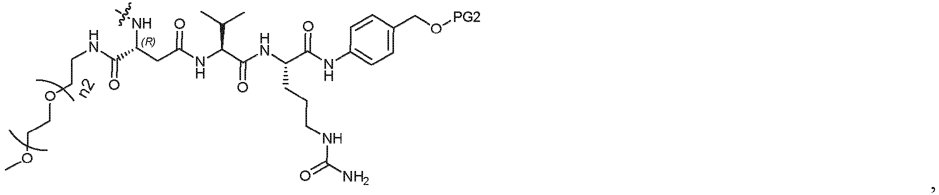 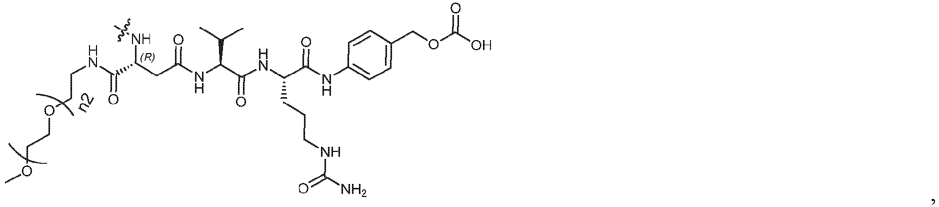 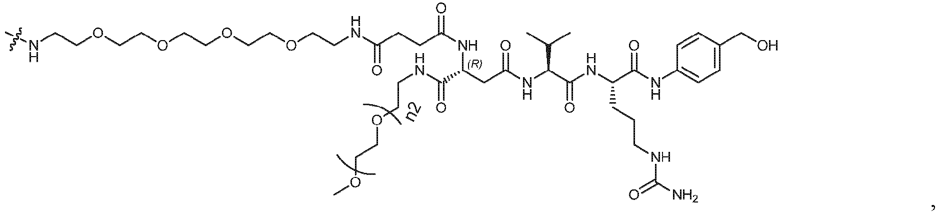 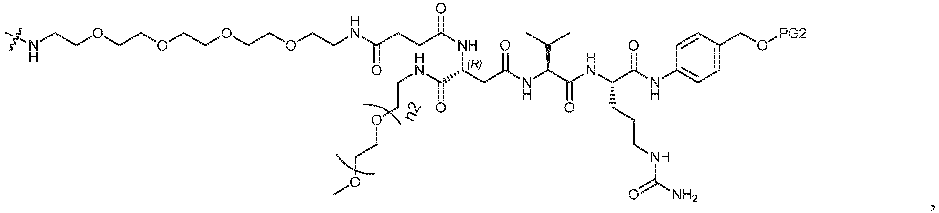 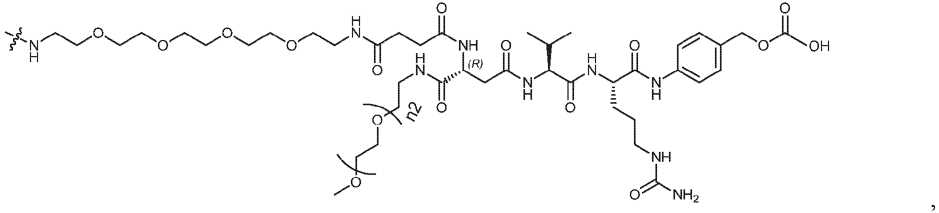 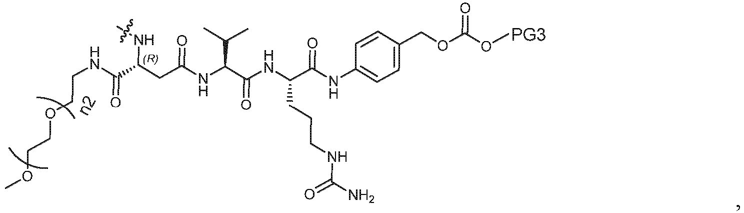 or 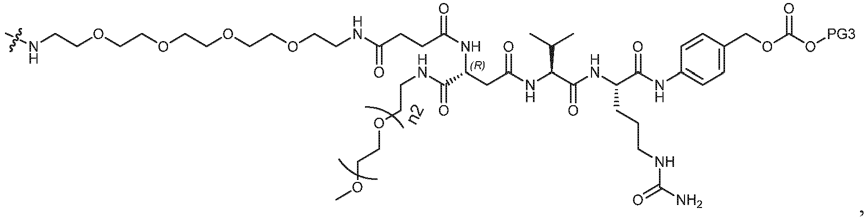 wherein n2, PG1, PG2 and PG3 are as defined in claim 1.

7. The compound according to any one of claims 1 to 6, wherein n2 is 7 or 11.

8. The compound according to any one of claims 1 to 7, wherein the PG1 is an amino-protecting group;
optionally, the amino-protecting group is selected from acetyl, trifluoroacetyl, tert-butoxycarbonyl (BOC, Boc), benzyloxycarbonyl (CBZ, Cbz) and 9-fluorenylmethyleneoxycarbonyl (Fmoc);
the PG2 is a hydroxyl-protecting group;
optionally, the hydroxyl-protecting group is selected from acetyl and silyl;
the PG3 is a carboxyl-protecting group;
optionally, the carboxyl-protecting group is selected from -CH₂CH₂SO₂Ph, cyanoethyl, 2-(trimethylsilyl)ethyl, 2-(trimethylsilyl)ethoxymethyl, 2-(p-toluenesulfonyl)ethyl, 2-(p-nitrobenzenesulfonyl)ethyl, 2-(diphenylphosphino)ethyl and nitroethyl.

9. A conjugate, comprising the compound according to any one of claims 1 to 8 and a drug moiety, wherein the compound is covalently bonded to the drug moiety via an R group.

10. The conjugate according to claim 9, wherein the drug is selected from one or more of eribulin, monomethyl auristatin E and SN-38, or an isotopic variant thereof.

11. The conjugate according to claim 10, wherein the conjugate is selected from the following specific compounds, or a tautomer, a stereoisomer, a pharmaceutically acceptable salt or an isotopic variant thereof, and the compounds are represented by the structure shown in formula (VI): 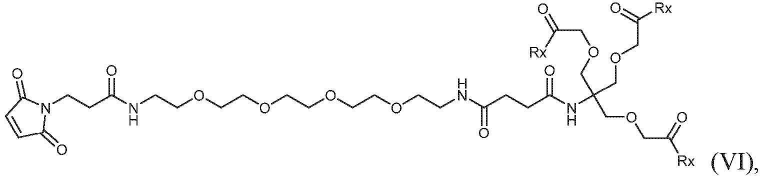 wherein, Rx is 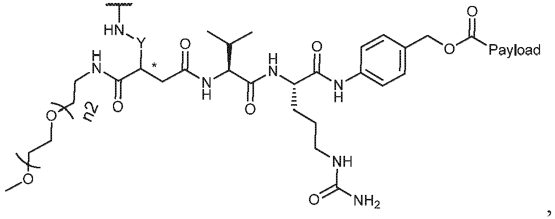 and n2, *, Y and payload are as defined in Table 1.

12. The conjugate according to claim 10, wherein the conjugate is selected from the following specific compounds, or a tautomer, a stereoisomer, a pharmaceutically acceptable salt or an isotopic variant thereof, and the compounds are represented by the structure shown in formula (VII): 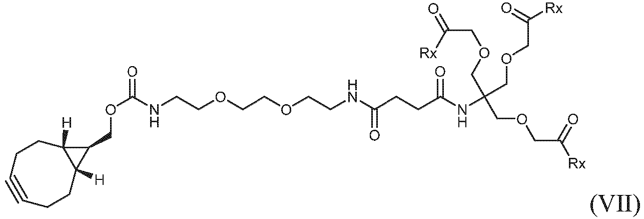 wherein, Rx is 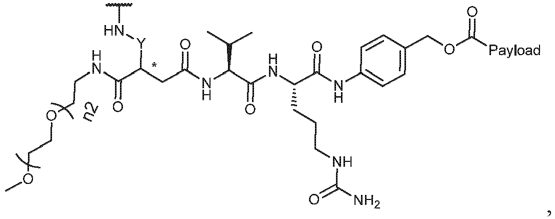 and n2, *, Y and payload are as defined in Table 1.

13. The conjugate according to claim 10, wherein the conjugate is selected from the following specific compounds, or a tautomer, a stereoisomer, a pharmaceutically acceptable salt or an isotopic variant thereof, and the compounds are represented by the structure shown in formula (VIII): 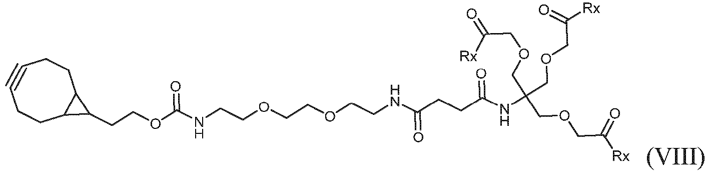 wherein, Rx is 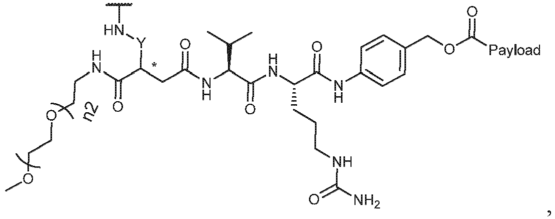 and n2, *, Y and payload are as defined in Table 1.

14. The conjugate according to claim 10, wherein the conjugate is selected from the following specific compounds, or a tautomer, a stereoisomer, a pharmaceutically acceptable salt or an isotopic variant thereof, and the compounds are represented by the structure shown in formula (IX): 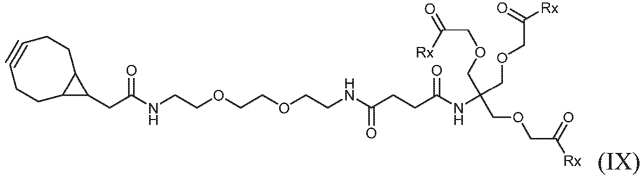 wherein, Rx is 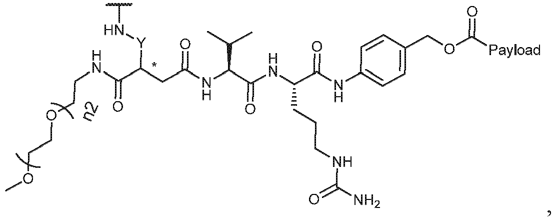 and n2, *, Y and payload are as defined in Table 1.

15. The conjugate according to any one of claims 9 to 14, further comprising a targeting moiety, wherein one or more of the conjugates are covalently bonded to the targeting moiety via an A group.

16. A conjugate, comprising a targeting moiety and one or more of the compounds according to any one of claims 1 to 8, wherein the compound is covalently bonded to the targeting moiety via an A group.

17. The conjugate according to claim 15 or 16, wherein the targeting moiety is a protein-based recognition molecule.

18. The conjugate according to claim 17, wherein the recognition molecule is an internalizing antibody or an internalizing antigen-binding fragment thereof targeting tumor cells.

19. The conjugate according to claim 18, wherein the antibody or antigen-binding fragment binds to antibodies selected from: anti-human epidermal growth factor receptor (HER2) antibody, EGFR, GPNMB, CD56, TACSTD2 (TROP2), CEACAM5, folate receptor-a, mesothelin, ENPP3, guanylate cyclase C, SLC44A4, NaPi2b, CD70, mucin 1, STEAP1, connexin 4, 5T4, SLTRK6, SC-16, LIV-1, P-cadherin, PSMA, extra domain B of fibronectin, endothelin receptor ETB, tenascin c, collagen IV, VEGFR2, periostin, CD30, CD79b, CD19, CD22, CD138, CD37, CD33 and CD74, etc.

20. A pharmaceutical composition, comprising the conjugate according to any one of claims 9 to 19 and a pharmaceutically acceptable carrier.

21. Use of the conjugate according to any one of claims 9 to 19 or the pharmaceutical composition according to claim 20 in the manufacture of a medicament for treating a patient having or at risk of having a cancer expressing a target antigen.

22. The conjugate according to any one of claims 9 to 19 or the pharmaceutical composition according to claim 20, for use in treating a patient having or at risk of having a cancer expressing a target antigen.

23. A method of treating a patient having or at risk of having a cancer expressing a target antigen, comprising administering to the patient the conjugate according to any one of claims 9 to 19 or the pharmaceutical composition according to claim 20.

24. The use according to claim 21, or the conjugate or the pharmaceutical composition for use according to claim 22, or the method according to claim 23, wherein the target antigen is human epidermal growth factor receptor 2.

25. The use according to claim 21, or the conjugate or the pharmaceutical composition for use according to claim 22, or the method according to claim 23, wherein the cancer expresses a high level of human epidermal growth factor receptor 2.

26. The use according to claim 21, or the conjugate or the pharmaceutical composition for use according to claim 22, or the method according to claim 23, wherein the cancer is selected from breast cancer, gastric cancer, bladder cancer and urothelial carcinoma.
